# EUROPEAN PATENT APPLICATION

(11) **EP 3 056 201 A1**
(43) Date of publication of application: **17.08.2016**
(21) Application number: 16153625.5
(22) Date of filing: 20.05.2011
(51) Int. Cl.: A61K 31/337, A61K 31/7068, A61K 31/16, A61K 45/06

(54) **COMBINATION THERAPY METHODS FOR TREATING PROLIFERATIVE DISEASES**

(30) Priority: 07.06.2010 US 352333 P; 25.02.2011 US 201161446909 P
(62) Divisional of application: 11792867.1
(71) Applicant: Abraxis BioScience, LLC, Los Angeles, CA 90025 (US)
(72) Inventor: Desai, Neil P., Los Angeles, CA California 90025 (US); Soon-Shiong, Patrick, Los Angeles, CA California 90049 (US)
(74) Representative: Jones Day

(57) **Abstract**

The invention provides a composition comprising nanoparticles comprising a taxane and a carrier protein for use in a method of treating a proliferative disease in an individual, wherein the method further comprises administering to the individual at least one other agent that inhibits a DNA methyltransferase. The invention further provides a kit and a medicine comprising a composition comprising nanoparticles comprising a taxane and a carrier protein, and at least one other agent that inhibits a DNA methyltransferase.

SEQUENCE LISTING

<110> Abraxis Bioscience, LLC

<120> COMBINATION THERAPY METHODS FOR TREATING PROLIFERATIVE DISEASES

<130> 103151PCEPT1

<140> EP16153625.5
<141> 2011-05-20

<150> US 61/446,909
<151> 2011-02-25

<150> US 61/352,333
<151> 2010-06-07

<160> 6

<170> BiSSAP 1.3

<210> 1
<211> 21
<212> DNA
<213> Homo sapiens

<220>
<223> Ex8-S, forward PCR amplification primer of SPARC

<400> 1
aagatccatg agaatgagaa g                                                21

<210> 2
<211> 19
<212> DNA
<213> Homo sapiens

<220>
<223> Ex9-AS, reverse PCR amplification primer of SPARC

<400> 2
aaaagcgggt ggtgcaatg                                                   19

<210> 3
<211> 23
<212> DNA
<213> Homo sapiens

<220>
<223> Sparc-unmsp-F, primer for methylation-specific PCR (sense)

<400> 3
ttttttagat tgtttggaga gtg                                              23

<210> 4
<211> 25
<212> DNA
<213> Homo sapiens

<220>

<223> Sparc-unmsp-R, primer for methylation-specific PCR (antisense)

<400> 4
aactaacaac ataaacaaaa atatc                                          25


<210> 5
<211> 20
<212> DNA
<213> Homo sapiens

<220>
<223> sparc-msp-F, primer for methylation-specific PCR (sense)

<400> 5
gagagcgcgt tttgtttgtc                                                20


<210> 6
<211> 22
<212> DNA
<213> Homo sapiens

<220>
<223> sparc-msp-R, primer for methylation-specific PCR (antisense)

<400> 6
aacgacgtaa acgaaaatat cg                                             22

## Description

### CROSS-REFERENCE TO RELATED APPLICATIONS

This application claims priority benefit to U.S. Provisional Patent Application Nos. 61/352,333 filed June 7, 2010, and 61/446,909, filed February 25, 2011, the contents of each are hereby incorporated herein by reference in their entirety.

### TECHNICAL FIELD

The present invention relates to methods and compositions for the treatment of proliferative diseases comprising the administration of a combination of a taxane and at least one other therapeutic agent useful in the treatment of proliferative diseases.

### BACKGROUND

Cancer is a leading cause of death world wide. Despite significant advances in the field of chemotherapy, many of the most prevalent forms of cancer still resist chemotherapeutic intervention.

Breast cancer is the most prevalent form of cancer in women. In 2009, an estimated 192,370 new cases of invasive breast cancer were expected to be diagnosed in women in the U.S., along with 62,280 new cases of non-invasive (in situ) breast cancer. About 40,170 women in the U.S. were expected to die in 2009 from breast cancer.

Triple-negative breast cancer is a subtype of breast cancer that is clinically negative for expression of estrogen receptor (ER), progesterone receptors (PR) and HER2 protein. Because triple-negative breast cancer cells do not express any of these receptors, they are generally unresponsive to standard receptor-mediated treatments. Chemotherapy is currently the most effective treatment for patients with triple negative breast cancer. However, many patients continue to suffer recurrence and death despite aggressive therapy, emphasizing the need for new therapeutic strategies.

Epigenetic alternations in the genome contribute to cancer initiation and progression. For example, histone hypoacetylation and abnormal DNA methylation in promoter regions of important genes can lead to gene silencing. Multiple genes are methylated and thus silenced in breast cancer. Pu RT. Mod. Path., 2003. The functions of epigenetic modifiers in treating various cancers have been investigated. Cooper et al., Gynecol. Oncol. 2007, 104(3):596-601;Ramalingam et al., J. Clin. Oncology, 2006 ASCO Annual Meeting Proceedings, Part I., Vol. 24 (18S); and Kim MS, Cancer Res 2003.

Taxanes (such as paclitaxel and docetaxel) have been shown to have significant antineoplastic and anticancer effects in a wide variety of cancers. For example, paclitaxel acts by interfering with the normal function of microtubule breakdown. Paclitaxel binds to the beta subunit of tubulin, the building blocks of microtubules, causing hyper-stabilization of the microtubule structures. The resulting paclitaxel/microtubule structure is unable to disassemble, thereby arresting mitosis and inhibiting angiogenesis. The poor aqueous solubility for the taxanes, however, presents significant challenges for developing effective taxane-based cancer therapeutics. Furthermore, the interaction of different taxane formulations with other therapeutic agents in the combination therapy context remains to be studied.

Albumin-based nanoparticle compositions have been developed as a drug delivery system for delivering substantially water insoluble drugs such as taxanes. See, for example, U.S. Pat. Nos. 5,916,596; 6,506,405; 6,749,868, and 6,537,579, and 7,820,788 and also in U.S. Pat. Pub. Nos. 2007/0082838. The albumin-based nanoparticle technology utilizes the natural properties of the protein albumin to transport and deliver substantially water insoluble drugs to the site of disease. These nanoparticles are readily incorporated into the body's own transport processes and are able to exploit the tumors' attraction to albumin, enabling the delivery of higher concentrations of the active drug encapsulated in the nanoparticles to the target site. In addition, the albumin-based nanoparticle technology offers the ability to improve a drug's solubility by avoiding the need for toxic chemicals, such as solvents, in the administration process, thus potentially improving safety through the elimination of solvent-related side effects.

Other references include PCT Application Nos. WO08/057562, WO2009126938A1, WO2009126401A1, WO2009126175A1.

The disclosures of all publications, patents, patent applications and published patent applications referred to herein are hereby incorporated herein by reference in their entirety.

### BRIEF SUMMARY OF THE INVENTION

The invention provides combination therapy methods of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies the epigenetics in a cell (also referred herein as an "epigenetic modifier" or "the other agent"). In some embodiments, the invention provides a method of treating a proliferative disease (such as cancer) in an individual comprising administering to the individual a) an effective amount of a composition comprising nanoparticles comprising paclitaxel coated with an albumin (such as Abraxane^{®} or "nab-paclitaxel"), and b) an effective amount of at least one other agent that modifies the epigenetics in a cell. In some embodiments, the other agent modifies (such as inhibits) DNA methylation. In some embodiments, the other agent modifies histone modification, which includes, but is not limited to, histone acetylation, histone methylation, histone sumoylation, and histone phosphorylation. In some embodiments, the other agent is an inhibitor of DNA methyltransferase (such as azacitidine). In some embodiments, the other agent is an inhibitor of DNA methyltransferase (such as decitabine).In some embodiments, the other agent is an inhibitor of a histone deacetylase (such as vorinostat).

In some embodiments, the proliferative disease is resistant or refractory to the treatment of taxane when administered alone or in conjunction with an agent other than the epigenetic modifier. In some embodiments, the proliferative disease is resistant or refractory to the treatment when the epigenetic modifier is administered alone or in conjunction with an agent other than the nanoparticle composition (such as a non-nanoparticle composition of a taxane including paclitaxel).

In some embodiments, the composition comprising nanoparticles (also referred to as "nanoparticle composition") and the other agent are administered simultaneously, either in the same composition or in separate compositions. In some embodiments, the nanoparticle composition and the other agent are administered sequentially, i.e., the nanoparticle composition is administered either prior to or after the administration of the other agent.

In some embodiments, the administration of the nanoparticle composition and the other agent is concurrent, i.e., the administration period of the nanoparticle composition and that of the other agent overlap with each other. In some embodiments, the nanoparticle composition is administered for at least one cycle (for example, at least any of 2, 3, or 4 cycles) prior to the administration of the other agent. In some embodiments, the other agent is administered for at least any of one, two, three, or four weeks after the termination of the nanoparticle composition. In some embodiments, the nanoparticle composition and the epigenetic modifier are administered over the same treatment cycles.

In some embodiments, the administration of the nanoparticle composition and the other agent are non-concurrent. For example, in some embodiments, the administration of the nanoparticle composition is terminated before the other agent is administered. In some embodiments, the administration of the other agent is terminated before the nanoparticle composition is administered.

In some embodiments, the other agent is a histone deacetylase inhibitor, including, but not limited to, vorinostat, romidepsin, panobinostat, belinostat, and entinostat. In some embodiments, the other agent is an inhibitor of DNA methyltransferase, including, but not limited to, 5-azacytidine (azacitidine or Vidaza), 5-aza-2'-deoxycytidine (decitabine or Dacogen), 1-β-D-arabinofuranosil-5-azacytosine, dihydro-5-azacytidine, antisense oligonucleotide MG98, and zebularine.

Thus, for example, in some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of a vorinostat. In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of an azacitidine. In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of a decitabine.

In some embodiments, there is provided a method of treating breast cancer in an individual, comprising administering to the individual a) an effective amount of a composition comprising nanoparticles comprising a taxane (such as paclitaxel) and a carrier protein (such as albumin), and b) an effective amount of at least one other agent that modifies the epigenetics in a cell. In some embodiments, there is provided a method of treating breast cancer in an individual, wherein the individual is negative for ER, PR, and HER2, comprising administering to the individual a) an effective amount of a composition comprising nanoparticles comprising a taxane (such as paclitaxel) and a carrier protein (such as albumin), and b) an effective amount of at least one other agent that modifies the epigenetics in a cell. In some embodiments, the method further comprises conducting definitive surgery within about 1, 2, 3, 4, 5, 6, 7, 8, 9, or 10 following the preoperative therapy.

The methods of the invention generally comprise administration of a composition comprising nanoparticles comprising a taxane (such as paclitaxel) and a carrier protein. In some embodiments, the nanoparticle composition comprises nanoparticles comprising paclitaxel and an albumin. In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 400, including for example about 20 to about 200 nm, about 30 to about 180 nm, and any one of about 40 to about 150, about 50 to about 120, and about 60 to about 100 nm.

In some embodiments, the carrier protein has sulfhydral groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the taxane (such as paclitaxel) coated with a carrier protein, such as albumin (e.g., human serum albumin). In some embodiments, the composition comprises taxane in both nanoparticle and non-nanoparticle form, wherein at least about any one of 50%, 60%, 70%, 80%, 90%, 95%, or 99% of the taxane in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles comprise a core of taxane that is substantially free of polymeric materials (such as polymeric matrix).

In some embodiments, the nanoparticle composition is substantially free (such as free) of surfactants (such as Cremophor®, Tween 80, or other organic solvents used for the administration of taxanes). In some embodiments, the nanoparticle composition contains less than about any one of 20%, 15%, 10%, 7.5%, 5%, 2.5%, or 1% organic solvent. In some embodiments, the weight ratio of carrier protein (such as albumin) and taxane in the nanoparticle composition is about 18:1 or less, such as about 15:1 or less, for example about 9:1 or less. In some embodiments, the weight ratio of carrier protein (such as albumin) and taxane in the composition falls within the range of any one of about 1:1 to about 18:1, about 2:1 to about 15:1, about 3:1 to about 13:1, about 4:1 to about 12:1, about 5:1 to about 10:1, about 9:1. In some embodiments, the weight ratio of carrier protein and taxane in the nanoparticle portion of the composition is about any one of 1:2, 1:3, 1:4, 1:5, 1:9, 1:10, 1:15, or less.

In some embodiments, the particle composition comprises one or more of the above characteristics.

In some embodiments, the nanoparticle composition is Abraxane^{®}. Nanoparticle compositions comprising other taxanes (such as docetaxel and ortataxel) may also comprise one or more of the above characteristics.

Thus, for example, in some embodiments, there is provided a method of treating breast cancer (such as triple negative breast cancer), comprising administering to said individual a) an effective amount of Abraxane®, and b) an effective amount of a histone deacetylase inhibitor. In some embodiments, there is provided a method of treating breast cancer (such as triple negative breast cancer), comprising administering to said individual a) an effective amount of Abraxane®, and b) an effective amount of a DNA methyltransferase inhibitor. In some embodiments, there is provided a method of treating breast cancer (such as triple negative breast cancer), comprising administering to said individual a) an effective amount of Abraxane®, and b) an effective amount of vorinostat. In some embodiments, the method further comprises administering to the individual an effective amount of a platinum-based agent.

In some embodiments, there is provided a method of treating breast cancer (such as triple negative breast cancer), comprising: a) intravenously administering an effective amount of Abraxane^{®} to the individual, and b) orally administering an effective amount of vorinostat. to the individual. In some embodiments, the method further comprises intravenously administering to the individual an effective amount of a platinum-based agent (such as carboplatin). In some embodiments, there is provided a method of treating breast cancer (such as triple negative breast cancer), comprising: a) intravenously administering about 80 to about 200 mg/m² (such as about 100 mg/m²) of Abraxane^{®} to the individual, and b) orally administering about 200 to about 500 mg (such as about 400 mg) of vorinostat to the individual. In some embodiments, the method further comprises intravenously administering to the individual an effective amount of a platinum-based agent (such as carboplatin) at the dose of AUC2.

In some embodiments, there is provided a method of treating breast cancer (such as triple negative breast cancer), comprising: a) intravenously administering about 80 to about 200 mg/m² (such as about 100 mg/m²) of Abraxane^{®} to the individual weekly, and b) orally administering about 200 to about 500 mg (such as about 400 mg) of vorinostat to the individual three out of every seven days. In some embodiments, the method further comprises intravenously administering to the individual an effective amount of carboplatin at the dose of AUC2 weekly. In some embodiments, the nanoparticle composition (and the carboplatin) is administered on day one of each week, and vorinostat is administered on days 1-3 of each week.

In some embodiments, there is provided a method of treating lung cancer (such as Non-small cell lung cancer (NSCLC)), comprising administering to said individual a) an effective amount of Abraxane®, and b) an effective amount of a DNA methyltransferase inhibitor. In some embodiments, there is provided a method of treating lung cancer (such as NSCLC), comprising administering to said individual a) an effective amount of Abraxane®, and b) an effective amount of decitabine.

In some embodiments, there is provided a method of treating lung cancer (such as NSCLC), comprising: a) intravenously administering an effective amount of Abraxane^{®} to the individual, and b) intravenously administering an effective amount of decitabine to the individual. In some embodiments, there is provided a method of treating lung cancer (such as NSCLC), comprising: a) intravenously administering about 80 to about 200 mg/m² (such as about 90 or 100 mg/m²) of Abraxane^{®} to the individual, and b) intravenously administering about 1 mg/m² to about 15 mg/m² (such as 4.5 mg/m²) of decitabine to the individual.

In some embodiments, there is provided a method of treating lung cancer (such as NSCLC), comprising: a) intravenously administering about 80 to about 200 mg/m² (such as about 100 mg/m²) of Abraxane^{®} to the individual, and b) intravenously administering about 5 mg/m² to about 100 mg/m² (such as 15 mg/m²) of decitabine to the individual.

In some embodiments, there is provided a method of treating lung cancer (such as NSCLC), comprising: a) intravenously administering about 80 to about 200 mg/m² (such as about 90 or 100 mg/m²) of Abraxane^{®} to the individual for five consecutive days, and b) intraperitoneally administering about 1 mg/m²/day to about 15 mg/m²/day (such as 4.5 mg/m²/day) of decitabine to the individual twice a day for five consecutive days.

In some embodiments, there is provided a method of treating lung cancer (such as NSCLC), comprising: a) intravenously administering about 80 to about 200 mg/m² (such as about 100 mg/m²) of Abraxane^{®} to the individual for weekly, and b) intravenously administering about 5 mg/m² to about 100 mg/m² (such as 15 mg/m²) of decitabine to the individual every eight hours for three consecutive days. In some embodiments, the decitabine treatment is repeated every six weeks for four or more cycles.

In some embodiments, there is provided a method of treating lung cancer (such as NSCLC), comprising: a) intravenously administering about 80 to about 200 mg/m² (such as about 100 mg/m²) of Abraxane^{®} to the individual for weekly, and b) intravenously administering about 5 mg/m² to about 100 mg/m² (such as 20 mg/m²) of decitabine to the individual daily for five consecutive days. In some embodiments, the decitabine treatment is repeated every four weeks for four or more cycles.

Also provided are kits and compositions useful for methods described herein.

The methods of the present application are useful for the treatment of various diseases, including, for example, breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the proliferative disease is solid tumor. In some embodiments, the proliferative disease is liquid tumor.

These and other aspects and advantages of the present invention will become apparent from the subsequent detailed description and the appended claims.
It is to be understood that one, some, or all of the properties of the various embodiments described herein may be combined to form other embodiments of the present invention.

### BRIEF DESCRIPTION OF FIGURES

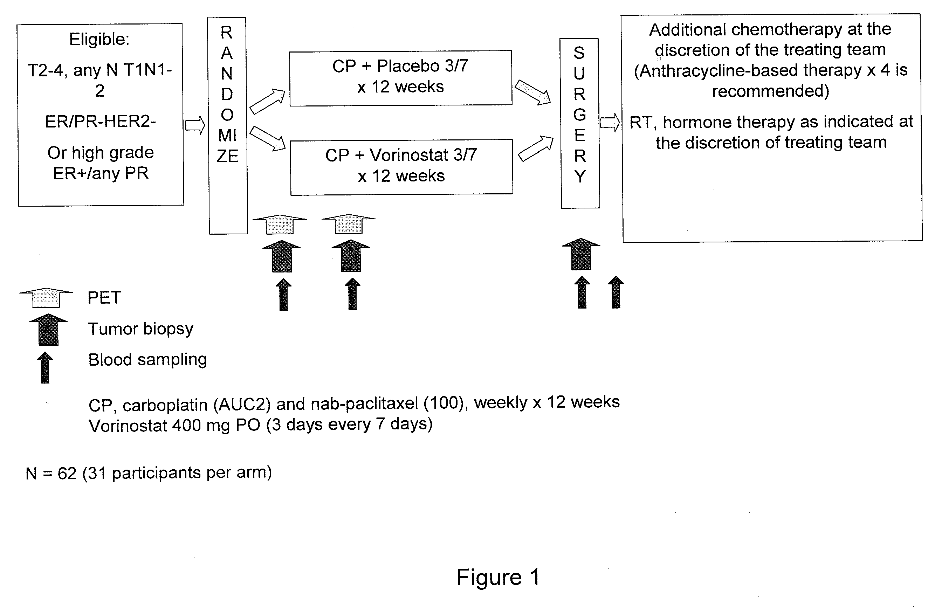

Figure 1 shows the study design for the treatment with carboplatin and *nab*-paclitaxel with or without vorinostat in patients with HER2-negative primary operable breast cancer.

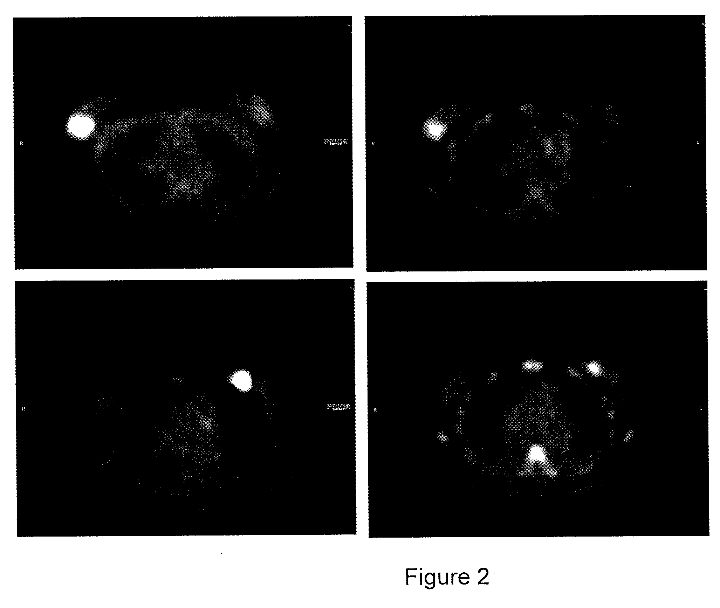

Figure 2 shows FDG-PET results prior to neoadjuvant therapy and D7 post neoadjuvant therapy from 2 patients. Patient 1 (top) shows FDG-PET prior to neoadjuvant therapy (left) documenting a right-sided breast mass (SUV of 12.4) and D7 post neoadjuvant therapy (right) revealing a reduction in SUV to 6.7. Patient 1 had a partial response to therapy. Patient 2 (bottom) shows FDG-PET prior to neoadjuvant therapy (left) documenting a left-sided breast mass (SUV of 31) and D7 post neoadjuvant therapy (right) revealing a reduction in SUV to 9.9. Patient 2 had a complete response to therapy.

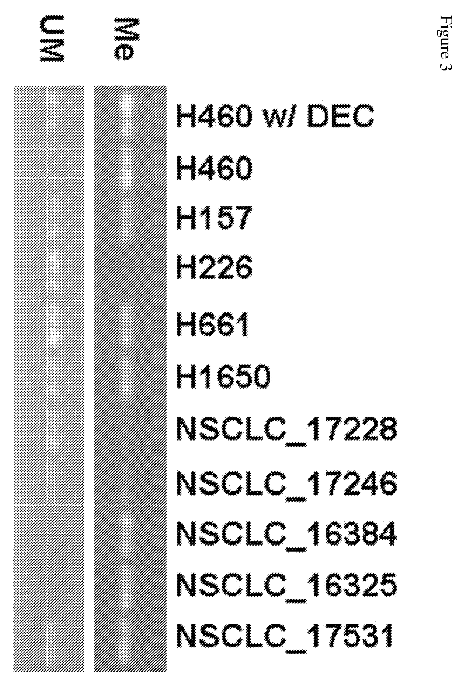

Figure 3 shows representative examples of a methylation-specific PCR assay for SPARC in NSCLC cell lines and NSCLC xenografts. H460 cells were treated with decitabine (5 µM) in vitro for 3 days (H460 w/ DEC). PCR products were visualized on 1.5% agarose gels. Me represents methylated and UM represents unmethylated.

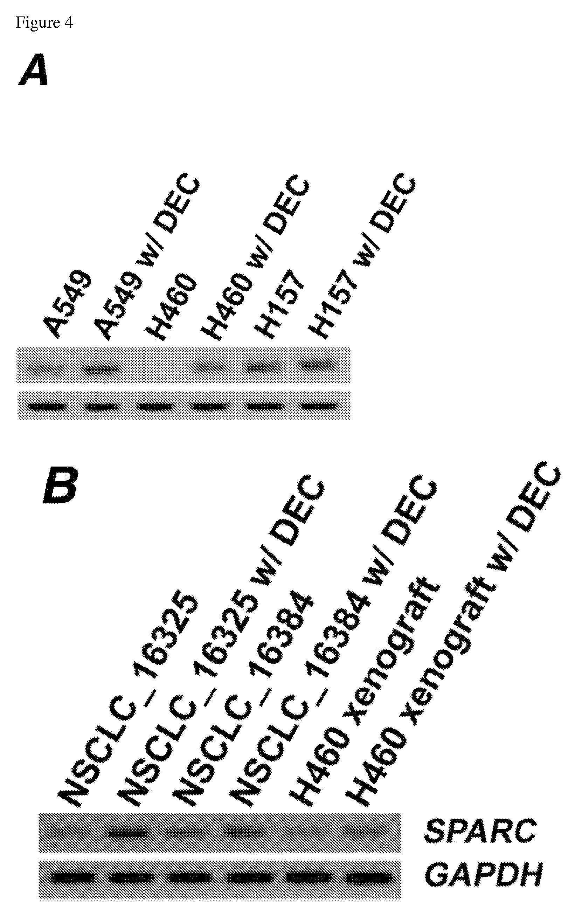

Figure 4 shows a western blot analysis demonstrating that SPARC expression in xenografts and NSCLC cell lines is up-regulated on treatment with decitabine. Panel A shows a western blot of NSCLC cell lines A549, H460, and H157, treated with 5 µM decitabine for 3 days in vitro and then harvested for RT-PCR. Panel B shows PD NSCLC xenografts and H460 xenografts treated with or without decitabine at 1.5 mg/kg/d. Xenografts were harvested for SPARC expression analysis. GAPDH was used as the endogenous control. GAPDH is glyceraldehyde-3-phosphate dehydrogenase.

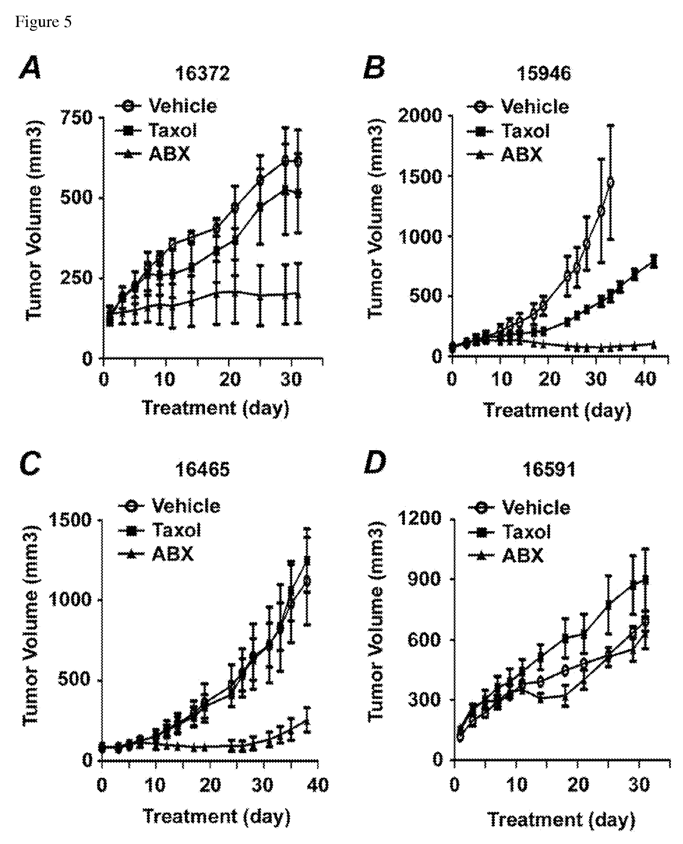

Figure 5 shows the efficacy of Abraxane^{®} compared with taxol in PD NSCLC xenografts. SCID mice bearing SPARC-positive xenografts (NSCLC_16372) (Panel A), SPARC-intermediate xenografts (NSCLC_15946) (Panel B), or SPARC-negative xenografts, NSCLC_16465 (Panel C) and NSCLC_16591 (Panel D). Cells were treated with vehicle, equitoxic dose of taxol (13.4 mg/kg), or Abraxane^{®} (30 mg/kg) (ABX). The overall antitumor efficacy of drugs was measured as tumor volumes every 2 to 3 days. The error bars represented the standard error of the mean.

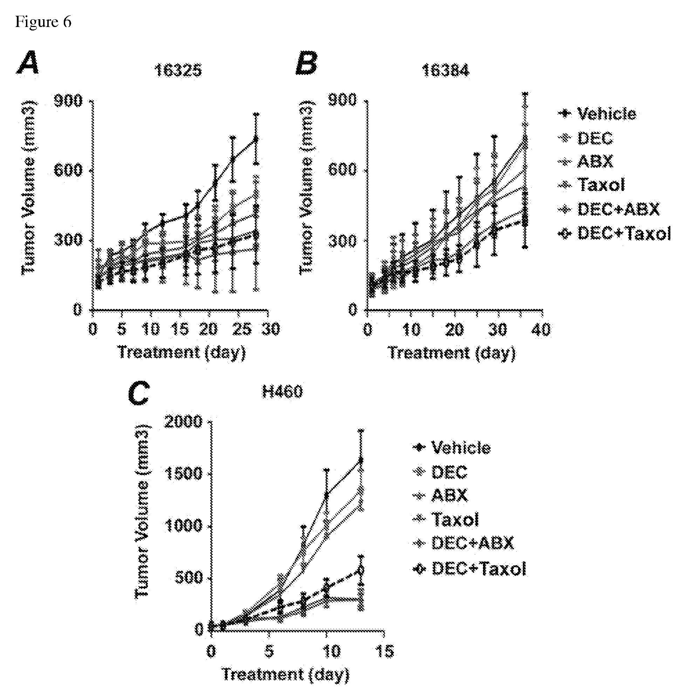

Figure 6 shows enhanced antitumor efficacy of taxol and Abraxane^{®} by pretreatment with decitabine in SPARC-negative xenografts. Panels A-C, SCID mice bearing SPARC-negative xenografts, NSCLC_16325 (Panel A), NSCLC_16384 (Panel B), or H460 (Panel C), were administered with decitabine (1.5 mg/kg) (DEC), taxol (13.4 mg/kg) or Abraxane^{®} (30 mg/kg) (ABX) alone or the combination of decitabine and taxol or decitabine and Abraxane®. The overall antitumor efficacy of drugs was measured as tumor volumes every 2 to 3 days. The error bars represented the standard error of the mean.

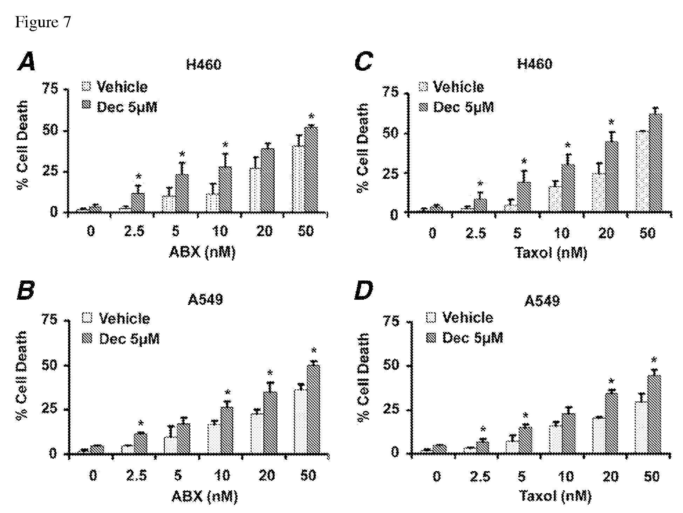

Figure 7 shows a cell death rate analysis with NSCLC cell lines A549 and H460 in vitro showing additive antitumor activity of Abraxane^{®} (ABX) or taxol by pretreatment with decitabine. Panels A and C show the results of H460 cells treated with escalating concentrations of Abraxane^{®} or taxol after decitabine 5 µM pretreatment. Panels B and D show the results of A549 cells treated with escalating concentrations of Abraxane^{®} of 0 to 50 nM or taxol, 0 to 50 nM, after decitabine 5 µM pretreatment. Treated cells were harvested for cell death rates assay (**p* < 0.05, compared with Abraxane^{®} or taxol treatment alone). Data represents mean ± standard deviation of three independent experiments.

### DETAILED DESCRIPTION OF THE INVENTION

The present invention provides methods of combination therapy comprising a first therapy comprising administration of nanoparticles comprising a taxane (such as paclitaxel) and a carrier protein (such as albumin) in conjunction with a second agent that modifies the epigenetics in a cell (also referred to as an "epigenetic modifier").

We hypothesize that combination therapy of a nanoparticle composition comprising a taxane (such as paclitaxel) and a carrier protein (such as albumin) with an epigenetic modifier would significantly improve the efficacy of nanoparticle forms of taxane-based therapy and/or the efficacy of the epigenetic modifier.

The present application thus provides methods of combination therapy. It is to be understood by a person of ordinary skill in the art that the combination therapy methods described herein requires that one agent or composition be administered in conjunction with another agent. "In conjunction with" refers to administration of one treatment modality in addition to another treatment modality, such as administration of a nanoparticle composition described herein in addition to administration of the other agent to the same individual. As such, "in conjunction with" refers to administration of one treatment modality before, during or after delivery of the other treatment modality to the individual.

The methods described herein are generally useful for treatment of proliferative diseases. As used herein, "treatment" is an approach for obtaining beneficial or desired clinical results. For purposes of this invention, beneficial or desired clinical results include, but are not limited to, any one or more of: alleviation of one or more symptoms, diminishment of extent of disease, preventing or delaying spread (e.g., metastasis, for example metastasis to the lung or to the lymph node) of disease, preventing or delaying recurrence of disease, delay or slowing of disease progression, amelioration of the disease state, and remission (whether partial or total). Also encompassed by "treatment" is a reduction of pathological consequence of a proliferative disease. The methods of the invention contemplate any one or more of these aspects of treatment.

An "agent that modifies the epigenetics in a cell" or "epigenetic modifier" refers to an agent that modifies an epigenetic status of a cell, namely, a phenotype or gene expression in the cell that is caused by mechanisms other than changes in the DNA sequence. An epigenetic status of a cell includes, for example, DNA methylation, histone modification(s) and RNA-associated silencing.

Individuals having "triple negative breast cancer" used herein refer to individuals who are clinically negative for expression of estrogen receptor (ER), progesterone receptors (PR) and HER2 protein.

The term "effective amount" used herein refers to an amount of a compound or composition sufficient to treat a specified disorder, condition or disease such as ameliorate, palliate, lessen, and/or delay one or more of its symptoms. In reference to cancers or other unwanted cell proliferation, an effective amount comprises an amount sufficient to cause a tumor to shrink and/or to decrease the growth rate of the tumor (such as to suppress tumor growth) or to prevent or delay other unwanted cell proliferation.

The term "individual" is a mammal, including humans. An individual includes, but is not limited to, human, bovine, horse, feline, canine, rodent, or primate. In some embodiments, the individual is human.

The methods may be practiced in an adjuvant setting. "Adjuvant setting" refers to a clinical setting in which an individual has had a history of a proliferative disease, particularly cancer, and generally (but not necessarily) been responsive to therapy, which includes, but is not limited to, surgery (such as surgical resection), radiotherapy, and chemotherapy. However, because of their history of the proliferative disease (such as cancer), these individuals are considered at risk of development of the disease. Treatment or administration in the "adjuvant setting" refers to a subsequent mode of treatment. The degree of risk (i.e., when an individual in the adjuvant setting is considered as "high risk" or "low risk") depends upon several factors, most usually the extent of disease when first treated.

The methods provided herein may also be practiced in a "neoadjuvant setting," i.e., the method may be carried out before the primary/definitive therapy. In some embodiments, the individual has previously been treated. In some embodiments, the individual has not previously been treated. In some embodiments, the treatment is a first line therapy.

It is understood that aspect and embodiments of the invention described herein include "consisting" and/or "consisting essentially of" aspects and embodiments.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

### Methods of combination therapy

The present invention provides methods of treating a proliferative disease (such as cancer) in an individual, comprising administering to the individual: a) an effective amount of a composition comprising nanoparticles (such as paclitaxel) and a carrier protein (such as albumin); and b) an effective amount of at least one other agent that modifies the epigenetics in a cell.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of at least one other agent that modifies the epigenetics in a cell, wherein the nanoparticle composition and the other agent are administered concurrently. In some embodiments, the administrations of the nanoparticle composition and the other agent are initiated at about the same time (for example, within any one of 1, 2, 3, 4, 5, 6, or 7 days). In some embodiments, the administrations of the nanoparticle composition and the other agent are terminated at about the same time (for example, within any one of 1, 2, 3, 4, 5, 6, or 7 days). In some embodiments, the administration of the other agent continues (for example for about any one of 0.5, 1, 2, 3, 4, 5, 6, 7, 8, 9, 10, 11, or 12 months) after the termination of the administration of the nanoparticle composition. In some embodiments, the administration of the other agent is initiated after (for example after about any one of 0.5, 1, 2, 3, 4, 5, 6, 7, 8, 9, 10, 11, or 12 months) the initiation of the administration of the nanoparticle composition. In some embodiments, the administrations of the nanoparticle composition and the other agent are initiated and terminated at about the same time. In some embodiments, the administrations of the nanoparticle composition and the other agent are initiated at about the same time and the administration of the other agent continues (for example for about any one of 0.5, 1, 2, 3, 4, 5, 6, 7, 8, 9, 10, 11, or 12 months) after the termination of the administration of the nanoparticle composition. In some embodiments, the administration of the nanoparticle composition and the other agent stop at about the same time and the administration of the other agent is initiated after (for example after about any one of 0.5, 1, 2, 3, 4, 5, 6, 7, 8, 9, 10, 11, or 12 months) the initiation of the administration of the nanoparticle composition. In some embodiments, the administration of the nanoparticle composition and the other agent stop at about the same time and the administration of the nanoparticle composition is initiated after (for example after about any one of 0.5, 1, 2, 3, 4, 5, 6, 7, 8, 9, 10, 11, or 12 months) the initiation of the administration of the other agent.

In some embodiments, the taxane is any of (and in some embodiments consisting essentially of) paclitaxel, docetaxel, and ortataxel. In some embodiments, the taxane is paclitaxel. In some embodiments, the taxane is docetaxel. In some embodiments, the nanoparticle composition comprises Abraxane^{®}. In some embodiments, the nanoparticle composition is Abraxane^{®}.

Thus, for example, in some embodiments, there is provided a method of treating a proliferative disease (such as cancer) in an individual, comprising administering to the individual: a) an effective amount of a composition comprising nanoparticles comprising a taxane (such as paclitaxel) coated with a carrier protein (such as albumin); and b) an effective amount of at least one other agent that modifies the epigenetics in a cell. In some embodiments, the nanoparticles have an average size of 20-400 nm, such as 40-200 nm. In some embodiments, there is provided a method of treating a proliferative disease (such as cancer) in an individual, comprising administering to the individual: a) an effective amount Abraxane^{®}; and b) an effective amount of at least one other agent that modifies the epigenetics in a cell. In some embodiments, the nanoparticle composition (such as Abraxane^{®}) and the other agent are administered concurrently. In some embodiments, the proliferative disease is a cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma.

In some embodiments, the other agent modifies DNA methylation or histone modification. In some embodiments, the other agent modifies (such as inhibits) DNA methylation. In some embodiments, the other agent modifies histone modification, which include, but not limited to, histone acetylation, histone methylation, histone sumoylation, and histone phosphorylation. In some embodiments, the other agent is an inhibitor of DNA methyltransferase (such as azacitidine). In some embodiments, the other agent is an inhibitor of DNA methyltransferase (such as decitabine). In some embodiments, the other agent is an inhibitor of a histone deacetylase (such as vorinostat).

Thus, for example, in some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that inhibits DNA methylation. In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of an inhibitor of DNA methyltransferase. Suitable agents that modify DNA methylation include, but are not limited to, 5-azacytidine (azacitidine or Vidaza), 5-aza-2'-deoxycytidine, 1-β-D-arabinofuranosil-5-azacytosine, dihydro-5-azacytidine, antisense oligonucleotide MG98, and zebularine.

In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies histone modification. In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies histone acetylation. In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies histone methylation. In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies histone sumoylation. In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies histone phosphorylation.

In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of an inhibitor of a histone deacetylase ("HDAC"). HDACs are classified in four groups based on their homology to yeast histone deacetylases. Class I includes HDAC1, -2, -3 and -8, which are related to yeast RPD3 gene. Class II includes HDAC4, -5, -6, -7, -9 and -10, which are related to yeast Hda1 gene. Class III, also known as the sirtuins, are related to the Sir2 gene and includes SIRT1, SIRT2, SIRT3, SIRT4, SIRT5, SIRT6, and SIRT7. Class IV, which contains only HDAC11, has features of both Class I and II. The histone deacetylase inhibitors described herein in some embodiments are specific to only one specific HDAC. In some embodiments, the histone deacetylase inhibitor is specific to one specific class of HDAC. In some embodiments, the histone deacetylase inhibitor is an inhibitor of two or more HDACs or two or more classes of HDACs. In some embodiments, the histone deacetylase inhibitor inhibits class I and II HDACs. In some embodiments, the histone deacetylase inhibitor inhibits class III HDAC.

In some embodiments, the other agent is a hydroxamic acid, including, but not limited to, vorinostat (suberoylanilide hydroxamic acid or "SAHA"), trichostatin A ("TSA"), LBH589 (panobinostat), PXD101 (belinostat), oxamflatin, tubacin, seriptaid, NVP-LAQ824, cinnamic acid hydroxamic acid (CBHA), CBHA derivatives, and ITF2357.

Vorinostat (rINN) or suberoylanilide hydroxamic acid (SAHA) is an inhibitor of histone deacetylases (HDAC). It is marketed under the name Zolinza for the treatment of cutaneous T cell lymphoma (CTCL) when the disease persists, gets worse, or comes back during or after treatment with other medicines.

Trichostatin A (TSA, 7-[4-(dimethylamino)phenyl]-N-hydroxy-4,6-dimethyl-7-oxohepta-2,4-dienamide) is an organic compound that selectively inhibits the class I and II mammalian histone deacetylase (HDAC) families of enzymes, but not class III HDACs (i.e., Sirtuins). TSA inhibits the eukaryotic cell cycle during the beginning of the growth stage.

In some embodiments, the other agent is a cyclic peptide, including, but not limited to, trapoxin B FK228 (romidepsin), trapoxin A, apicidin, depsipeptide, and CHAP.

In some embodiments, the other agent is a benzamide, including, but not limited to, mocetinostat (MGCD0103), benzamide M344, BML-210, entinostat (SNDX-275 or MS-275), pimelic diphenylamide 4b, pimelic diphenylamide 106, MS-994, CI-994 (acetyldinaline, PD 123654, and 4-acetylamino-N-(Uaminophenyl)-benzamide. Mocetinostat (*N*-(2-Aminophenyl)-4-[[(4-pyridin-3-ylpyrimidin-2-yl)amino]methyl] benzamide) works by inhibiting mainly histone deacetylase 1 (HDAC1). It also inhibits HDAC2, HDAC3, and HDAC11.

In some embodiments, the other agent is an electrophilic ketone, including, but not limited to, trifluoromethyl ketones and ketoamides.

In some embodiments, the other agent is an aliphatic acid compound, including, but not limited to, butyrate, phenylbutyrate, valproic acid (vpa), and phenylacetate.

In some embodiments, the other agent is selected from the group consisting of vorinostat (SAHA), belinostat (PXD101), LAQ824, panobinostat (LBH589); entinostat (MS275), CI994, and mocetinostat (MGCD0103). In some embodiments, the other agent is selected from the group consisting of vorinostat, romidepsin, panobinostat, valproic acid, and mocetinostat.

The other agents described herein can be the agents themselves, pharmaceutically acceptable salts thereof, and pharmaceutically acceptable esters thereof, as well as stereoisomer, enantiomers, racemic mixtures, and the like. The other agent or agents as described can be administered as well as a pharmaceutical composition containing the agent(s), wherein the pharmaceutical composition comprises a pharmaceutically acceptable carrier vehicle, or the like.

Reference to an agent herein applies to the other agent or its derivatives and accordingly the invention contemplates and includes either of these embodiments (agent; agent or derivative(s)). "Derivatives" or "analogs" of an agent or other chemical moiety include, but are not limited to, compounds that are structurally similar to the other agent or moiety or are in the same general chemical class as the other agent or moiety. In some embodiments, the derivative or analog of the other agent or moiety retains similar chemical and/or physical property (including, for example, functionality) of the other agent or moiety.

In some embodiments, the other agent is vorinostat. Thus, for example, in some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of vorinostat. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}); and b) an effective amount of vorinostat. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of vorinostat, wherein the nanoparticle composition is administered intravenously, wherein the vorinostat is administered orally. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}); and b) an effective amount of vorinostat wherein the nanoparticle composition is administered intravenously and wherein the vorinostat is administered orally. In some embodiments, the nanoparticle composition and the vorinostat are administered concurrently. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² for example about 100 mg/m²), and b) about 50-1000 mg/day (including for example about 200-500, such as 400 mg/day) vorinostat. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}), wherein the taxane is in the dosage range of about 60-300 mg/m² (including for example about 80-200 mg/m² for example about 100 mg/m²), and b) about 50-1000 mg/day (including for example about 200-500, such as 400 mg/day) vorinostat. In some embodiments, the nanoparticle composition is administered intravenously. In some embodiments, the vorinostat is administered orally. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of a DNA methyltransferase inhibitor. In some embodiments, there is provided a method of treating cancer (such as breast cancer, including triple negative breast cancer) in an individual, comprising administering to the individual: a) an effective amount of a composition comprising nanoparticles comprising a taxane (such as paclitaxel) and a carrier protein (such as albumin); and b) an effective amount of a DNA methyltransferase inhibitor. In some embodiments, there is provided a method of treating cancer (such as breast cancer, including triple negative breast cancer) in an individual, comprising administering to the individual: a) an effective amount of a composition comprising nanoparticles comprising paclitaxel and albumin; and b) an effective amount of azacitidine. In some embodiments, there is provided a method of treating cancer (such as breast cancer, including triple negative breast cancer) in an individual, comprising: a) intravenously administering an effective amount of a composition comprising nanoparticles comprising paclitaxel and albumin; b) intravenously or subcutaneously administering an effective amount of azacitidine.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of azacitidine. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}); and b) an effective amount of azacitidine. In some embodiments, the nanoparticle composition and the azacitidine are administered concurrently. In some embodiments, the nanoparticle composition is administered intravenously. In some embodiments, the azacitidine is administered intravenously or subcutaneously. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of azacitidine, wherein the nanoparticle composition and the azacitidine are administered intravenously. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}); and b) an effective amount of azacitidine. In some embodiments, the nanoparticle composition is administered intravenously. In some embodiments, the azacitidine is administered intravenously or subcutaneously. In some embodiments, the nanoparticle composition and the azacitidine are administered concurrently. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² for example about 100 mg/m²), and b) about 5-500 mg/m² (including for example about 10-200 mg/m² for example about 50-100 mg/m² or for example about 75 mg/m²) azacitidine. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}), wherein the taxane is in the dosage range of about 60-300 mg/m² (including for example about 80-200 mg/m² for example about 100 mg/m²), and b) about 5-500 mg/m² (including for example about 10-200 mg/m² for example about 50-100 mg/m² for example about 75 mg/m²) azacitidine. In some embodiments, the nanoparticle composition is administered intravenously. In some embodiments, the azacitidine is administered intravenously or subcutaneously. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about100 mg/m²); b) intravenously or subcutaneously administering about 20 to about 200 mg/m² (such as about 50-100 mg/m² or for example about 75 mg/m²) azacitidine. In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about100 mg/m²) weekly; b) intravenously or subcutaneously administering about 20 to about 200 mg/m² (such as about 50-100 mg/ m², for example about 75 mg/m²) azacitidine daily. In some embodiments, the administrations of the nanoparticle composition and the azacitidine are concurrent. In some embodiments, the nanoparticle composition is administered three out of four weeks and the azacitidine is administered on days 1-5 on a four week cycle.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of a DNA methyltransferase inhibitor. In some embodiments, there is provided a method of treating cancer (such as breast cancer, including triple negative breast cancer) in an individual, comprising administering to the individual: a) an effective amount of a composition comprising nanoparticles comprising a taxane (such as paclitaxel) and a carrier protein (such as albumin); and b) an effective amount of a DNA methyltransferase inhibitor. In some embodiments, there is provided a method of treating cancer (such as breast cancer, including triple negative breast cancer) in an individual, comprising administering to the individual: a) an effective amount of a composition comprising nanoparticles comprising paclitaxel and albumin; and b) an effective amount of decitabine. In some embodiments, there is provided a method of treating cancer (such as breast cancer, including triple negative breast cancer) in an individual, comprising: a) intravenously administering an effective amount of a composition comprising nanoparticles comprising paclitaxel and albumin; b) intravenously or intraperitoneally administering an effective amount of azacitidine.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of decitabine. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}); and b) an effective amount of decitabine. In some embodiments, the nanoparticle composition and the decitabine are administered concurrently. In some embodiments, the nanoparticle composition is administered intravenously or intraperitoneally. In some embodiments, the decitabine is administered intravenously or intraperitoneally. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of decitabine, wherein the nanoparticle composition and the decitabine are administered intravenously. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}); and b) an effective amount of decitabine. In some embodiments, the nanoparticle composition is administered intravenously. In some embodiments, the decitabine is administered intravenously or intraperitoneally. In some embodiments, the nanoparticle composition and the decitabine are administered concurrently. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer.

In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² for example about 100 mg/m²), and b) about 5-500 mg/ m² (including for example about 10-200 mg/m² for example about 50-100 mg/m²) decitabine. In some embodiments, there is provided a method of treating a proliferative disease (such as cancer) in an individual, comprising administering to the individual: a) an effective amount of a composition comprising nanoparticles comprising paclitaxel coated with an albumin (such as Abraxane^{®}), wherein the taxane is in the dosage range of about 60-300 mg/m² (including for example about 80-200 mg/m² for example about 100 mg/m²), and b) about 5-500 mg/m² (including for example about 10-200 mg/m² for example about 50-100 mg/m²) decitabine. In some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² for example about 100 mg/m²), and b) about 5-500 mg/ m² (including for example about 10-200 mg/m² for example about 15-20 mg/m²) decitabine. In some embodiments, the nanoparticle composition is administered intravenously. In some embodiments, the decitabine is administered intravenously or intraperitoneally. In some embodiments, the proliferative disease is cancer selected from the group consisting of breast cancer, lung cancer (such as small cell lung cancer and non-small cell lung cancer), renal cancer, bladder cancer, pancreatic cancer, ovarian cancer, prostate cancer, brain cancer, colorectal cancer, leukemia, lymphoma, and multiple myeloma. In some embodiments, the cancer is breast cancer, such as triple negative breast cancer. In some embodiments, the cancer is luminal B type breast cancer. In some embodiments, the cancer is ovarian cancer.

In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about100 mg/m²); b) intravenously or intraperitoneally administering about 5 to about 200 mg/m² (such as about 15-20 mg/m²) decitabine. In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about100 mg/m²) weekly; b) intravenously or intraperitoneally administering about 5 to about 200 mg/m² (such as about 15-20 mg/m²) decitabine daily. In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about100 mg/m²) weekly; b) intravenously or intraperitoneally administering about 5 to about 200 mg/m² (such as about 15-20 mg/m²) decitabine every eight hours for three days. In some embodiments, the nanoparticle composition is administered three out of four weeks and the decitabine is administered on days 1-3 on a six week cycle. In some embodiments, the administrations of the nanoparticle composition and the decitabine are concurrent. In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about 100 mg/m²) weekly; b) intravenously or intraperitoneally administering about 5 to about 200 mg/m² (such as about 15-20 mg/m²) decitabine daily for five days. In some embodiments, the administrations of the nanoparticle composition and the decitabine are concurrent. In some embodiments, the nanoparticle composition is administered three out of four weeks and the decitabine is administered on days 1-5 on a four week cycle.

In some embodiments, there is provided a method of treating cancer (such as ovarian cancer) in an individual, comprising: a) intravenously or intraperitoneally administering a composition comprising nanoparticles comprising paclitaxel and albumin at the dose of about 13-30 mg/kg; b) intravenously or intraperitoneally administering about 0/5 mg/kg to about 4 mg/kg (such as 2 mg/kg) decitabine.

In some embodiments, there is provided a method of treating cancer (such as lung cancer) in an individual, comprising: a) intravenously or intraperitoneally administering a composition comprising nanoparticles comprising paclitaxel and albumin at the dose of about 13-30 mg/kg; b) intravenously or intraperitoneally administering about 1.5 mg/kg 0.5 mg/kg to about 4.0 mg/kg (such as 1.5 mg/kg) decitabine. In some embodiments, there is provided a method of treating lung cancer (such as NSCLC) in an individual, comprising: a) intravenously or intraperitoneally administering a composition comprising nanoparticles comprising paclitaxel and albumin at the dose of about 13-30 mg/kg/day for five consecutive days; b) intravenously or intraperitoneally administering about 0.5 mg/kg/day to about 4.0 mg/kg/day (such as 1.5 mg/kg/day) decitabine, twice a day for five consecutive days.

In some embodiments, there is provided a method of treating cancer (such as lung cancer) in an individual, comprising: a) intravenously or intraperitoneally administering a composition comprising nanoparticles comprising paclitaxel and albumin at the dose of about 60-300 mg/m² (including for example about 80-200 mg/m² for example about 100 mg/m²) b) intravenously or intraperitoneally administering about 5 mg/m² to about 100 mg/m² (such as 15 mg/m²) decitabine. In some embodiments, there is provided a method of treating lung cancer (such as NSCLC) in an individual, comprising: a) intravenously or intraperitoneally administering a composition comprising nanoparticles comprising paclitaxel and albumin at the dose of about 60-300 mg/m²/day for five consecutive days; b) intravenously or intraperitoneally administering about 5 mg/m² to about 100 mg/m² (such as 15 mg/m²) decitabine, three times a day for three consecutive days. In some embodiments, the decitabine administration is repeated every six weeks. In some embodiments, the decitabine administration is repeated every six weeks for at least four cycles.

In some embodiments, there is provided a method of treating cancer (such as lung cancer) in an individual, comprising: a) intravenously or intraperitoneally administering a composition comprising nanoparticles comprising paclitaxel and albumin at the dose of about 60-300 mg/m² (including for example about 80-200 mg/m² for example about 100 mg/m²); b) intravenously or intraperitoneally administering about 5 mg/m² to about 100 mg/m² (such as 20 mg/m²) decitabine. In some embodiments, the decitabine administration is repeated daily for five days. In some embodiments, the decitabine administration is daily for five days, repeated for four cycles.

The methods described herein are suitable for treating various cancers, such as cancers described herein, including a cancer selected from the group consisting of lymphoid neoplasm, ovarian cancer, endometrial cancer, lung cancer, sarcoma, pancreatic cancer, and breast cancer.

Thus, for example, in some embodiments, there is provided a method of treating a lymphoid neoplasm (for example, chronic lymphocytic leukemia/small lymphocytic leukemia (CLL/SLL) or lymphoma, such as refractory diffuse large B-cell (DLBC) lymphoma) in an individual comprising administering to the individual:
a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating a lymphoid neoplasm (for example, CLL/SLL orlymphoma, such as refractory DLBC lymphoma) in an individual comprising administering to the individual: a) an effective amount of a composition comprising nanoparticles comprising a taxane (such as paclitaxel) and a carrier protein (such as albumin), and b) an effective amount of azacitidine. In some embodiments, there is provided a method of treating a lymphoid neoplasm (for example, CLL/SLL or lymphoma, such as refractory DLBC lymphoma) in an individual comprising administering to the individual: a) an effective amount of a composition comprising nanoparticles comprising paclitaxel coated with an albumin (such as Abraxane^{®}), and
b) an effective amount of a DNA methyltransferase inhibitor. In some embodiments, there is provided a method of treating a lymphoid neoplasm (for example, CLL/SLL or lymphoma, such as refractory DLBC lymphoma) in an individual comprising administering to the individual: a) an effective amount of a composition comprising nanoparticles comprising paclitaxel coated with an albumin (such as Abraxane^{®}), and b) an effective amount of azacitidine. In some embodiments, the nanoparticle composition is administered intravenously. In some embodiments, the azacitidine is administered intravenously or subcutaneously. In some embodiments, the lymphoid neoplasm is chronic lymphocytic leukemia or small lymphocytic lymphoma.

In some embodiments the lymphoid neoplasm is a B-cell neoplasm. Examples of B-cell neoplasms include, but are not limited to, precursor B-cell neoplasms (e.g., precursor B-lymphoblastic leukemia/lymphoma) and peripheral B-cell neoplasms (e.g., B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (e.g., cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (e.g., extranodal (e.g., MALT-type +/- monocytoid B cells) and/or Nodal (e.g., +/- monocytoid B cells)), splenic marginal zone lymphoma (e.g., +/- villous lymphocytes), Hairy cell leukemia, plasmacytoma/plasma cell myeloma (e.g., myeloma and multiple myeloma), diffuse large B-cell lymphoma (e.g., primary mediastinal (thymic) B-cell lymphoma), refractory diffuse large B-cell lymphoma, intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia).

In some embodiments the lymphoid neoplasm is a T-cell and/or putative NK-cell neoplasm. Examples of T-cell and/or putative NK-cell neoplasms include, but are not limited to, precursor T-cell neoplasm (precursor T-lymphoblastic lymphoma/leukemia) and peripheral T-cell and NK-cell neoplasms (e.g., T-cell chronic lymphocytic leukemia/prolymphocytic leukemia, and large granular lymphocyte leukemia (LGL) (e.g., T-cell type and/or NK-cell type), cutaneous T-cell lymphoma (e.g., mycosis fungoides/Sezary syndrome), primary T-cell lymphomas unspecified (e.g., cytological categories (e.g., medium-sized cell, mixed medium and large cell), large cell, lymphoepitheloid cell, subtype hepatosplenic .gamma..delta. T-cell lymphoma, and subcutaneous panniculitic T-cell lymphoma), angioimmunoblastic T-cell lymphoma (AILD), angiocentric lymphoma, intestinal T-cell lymphoma (e.g., +/- enteropathy associated), adult T-cell lymphoma/leukemia (ATL), anaplastic large cell lymphoma (ALCL) (e.g., CD30+, T- and null-cell types), anaplastic large-cell lymphoma, and Hodgkin's like).

In some embodiments the lymphoid neoplasm (e.g., lymphoma) is Hodgkin's disease. For example, the Hodgkin's disease may be lymphocyte predominance, nodular sclerosis, mixed cellularity, lymphocyte depletion, and/or lymphocyte-rich.

In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 75 mg/m² azacitidine. In some embodiments, the nanoparticle composition is administered first followed by administration of the azacitidine. In some embodiments, the azacitidine is administered first followed by administration of the nanoparticle composition. In some embodiments, the administration of the nanoparticle composition and the azacitidine are concurrent. In some embodiments, the nanoparticle composition is administered three out of four weeks and the azacitidine is administered on days 1-5 on a four week cycle. In some embodiments, the azacitadine is administered on days 1-5, followed by administration of the nanoparticle composition on days 8, 15, and 22 of a 28-day cycle, for a total of 6 cycles.

In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine, wherein the azacitadine is administered on days 1-5, followed by administration of the nanoparticle composition on days 8, 15, and 22 of a 28-day cycle, for a total of 6 cycles. In some embodiments, there is provided a method of treating a lymphoid neoplasm (for example, CLL/SLL or lymphoma, such as refractory DLBC lymphoma) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) subcutaneously administering to the individual about 50-100 mg/m² (such as 75 mg/m²) azacitidine, wherein the azacitadine is subcutaneously administered on days 1-5, followed by intravenous administration of the nanoparticle composition on days 8, 15, and 22 of a 28-day cycle, for a total of 6 cycles. In some embodiments, the individual suffering from the lymphoid neoplasm (for example, CLL/SLL or lymphoma, such as refractory DLBC lymphoma) has not had prior cytotoxic regimens. In some embodiments, the individual suffering from the lymphoid neoplasm (for example, CLL/SLL or lymphoma, such as refractory DLBC lymphoma) has had no more than 2 prior cytotoxic regimens. In some embodiments, the individual suffering from the lymphoid neoplasm (for example, lymphoma or for example, refractory diffuse large B-cell lymphoma) has had more than 2 prior cytotoxic regimens.

In some embodiments, there is provided a method of treating ovarian cancer in an individual comprising administering to the individual: a) an effective amount of a composition comprising nanoparticles comprising a taxane (such as paclitaxel) and a carrier protein (such as albumin) and b) an effective amount of a DNA methyltransferase inhibitor. In some embodiments, there is provided a method of treating ovarian cancer in an individual comprising administering to the individual: a) an effective amount of a composition comprising nanoparticles comprising a taxane (such as paclitaxel) and a carrier protein (such as albumin), and b) an effective amount of azacitidine. In some embodiments, there is provided a method of treating ovarian cancer in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating ovarian cancer in an individual comprising administering to the individual: a) an effective amount of a composition comprising nanoparticles comprising paclitaxel coated with an albumin (such as Abraxane^{®}), and b) an effective amount of azacitidine. In some embodiments, the nanoparticle composition is administered intravenously. In some embodiments, the azacitidine is administered intravenously or subcutaneously.

In some embodiments, the ovarian cancer is ovarian epithelial cancer. Exemplary ovarian epithelial cancer histological classifications include: serous cystomas (e.g., serous benign cystadenomas, serous cystadenomas with proliferating activity of the epithelial cells and nuclear abnormalities but with no infiltrative destructive growth, or serous cystadenocarcinomas), mucinous cystomas (e.g., mucinous benign cystadenomas, mucinous cystadenomas with proliferating activity of the epithelial cells and nuclear abnormalities but with no infiltrative destructive growth, or mucinous cystadenocarcinomas), endometrioid tumors (e.g., endometrioid benign cysts, endometrioid tumors with proliferating activity of the epithelial cells and nuclear abnormalities but with no infiltrative destructive growth, or endometrioid adenocarcinomas), clear cell (mesonephroid) tumors (e.g., benign clear cell tumors, clear cell tumors with proliferating activity of the epithelial cells and nuclear abnormalities but with no infiltrative destructive growth, or clear cell cystadenocarcinomas), unclassified tumors that cannot be allotted to one of the above groups, or other malignant tumors. In various embodiments, the ovarian epithelial cancer is stage I (e.g., stage IA, IB, or IC), stage II (e.g., stage IIA, IIB, or IIC), stage III (e.g., stage IIIA, IIIB, or IIIC), or stage IV. In some embodiments, the individual may be a human who has a gene, genetic mutation, or polymorphism associated with ovarian cancer (e.g., BRCA1 or BRCA2) or has one or more extra copies of a gene associated with ovarian cancer (e.g., one or more extra copies of the HER2 gene).

In some embodiments, the ovarian cancer is an ovarian germ cell tumor. Exemplary histologic subtypes include dysgerminomas or other germ cell tumors (e.g., endodermal sinus tumors such as hepatoid or intestinal tumors, embryonal carcinomas, olyembryomas, choriocarcinomas, teratomas, or mixed form tumors). Exemplary teratomas are immature teratomas, mature teratomas, solid teratomas, and cystic teratomas (e.g., dermoid cysts such as mature cystic teratomas, and dermoid cysts with malignant transformation). Some teratomas are monodermal and highly specialized, such as struma ovarii, carcinoid, struma ovarii and carcinoid, or others (e.g., malignant neuroectodermal and ependymomas). In some embodiments, the ovarian germ cell tumor is stage I (e.g., stage IA, IB, or IC), stage II (e.g., stage IIA, IIB, or IIC), stage III (e.g., stage IIIA, IIIB, or IIIC), or stage IV.

In some embodiments, there is provided a method of treating ovarian cancer in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating ovarian cancer in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating ovarian cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine. In some embodiments, the nanoparticle composition is administered first followed by administration of the azacitidine. In some embodiments, the azacitidine is administered first followed by administration of the nanoparticle composition. In some embodiments, the administrations of the nanoparticle composition and the azacitidine are concurrent. In some embodiments, the nanoparticle composition is administered three out of four weeks and the azacitidine is administered on the first, second, third, fourth, fifth, or sixth day (such as on days 1-5) on a four week cycle. In some embodiments, the azacitadine is administered on days 1-5, followed by administration of the nanoparticle composition on days 8, 15, and 22 of a 28-day cycle, for a total of 6 cycles.

In some embodiments, there is provided a method of treating ovarian cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine, wherein the azacitadine is administered on days 1-5, followed by administration of the nanoparticle composition on days 8, 15, and 22 of a 28-day cycle, for a total of 6 cycles. In some embodiments, there is provided a method of treating ovarian cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) subcutaneously administering to the individual about 50-100 mg/m² (such as 75 mg/m²) azacitidine, wherein the azacitadine is subcutaneously administered on days 1-5, followed by intravenous administration of the nanoparticle composition on days 8, 15, and 22 of a 28-day cycle, for a total of 6 cycles. In some embodiments, the individual suffering from ovarian cancer has not had prior cytotoxic regimens. In some embodiments, the individual suffering from ovarian cancer has had no more than 2 prior cytotoxic regimens. In some embodiments, the individual suffering from ovarian cancer has had more than 2 prior cytotoxic regimens.

In some embodiments, there is provided a method of treating endometrial cancer (e.g. uterine cancer) in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating endometrial cancer (e.g. uterine cancer) in an individual comprising administering to the individual: a) an effective amount of a composition comprising nanoparticles comprising a taxane (such as paclitaxel) and a carrier protein (such as albumin), and b) an effective amount of azacitidine. In some embodiments, there is provided a method of treating endometrial cancer (e.g. uterine cancer) in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating endometrial cancer (e.g. uterine cancer) in an individual comprising administering to the individual: a) an effective amount of a composition comprising nanoparticles comprising paclitaxel coated with an albumin (such as Abraxane^{®}), and b) an effective amount of azacitidine. In some embodiments, the nanoparticle composition is administered intravenously. In some embodiments, the azacitidine is administered intravenously or subcutaneously.

In some embodiments, the endometrial cancer is uterine papillary serous carcinoma or uterine serous adenocarcinoma. In some embodiments, the endometrial cancer is endometrial stromal sarcoma. In some embodiments the endometrial cancer includes, but is not limited to, endometrial intraepithelial neoplasia, endometrial intraepithelial neoplasm, or endometrial adenocarcinoma.

In some embodiments, there is provided a method of treating endometrial cancer (e.g. uterine cancer) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating endometrial cancer (e.g. uterine cancer) in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating endometrial cancer (e.g. uterine cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²). In some embodiments, the nanoparticle composition is administered first followed by administration of the azacitidine. In some embodiments, the azacitidine is administered first followed by administration of the nanoparticle composition. In some embodiments, the administrations of the nanoparticle composition and the azacitidine are concurrent. In some embodiments, the nanoparticle composition is administered three out of four weeks and the azacitidine is administered on the first, second, third, fourth, fifth, or sixth day (such as on days 1-5) on a four week cycle. In some embodiments, the azacitadine is administered on days 1-5, followed by administration of the nanoparticle composition on days 8, 15, and 22 of a 28-day cycle, for a total of 6 cycles.

In some embodiments, there is provided a method of treating endometrial cancer (e.g. uterine cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine, wherein the azacitadine is administered on days 1-5, followed by administration of the nanoparticle composition on days 8, 15, and 22 of a 28-day cycle, for a total of 6 cycles. In some embodiments, there is provided a method of treating endometrial cancer (e.g. uterine cancer) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) subcutaneously administering to the individual about 50-100 mg/m² (such as 75 mg/m²) azacitidine, wherein the azacitadine is subcutaneously administered on days 1-5, followed by intravenous administration of the nanoparticle composition on days 8, 15, and 22 of a 28-day cycle, for a total of 6 cycles. In some embodiments, the individual suffering from endometrial cancer (e.g. uterine cancer) has not had prior cytotoxic regimens. In some embodiments, the individual suffering from endometrial cancer (e.g. uterine cancer) has had no more than 2 prior cytotoxic regimens. In some embodiments, the individual suffering from endometrial cancer (e.g. uterine cancer) has had more than 2 prior cytotoxic regimens.

In some embodiments, there is provided a method of treating lung cancer in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating lung cancer in an individual comprising administering to the individual: a) an effective amount of a composition comprising nanoparticles comprising a taxane (such as paclitaxel) and a carrier protein (such as albumin), and b) an effective amount of azacitidine. In some embodiments, there is provided a method of treating lung cancer in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating lung cancer in an individual comprising administering to the individual: a) an effective amount of a composition comprising nanoparticles comprising paclitaxel coated with an albumin (such as Abraxane^{®}), and b) an effective amount of azacitidine. In some embodiments, the nanoparticle composition is administered intravenously. In some embodiments, the azacitidine is administered intravenously or subcutaneously.

In some embodiments, the lung cancer is a non-small cell lung cancer (NSCLC). Examples of NCSLC include, but are not limited to, large-cell carcinoma (e.g., large-cell neuroendocrine carcinoma, combined large-cell neuroendocrine carcinoma, basaloid carcinoma, lymphoepithelioma-like carcinoma, clear cell carcinoma, and large-cell carcinoma with rhabdoid phenotype), adenocarcinoma (e.g., acinar, papillary (e.g., bronchioloalveolar carcinoma, nonmucinous, mucinous, mixed mucinous and nonmucinous and indeterminate cell type), solid adenocarcinoma with mucin, adenocarcinoma with mixed subtypes, well-differentiated fetal adenocarcinoma, mucinous (colloid) adenocarcinoma, mucinous cystadenocarcinoma, signet ring adenocarcinoma, and clear cell adenocarcinoma), neuroendocrine lung tumors, and squamous cell carcinoma (e.g., papillary, clear cell, small cell, and basaloid). In some embodiments, the NSCLC may be, according to TNM classifications, a stage T tumor (primary tumor), a stage N tumor (regional lymph nodes), or a stage M tumor (distant metastasis).

In some embodiments, the lung cancer is a carcinoid (typical or atypical), adenosquamous carcinoma, cylindroma, or carcinoma of the salivary gland (e.g., adenoid cystic carcinoma or mucoepidermoid carcinoma). In some embodiments, the lung cancer is a carcinoma with pleomorphic, sarcomatoid, or sarcomatous elements (e.g., carcinomas with spindle and/or giant cells, spindle cell carcinoma, giant cell carcinoma, carcinosarcoma, or pulmonary blastoma). In some embodiments, the lung cancer is small cell lung cancer (SCLC; also called oat cell carcinoma). The small cell lung cancer may be limited-stage, extensive stage or recurrent small cell lung cancer. In some embodiments, the individual may be a human who has a gene, genetic mutation, or polymorphism suspected or shown to be associated with lung cancer (e.g., SASH1, LATS1, IGF2R, PARK2, KRAS, PTEN, Kras2, Krag, Pas1, ERCC1, XPD, IL8RA, EGFR, α₁-AD, EPHX, MMP1, MMP2, MMP3, MMP12, ILIβ, RAS, and/or AKT) or has one or more extra copies of a gene associated with lung cancer.

In some embodiments, there is provided a method of treating lung cancer in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating lung cancer in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating lung cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine. In some embodiments, the nanoparticle composition is administered first followed by administration of the azacitidine. In some embodiments, the azacitidine is administered first followed by administration of the nanoparticle composition. In some embodiments, the administrations of the nanoparticle composition and the azacitidine are concurrent. In some embodiments, the nanoparticle composition is administered three out of four weeks and the azacitidine is administered on the first, second, third, fourth, fifth, or sixth day (such as on days 1-5) on a four week cycle. In some embodiments, the azacitadine is administered on days 1-5, followed by administration of the nanoparticle composition on days 8, 15, and 22 of a 28-day cycle, for a total of 6 cycles.

In some embodiments, there is provided a method of treating lung cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine, wherein the azacitadine is administered on days 1-5, followed by administration of the nanoparticle composition on days 8, 15, and 22 of a 28-day cycle, for a total of 6 cycles. In some embodiments, there is provided a method of treating lung cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) subcutaneously administering to the individual about 50-100 mg/m² (such as 75 mg/m²) azacitidine, wherein the azacitadine is subcutaneously administered on days 1-5, followed by intravenous administration of the nanoparticle composition on days 8, 15, and 22 of a 28-day cycle, for a total of 6 cycles. In some embodiments, the individual suffering from lung cancer has not had prior cytotoxic regimens. In some embodiments, the individual suffering from lung cancer has had no more than 2 prior cytotoxic regimens. In some embodiments, the individual suffering from lung cancer has had more than 2 prior cytotoxic regimens.

In some embodiments, there is provided a method of treating sarcoma in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating sarcoma in an individual comprising administering to the individual: a) an effective amount of a composition comprising nanoparticles comprising a taxane (such as paclitaxel) and a carrier protein (such as albumin), and b) an effective amount of azacitidine. In some embodiments, there is provided a method of treating sarcoma in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating sarcoma in an individual comprising administering to the individual: a) an effective amount of a composition comprising nanoparticles comprising paclitaxel coated with an albumin (such as Abraxane^{®}), and b) an effective amount of azacitidine. In some embodiments, the nanoparticle composition is administered intravenously. In some embodiments, the azacitidine is administered intravenously or subcutaneously.

In some embodiments, the sarcoma includes, but is not limited to, sarcomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, Kaposi's sarcoma, soft tissue sarcoma, uterine sacronomasynovioma, mesothelioma, Ewing's tumor, leiomyosarcoma, or rhabdomyosarcoma.

In some embodiments, there is provided a method of treating sarcoma in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating sarcoma in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating sarcoma in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine. In some embodiments, the nanoparticle composition is administered first followed by administration of the azacitidine. In some embodiments, the azacitidine is administered first followed by administration of the nanoparticle composition. In some embodiments, the administrations of the nanoparticle composition and the azacitidine are concurrent. In some embodiments, the nanoparticle composition is administered three out of four weeks and the azacitidine is administered on the first, second, third, fourth, fifth, or sixth day (such as on days 1-5) on a four week cycle. In some embodiments, the azacitadine is administered on days 1-5, followed by administration of the nanoparticle composition on days 8, 15, and 22 of a 28-day cycle, for a total of 6 cycles.

In some embodiments, there is provided a method of treating sarcoma in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine, wherein the azacitadine is administered on days 1-5, followed by administration of the nanoparticle composition on days 8, 15, and 22 of a 28-day cycle, for a total of 6 cycles. In some embodiments, there is provided a method of treating sarcoma in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) subcutaneously administering to the individual about 50-100 mg/m² (such as 75 mg/m²) azacitidine, wherein the azacitadine is subcutaneously administered on days 1-5, followed by intravenous administration of the nanoparticle composition on days 8, 15, and 22 of a 28-day cycle, for a total of 6 cycles. In some embodiments, the individual suffering from sarcoma has not had prior cytotoxic regimens. In some embodiments, the individual suffering from sarcoma has had no more than 2 prior cytotoxic regimens. In some embodiments, the individual suffering from sarcoma has had more than 2 prior cytotoxic regimens.

In some embodiments, there is provided a method of treating pancreatic cancer in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating pancreatic cancer in an individual comprising administering to the individual: a) an effective amount of a composition comprising nanoparticles comprising a taxane (such as paclitaxel) and a carrier protein (such as albumin), and b) an effective amount of azacitidine. In some embodiments, there is provided a method of treating pancreatic cancer in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating pancreatic cancer in an individual comprising administering to the individual: a) an effective amount of a composition comprising nanoparticles comprising paclitaxel coated with an albumin (such as Abraxane^{®}), and b) an effective amount of azacitidine. In some embodiments, the nanoparticle composition is administered intravenously. In some embodiments, the azacitidine is administered intravenously or subcutaneously.

In some embodiments, the pancreatic cancer includes, but is not limited to, serous microcystic adenoma, intraductal papillary mucinous neoplasm, mucinous cystic neoplasm, solid pseudopapillary neoplasm, pancreatic adenocarcinoma, pancreatic ductal carcinoma, or pancreatoblastoma.

In some embodiments, there is provided a method of treating pancreatic cancer in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 50-100 mg/m²) azacitidine. In some embodiments, there is provided a method of treating pancreatic cancer in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating pancreatic cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine. In some embodiments, the nanoparticle composition is administered first followed by administration of the azacitidine. In some embodiments, the azacitidine is administered first followed by administration of the nanoparticle composition. In some embodiments, the administrations of the nanoparticle composition and the azacitidine are concurrent. In some embodiments, the nanoparticle composition is administered three out of four weeks and the azacitidine is administered on the first, second, third, fourth, fifth, or sixth day (such as on days 1-5) on a four week cycle. In some embodiments, the azacitadine is administered on days 1-5, followed by administration of the nanoparticle composition on days 8, 15, and 22 of a 28-day cycle, for a total of 6 cycles.

In some embodiments, there is provided a method of treating pancreatic cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine, wherein the azacitadine is administered on days 1-5, followed by administration of the nanoparticle composition on days 8, 15, and 22 of a 28-day cycle, for a total of 6 cycles. In some embodiments, there is provided a method of treating pancreatic cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) subcutaneously administering to the individual about 50-100 mg/m² (such as 75 mg/m²) azacitidine, wherein the azacitadine is subcutaneously administered on days 1-5, followed by intravenous administration of the nanoparticle composition on days 8, 15, and 22 of a 28-day cycle, for a total of 6 cycles. In some embodiments, the individual suffering from pancreatic cancer has not had prior cytotoxic regimens. In some embodiments, the individual suffering from pancreatic cancer has had no more than 2 prior cytotoxic regimens. In some embodiments, the individual suffering from pancreatic cancer has had more than 2 prior cytotoxic regimens.

In some embodiments, there is provided a method of treating breast cancer (for example, HER2 negative breast cancer or for example, triple negative breast cancer) in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating breast cancer (for example, HER2 negative breast cancer or for example, triple negative breast cancer) in an individual comprising administering to the individual: a) an effective amount of a composition comprising nanoparticles comprising a taxane (such as paclitaxel) and a carrier protein (such as albumin), and b) an effective amount of azacitidine. In some embodiments, there is provided a method of treating breast cancer (for example, HER2 negative breast cancer or for example, triple negative breast cancer) in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating breast cancer (for example, HER2 negative breast cancer or for example, triple negative breast cancer) in an individual comprising administering to the individual: a) an effective amount of a composition comprising nanoparticles comprising paclitaxel coated with an albumin (such as Abraxane^{®}), and b) an effective amount of azacitidine. In some embodiments, the nanoparticle composition is administered intravenously. In some embodiments, the azacitidine is administered intravenously or subcutaneously.

In some embodiments, the breast cancer is early stage breast cancer, non-metastatic breast cancer, stage IV breast cancer, locally advanced breast cancer, metastatic breast cancer, hormone receptor positive metastatic breast cancer, breast cancer in remission, breast cancer in an adjuvant setting, ductal carcinoma in situ (DCIS), invasive ductal carcinoma (IDC), or breast cancer in a neoadjuvant setting. In some embodiments, the breast cancer is hormone receptor positive metastatic breast cancer. In some embodiments, the breast cancer (which may be HER2 positive or HER2 negative) is advanced breast cancer. In some embodiments, the breast cancer is ductal carcinoma in situ. In some embodiments, the individual may be a human who has a gene, genetic mutation, or polymorphism associated with breast cancer *(e.g.,* BRCA1, BRCA2, ATM, CHEK2, RAD51, AR, DIRAS3, ERBB2, TP53, AKT, PTEN, and/or PI3K) or has one or more extra copies of a gene (*e.g.,* one or more extra copies of the HER2 gene) associated with breast cancer.

In some embodiments, there is provided a method of treating breast cancer (for example, HER2 negative breast cancer or for example, triple negative breast cancer) in an individual, comprising administering to the individual: a) an effective amount of a composition comprising nanoparticles comprising a taxane (such as paclitaxel) and albumin, wherein the taxane is in the dosage range of about 60-300 mg/m² (including for example about 80-200 mg/m² or for example about 100 mg/m²), and b) about 5-500 mg/m² (including for example about 10-200 mg/m² or for example about 50-100 mg/m²) azacitidine. In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 50-100 mg/m² or for example about 75 mg/m²) azacitidine. In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine. In some embodiments, the nanoparticle composition is administered first followed by administration of the azacitidine. In some embodiments, the azacitidine is administered first followed by administration of the nanoparticle composition. In some embodiments, the administrations of the nanoparticle composition and the azacitidine are concurrent. In some embodiments, the nanoparticle composition is administered three out of four weeks and the azacitidine is administered on the first, second, third, fourth, fifth, or sixth day (such as on days 1-5) on a four week cycle. In some embodiments, the azacitadine is administered on days 1-5, followed by administration of the nanoparticle composition on days 8, 15, and 22 of a 28-day cycle, for a total of 6 cycles.

In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 50 mg/m² to about 100 mg/m² (such as 75 mg/m²) azacitidine, wherein the azacitadine is administered on days 1-5 followed by administration of the nanoparticle composition on days 8, 15, and 22 of a 28-day cycle, for a total of 6 cycles. In some embodiments, there is provided a method of treating breast cancer (for example, HER2 negative breast cancer or for example, triple negative breast cancer) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) subcutaneously administering to the individual about 50-100 mg/m² (such as 75 mg/m²) azacitidine, wherein the azacitadine is subcutaneously administered on days 1-5, followed by intravenous administration of the nanoparticle composition on days 8, 15, and 22 of a 28-day cycle, for a total of 6 cycles. In some embodiments, the individual suffering from breast cancer (for example, HER2 negative breast cancer or for example, triple negative breast cancer) has not had prior cytotoxic regimens. In some embodiments, the individual suffering from breast cancer (for example, HER2 negative breast cancer or for example, triple negative breast cancer) has had no more than 2 prior cytotoxic regimens. In some embodiments, the individual suffering from breast cancer (for example, HER2 negative breast cancer or for example, triple negative breast cancer) has had more than 2 prior cytotoxic regimens.

In some embodiments, there is provided a method of treating a lymphoid neoplasm (for example, chronic lymphocytic leukemia/small lymphocytic leukemia (CLL/SLL) or lymphoma, such as refractory diffuse large B-cell (DLBC) lymphoma) in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating a lymphoid neoplasm (for example, CLL/SLL orlymphoma, such as refractory DLBC lymphoma) in an individual comprising administering to the individual: a) an effective amount of a composition comprising nanoparticles comprising a taxane (such as paclitaxel) and a carrier protein (such as albumin), and b) an effective amount of decitabine. In some embodiments, there is provided a method of treating a lymphoid neoplasm (for example, CLL/SLL or lymphoma, such as refractory DLBC lymphoma) in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating a lymphoid neoplasm (for example, CLL/SLL or lymphoma, such as refractory DLBC lymphoma) in an individual comprising administering to the individual: a) an effective amount of a composition comprising nanoparticles comprising paclitaxel coated with an albumin (such as Abraxane^{®}), and b) an effective amount of decitabine. In some embodiments, the nanoparticle composition is administered intravenously. In some embodiments, the decitabine is administered intravenously or intraperitoneally. In some embodiments, the lymphoid neoplasm is chronic lymphocytic leukemia or small lymphocytic lymphoma.

In some embodiments the lymphoid neoplasm is a B-cell neoplasm. Examples of B-cell neoplasms include, but are not limited to, precursor B-cell neoplasms (e.g., precursor B-lymphoblastic leukemia/lymphoma) and peripheral B-cell neoplasms (e.g., B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (e.g., cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (e.g., extranodal (e.g., MALT-type +/- monocytoid B cells) and/or Nodal (e.g., +/- monocytoid B cells)), splenic marginal zone lymphoma (e.g., +/- villous lymphocytes), Hairy cell leukemia, plasmacytoma/plasma cell myeloma (e.g., myeloma and multiple myeloma), diffuse large B-cell lymphoma (e.g., primary mediastinal (thymic) B-cell lymphoma), refractory diffuse large B-cell lymphoma, intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia).

In some embodiments the lymphoid neoplasm is a T-cell and/or putative NK-cell neoplasm. Examples of T-cell and/or putative NK-cell neoplasms include, but are not limited to, precursor T-cell neoplasm (precursor T-lymphoblastic lymphoma/leukemia) and peripheral T-cell and NK-cell neoplasms (e.g., T-cell chronic lymphocytic leukemia/prolymphocytic leukemia, and large granular lymphocyte leukemia (LGL) (e.g., T-cell type and/or NK-cell type), cutaneous T-cell lymphoma (e.g., mycosis fungoides/Sezary syndrome), primary T-cell lymphomas unspecified (e.g., cytological categories (e.g., medium-sized cell, mixed medium and large cell), large cell, lymphoepitheloid cell, subtype hepatosplenic .gamma..delta. T-cell lymphoma, and subcutaneous panniculitic T-cell lymphoma), angioimmunoblastic T-cell lymphoma (AILD), angiocentric lymphoma, intestinal T-cell lymphoma (e.g., +/- enteropathy associated), adult T-cell lymphoma/leukemia (ATL), anaplastic large cell lymphoma (ALCL) (e.g., CD30+, T- and null-cell types), anaplastic large-cell lymphoma, and Hodgkin's like).

In some embodiments the lymphoid neoplasm (e.g., lymphoma) is Hodgkin's disease. For example, the Hodgkin's disease may be lymphocyte predominance, nodular sclerosis, mixed cellularity, lymphocyte depletion, and/or lymphocyte-rich.

In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15-20 mg/m²) decitabine. In some embodiments, the nanoparticle composition is administered first followed by administration of the decitabine. In some embodiments, the decitabine is administered first followed by administration of the nanoparticle composition. In some embodiments, the administration of the nanoparticle composition and the decitabine are concurrent. In some embodiments, the nanoparticle composition is administered three out of four weeks and the decitiabine is administered on three times a day on days 1-3 on a six week cycle. In some embodiments, the nanoparticle composition is administered three out of four weeks and the decitabine is administered on days 1-5 on a four week cycle. In some embodiments, the decitabine is administered three times a day on days 1-3, followed by administration of the nanoparticle composition on days 8, 15, and 22 of a 28-day cycle, for a total of 6 cycles. In some embodiments, the decitabine is administered on days 1-5, followed by administration of the nanoparticle composition on days 8, 15, and 22 of a 28-day cycle, for a total of 6 cycles.

In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine, wherein the decitabine is administered every eight hours on days 1-3, followed by administration of the nanoparticle composition on days 8, 15, and 22 of a 28-day cycle, for a total of 6 cycles. In some embodiments, there is provided a method of treating a lymphoid neoplasm (for example, CLL/SLL or lymphoma, such as refractory DLBC lymphoma) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine, wherein the decitabine is administered on days 1-5, followed by administration of the nanoparticle composition on days 8, 15, and 22 of a 28-day cycle, for a total of 6 cycles. In some embodiments, there is provided a method of treating a lymphoid neoplasm (for example, CLL/SLL or lymphoma, such as refractory DLBC lymphoma) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5 mg/m² to about 100 mg/m² (such as 15 mg/m²) decitabine wherein the decitabine is intravenously administered every eight hours on days 1-3, followed by intravenous administration of the nanoparticle composition on days 8, 15, and 22 of a 28-day cycle, for a total of 6 cycles. In some embodiments, there is provided a method of treating a lymphoid neoplasm (for example, CLL/SLL or lymphoma, such as refractory DLBC lymphoma) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5 mg/m² to about 100 mg/m² (such as 20 mg/m²) decitabine wherein the decitabine is intravenously administered on days 1-5, followed by intravenous administration of the nanoparticle composition on days 8, 15, and 22 of a 28-day cycle, for a total of 6 cycles. In some embodiments, the individual suffering from the lymphoid neoplasm (for example, CLL/SLL or lymphoma, such as refractory DLBC lymphoma) has not had prior cytotoxic regimens. In some embodiments, the individual suffering from the lymphoid neoplasm (for example, CLL/SLL or lymphoma, such as refractory DLBC lymphoma) has had no more than 2 prior cytotoxic regimens. In some embodiments, the individual suffering from the lymphoid neoplasm (for example, lymphoma or for example, refractory diffuse large B-cell lymphoma) has had more than 2 prior cytotoxic regimens.

In some embodiments, there is provided a method of treating ovarian cancer in an individual comprising administering to the individual: a) an effective amount of a composition comprising nanoparticles comprising a taxane (such as paclitaxel) and a carrier protein (such as albumin) and b) an effective amount of a DNA methyltransferase inhibitor. In some embodiments, there is provided a method of treating ovarian cancer in an individual comprising administering to the individual: a) an effective amount of a composition comprising nanoparticles comprising a taxane (such as paclitaxel) and a carrier protein (such as albumin), and b) an effective amount of decitabine. In some embodiments, there is provided a method of treating ovarian cancer in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating ovarian cancer in an individual comprising administering to the individual: a) an effective amount of a composition comprising nanoparticles comprising paclitaxel coated with an albumin (such as Abraxane^{®}), and b) an effective amount of decitabine. In some embodiments, the nanoparticle composition is administered intravenously. In some embodiments, the decitabine is administered intravenously or intraperitoneally.

In some embodiments, the ovarian cancer is ovarian epithelial cancer. Exemplary ovarian epithelial cancer histological classifications include: serous cystomas (e.g., serous benign cystadenomas, serous cystadenomas with proliferating activity of the epithelial cells and nuclear abnormalities but with no infiltrative destructive growth, or serous cystadenocarcinomas), mucinous cystomas (e.g., mucinous benign cystadenomas, mucinous cystadenomas with proliferating activity of the epithelial cells and nuclear abnormalities but with no infiltrative destructive growth, or mucinous cystadenocarcinomas), endometrioid tumors (e.g., endometrioid benign cysts, endometrioid tumors with proliferating activity of the epithelial cells and nuclear abnormalities but with no infiltrative destructive growth, or endometrioid adenocarcinomas), clear cell (mesonephroid) tumors (e.g., benign clear cell tumors, clear cell tumors with proliferating activity of the epithelial cells and nuclear abnormalities but with no infiltrative destructive growth, or clear cell cystadenocarcinomas), unclassified tumors that cannot be allotted to one of the above groups, or other malignant tumors. In various embodiments, the ovarian epithelial cancer is stage I (e.g., stage IA, IB, or IC), stage II (e.g., stage IIA, IIB, or IIC), stage III (e.g., stage IIIA, IIIB, or IIIC), or stage IV. In some embodiments, the individual may be a human who has a gene, genetic mutation, or polymorphism associated with ovarian cancer (e.g., BRCA1 or BRCA2) or has one or more extra copies of a gene associated with ovarian cancer (e.g., one or more extra copies of the HER2 gene).

In some embodiments, the ovarian cancer is an ovarian germ cell tumor. Exemplary histologic subtypes include dysgerminomas or other germ cell tumors (e.g., endodermal sinus tumors such as hepatoid or intestinal tumors, embryonal carcinomas, olyembryomas, choriocarcinomas, teratomas, or mixed form tumors). Exemplary teratomas are immature teratomas, mature teratomas, solid teratomas, and cystic teratomas (e.g., dermoid cysts such as mature cystic teratomas, and dermoid cysts with malignant transformation). Some teratomas are monodermal and highly specialized, such as struma ovarii, carcinoid, struma ovarii and carcinoid, or others (e.g., malignant neuroectodermal and ependymomas). In some embodiments, the ovarian germ cell tumor is stage I (e.g., stage IA, IB, or IC), stage II (e.g., stage IIA, IIB, or IIC), stage III (e.g., stage IIIA, IIIB, or IIIC), or stage IV.

In some embodiments, there is provided a method of treating ovarian cancer in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating ovarian cancer in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating ovarian cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine. In some embodiments, the nanoparticle composition is administered first followed by administration of the decitabine. In some embodiments, the decitabine is administered first followed by administration of the nanoparticle composition. In some embodiments, the administrations of the nanoparticle composition and the decitabine are concurrent. In some embodiments, the nanoparticle composition is administered three out of four weeks and the decitabine is administered three times a day on the first, second, and third day on a six week cycle. In some embodiments, the nanoparticle composition is administered three out of four weeks and the decitabine is administered on the first, second, third, fourth and fifth day on a six week cycle. In some embodiments, the decitabine is administered three times a day on days 1-3, followed by administration of the nanoparticle composition on days 8, 15, and 22 of a 28-day cycle, for a total of 6 cycles. In some embodiments, the decitabine is administered on days 1-5, followed by administration of the nanoparticle composition on days 8, 15, and 22 of a 28-day cycle, for a total of 6 cycles.

In some embodiments, there is provided a method of treating ovarian cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine, wherein the decitabine is administered three times a day on days 1-3, followed by administration of the nanoparticle composition on days 8, 15, and 22 of a 28-day cycle, for a total of 6 cycles. In some embodiments, there is provided a method of treating ovarian cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine, wherein the decitabine is administered on days 1-5, followed by administration of the nanoparticle composition on days 8, 15, and 22 of a 28-day cycle, for a total of 6 cycles. In some embodiments, there is provided a method of treating ovarian cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravascularly or intraperitoneally administering to the individual about 5-100 mg/m² (such as 15 mg/m²) decitabine, wherein the decitabine is intravenously or intraperitoneally administered three times a day on days 1-3, followed by intravenous administration of the nanoparticle composition on days 8, 15, and 22 of a 28-day cycle, for a total of 6 cycles. In some embodiments, there is provided a method of treating ovarian cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravascularly or intraperitoneally administering to the individual about 5-100 mg/m² (such as 20 mg/m²) decitabine, wherein the decitabine is intravenously or intraperitoneally administered on days 1-5, followed by intravenous administration of the nanoparticle composition on days 8, 15, and 22 of a 28-day cycle, for a total of 6 cycles. In some embodiments, the individual suffering from ovarian cancer has not had prior cytotoxic regimens. In some embodiments, the individual suffering from ovarian cancer has had no more than 2 prior cytotoxic regimens. In some embodiments, the individual suffering from ovarian cancer has had more than 2 prior cytotoxic regimens.

In some embodiments, there is provided a method of treating endometrial cancer (e.g. uterine cancer) in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating endometrial cancer (e.g. uterine cancer) in an individual comprising administering to the individual: a) an effective amount of a composition comprising nanoparticles comprising a taxane (such as paclitaxel) and a carrier protein (such as albumin), and b) an effective amount of decitabine. In some embodiments, there is provided a method of treating endometrial cancer (e.g. uterine cancer) in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating endometrial cancer (e.g. uterine cancer) in an individual comprising administering to the individual: a) an effective amount of a composition comprising nanoparticles comprising paclitaxel coated with an albumin (such as Abraxane^{®}), and b) an effective amount of decitabine. In some embodiments, the nanoparticle composition is administered intravenously. In some embodiments, the decitabine is administered intravenously or intraperitoneally.

In some embodiments, the endometrial cancer is uterine papillary serous carcinoma or uterine serous adenocarcinoma. In some embodiments, the endometrial cancer is endometrial stromal sarcoma. In some embodiments the endometrial cancer includes, but is not limited to, endometrial intraepithelial neoplasia, endometrial intraepithelial neoplasm, or endometrial adenocarcinoma.

In some embodiments, there is provided a method of treating endometrial cancer (e.g. uterine cancer) in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating endometrial cancer (e.g. uterine cancer) in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating endometrial cancer (e.g. uterine cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 15-100 mg/m² decitabine. In some embodiments, the nanoparticle composition is administered first followed by administration of the decitabine. In some embodiments, the decitabine is administered first followed by administration of the nanoparticle composition. In some embodiments, the administrations of the nanoparticle composition and the decitabine are concurrent. In some embodiments, the nanoparticle composition is administered three out of four weeks and the decitabine is administered every eight hours on the first, second, third, fourth, fifth, or sixth day (such as on days 1-3) on a four week cycle. In some embodiments, the nanoparticle composition is administered three out of four weeks and the decitabine is administered every eight hours on the first, second, third fourth and fifth days (such as on days 1-5) on a four week cycle. In some embodiments, the decitabine is administered every eight hours on days 1-3, followed by administration of the nanoparticle composition on days 8, 15, and 22 of a 28-day cycle, for a total of 6 cycles. In some embodiments, the decitabine is administered on days 1-5, followed by administration of the nanoparticle composition on days 8, 15, and 22 of a 28-day cycle, for a total of 6 cycles.

In some embodiments, there is provided a method of treating endometrial cancer (e.g. uterine cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine, wherein the decitabine is administered every eight hours on days 1-3, followed by administration of the nanoparticle composition on days 8, 15, and 22 of a 28-day cycle, for a total of 6 cycles. In some embodiments, there is provided a method of treating endometrial cancer (e.g. uterine cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine, wherein the decitabine is administered on days 1-5, followed by administration of the nanoparticle composition on days 8, 15, and 22 of a 28-day cycle, for a total of 6 cycles. In some embodiments, there is provided a method of treating endometrial cancer (e.g. uterine cancer) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5-100 mg/m² (such as 20 mg/m²) decitabine, wherein the decitabine is intravenously or intraperitoneally administered on days 1-5, followed by intravenous administration of the nanoparticle composition on days 8, 15, and 22 of a 28-day cycle, for a total of 6 cycles. In some embodiments, the individual suffering from endometrial cancer (e.g. uterine cancer) has not had prior cytotoxic regimens. In some embodiments, the individual suffering from endometrial cancer (e.g. uterine cancer) has had no more than 2 prior cytotoxic regimens. In some embodiments, the individual suffering from endometrial cancer (e.g. uterine cancer) has had more than 2 prior cytotoxic regimens.

In some embodiments, there is provided a method of treating lung cancer in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating lung cancer in an individual comprising administering to the individual: a) an effective amount of a composition comprising nanoparticles comprising a taxane (such as paclitaxel) and a carrier protein (such as albumin), and b) an effective amount of decitabine. In some embodiments, there is provided a method of treating lung cancer in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating lung cancer in an individual comprising administering to the individual: a) an effective amount of a composition comprising nanoparticles comprising paclitaxel coated with an albumin (such as Abraxane^{®}), and b) an effective amount of decitabine. In some embodiments, the nanoparticle composition is administered intravenously. In some embodiments, the decitabine is administered intravenously or intraperitoneally.

In some embodiments, the lung cancer is a non-small cell lung cancer (NSCLC). Examples of NCSLC include, but are not limited to, large-cell carcinoma (e.g., large-cell neuroendocrine carcinoma, combined large-cell neuroendocrine carcinoma, basaloid carcinoma, lymphoepithelioma-like carcinoma, clear cell carcinoma, and large-cell carcinoma with rhabdoid phenotype), adenocarcinoma (e.g., acinar, papillary (e.g., bronchioloalveolar carcinoma, nonmucinous, mucinous, mixed mucinous and nonmucinous and indeterminate cell type), solid adenocarcinoma with mucin, adenocarcinoma with mixed subtypes, well-differentiated fetal adenocarcinoma, mucinous (colloid) adenocarcinoma, mucinous cystadenocarcinoma, signet ring adenocarcinoma, and clear cell adenocarcinoma), neuroendocrine lung tumors, and squamous cell carcinoma (e.g., papillary, clear cell, small cell, and basaloid). In some embodiments, the NSCLC may be, according to TNM classifications, a stage T tumor (primary tumor), a stage N tumor (regional lymph nodes), or a stage M tumor (distant metastasis).

In some embodiments, the lung cancer is a carcinoid (typical or atypical), adenosquamous carcinoma, cylindroma, or carcinoma of the salivary gland (e.g., adenoid cystic carcinoma or mucoepidermoid carcinoma). In some embodiments, the lung cancer is a carcinoma with pleomorphic, sarcomatoid, or sarcomatous elements (e.g., carcinomas with spindle and/or giant cells, spindle cell carcinoma, giant cell carcinoma, carcinosarcoma, or pulmonary blastoma). In some embodiments, the lung cancer is small cell lung cancer (SCLC; also called oat cell carcinoma). The small cell lung cancer may be limited-stage, extensive stage or recurrent small cell lung cancer. In some embodiments, the individual may be a human who has a gene, genetic mutation, or polymorphism suspected or shown to be associated with lung cancer (e.g., SASH1, LATS1, IGF2R, PARK2, KRAS, PTEN, Kras2, Krag, Pas1, ERCC1, XPD, IL8RA, EGFR, α₁-AD, EPHX, MMP1, MMP2, MMP3, MMP12, IL1β, RAS, and/or AKT) or has one or more extra copies of a gene associated with lung cancer.

In some embodiments, there is provided a method of treating lung cancer in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating lung cancer in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating lung cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 to 20 mg/m²) decitabine. In some embodiments, the nanoparticle composition is administered first followed by administration of the decitabine. In some embodiments, the decitabine is administered first followed by administration of the nanoparticle composition. In some embodiments, the administrations of the nanoparticle composition and the decitabine are concurrent. In some embodiments, the nanoparticle composition is administered three out of four weeks and the decitabine is administered every eight hours on the first, second, third, fourth, fifth, or sixth day (such as on days 1-3) on a four week cycle. In some embodiments, the nanoparticle composition is administered three out of four weeks and the decitabine is administered every eight hours on the first, second, third fourth and fifth days (such as on days 1-5) on a four week cycle. In some embodiments, the decitabine is administered every eight hours on days 1-3, followed by administration of the nanoparticle composition on days 8, 15, and 22 of a 28-day cycle, for a total of 6 cycles. In some embodiments, the decitabine is administered on days 1-5, followed by administration of the nanoparticle composition on days 8, 15, and 22 of a 28-day cycle, for a total of 6 cycles.

In some embodiments, there is provided a method of treating lung cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine, wherein the decitabine is administered three times a day on days 1-3, followed by administration of the nanoparticle composition on days 8, 15, and 22 of a 28-day cycle, for a total of 6 cycles. In some embodiments, there is provided a method of treating lung cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine, wherein the decitabine is administered on days 1-5, followed by administration of the nanoparticle composition on days 8, 15, and 22 of a 28-day cycle, for a total of 6 cycles. In some embodiments, there is provided a method of treating lung cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5 mg/m² to about 100 mg/m² (such as 15 mg/m²) decitabine, wherein the decitabine is intravenously or intraperitoneally administered three times a day on days 1-3, followed by intravenous administration of the nanoparticle composition on days 8, 15, and 22 of a 28-day cycle, for a total of 6 cycles. In some embodiments, there is provided a method of treating lung cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 20 mg/m²) and b) intravenously or intraperitoneally administering to the individual about 5-100 mg/m² (such as 20 mg/m²) decitabine, wherein the decitabine is intravenously or intraperitoneally administered on days 1-5, followed by intravenous administration of the nanoparticle composition on days 8, 15, and 22 of a 28-day cycle, for a total of 6 cycles. In some embodiments, the individual suffering from lung cancer has not had prior cytotoxic regimens. In some embodiments, the individual suffering from lung cancer has had no more than 2 prior cytotoxic regimens. In some embodiments, the individual suffering from lung cancer has had more than 2 prior cytotoxic regimens.

In some embodiments, there is provided a method of treating sarcoma in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating sarcoma in an individual comprising administering to the individual: a) an effective amount of a composition comprising nanoparticles comprising a taxane (such as paclitaxel) and a carrier protein (such as albumin), and b) an effective amount of decitabine. In some embodiments, there is provided a method of treating sarcoma in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating sarcoma in an individual comprising administering to the individual: a) an effective amount of a composition comprising nanoparticles comprising paclitaxel coated with an albumin (such as Abraxane^{®}), and b) an effective amount of decitabine. In some embodiments, the nanoparticle composition is administered intravenously. In some embodiments, the decitabine is administered intravenously or intraperitoneally.

In some embodiments, the sarcoma includes, but is not limited to, sarcomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, Kaposi's sarcoma, soft tissue sarcoma, uterine sacronomasynovioma, mesothelioma, Ewing's tumor, leiomyosarcoma, or rhabdomyosarcoma.

In some embodiments, there is provided a method of treating sarcoma in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating sarcoma in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating sarcoma in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15-20 mg/m²) decitabine. In some embodiments, the nanoparticle composition is administered first followed by administration of the decitabine. In some embodiments, the decitabine is administered first followed by administration of the nanoparticle composition. In some embodiments, the administrations of the nanoparticle composition and the decitabine are concurrent. In some embodiments, the nanoparticle composition is administered three out of four weeks and the decitabine is administered on the first, second, third, fourth, fifth, or sixth day (such as on days 1-3) on a four week cycle. In some embodiments, the nanoparticle composition is administered three out of four weeks and the decitabine is administered on the first, second, third, fourth, fifth, or sixth day (such as on days 1-5) on a four week cycle. In some embodiments, the decitabine is administered three times a day on days 1-3, followed by administration of the nanoparticle composition on days 8, 15, and 22 of a 28-day cycle, for a total of 6 cycles. In some embodiments, the decitabine is administered on days 1-5, followed by administration of the nanoparticle composition on days 8, 15, and 22 of a 28-day cycle, for a total of 6 cycles

In some embodiments, there is provided a method of treating sarcoma in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine, wherein the decitabine is administered three times a day on days 1-3, followed by administration of the nanoparticle composition on days 8, 15, and 22 of a 28-day cycle, for a total of 6 cycles. In some embodiments, there is provided a method of treating sarcoma in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine, wherein the decitabine is administered on days 1-5, followed by administration of the nanoparticle composition on days 8, 15, and 22 of a 28-day cycle, for a total of 6 cycles. In some embodiments, there is provided a method of treating sarcoma in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5 mg/m² to about 100 mg/m² (such as 15 mg/m²) decitabine, wherein the decitabine is intravenously or intraperitoneally administered three times a day on days 1-3, followed by intravenous administration of the nanoparticle composition on days 8, 15, and 22 of a 28-day cycle, for a total of 6 cycles. In some embodiments, there is provided a method of treating sarcoma in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5-100 mg/m² (such as 20 mg/m²) decitabine, wherein the decitabine is intravenously or intraperitoneally administered on days 1-5, followed by intravenous administration of the nanoparticle composition on days 8, 15, and 22 of a 28-day cycle, for a total of 6 cycles. In some embodiments, the individual suffering from sarcoma has not had prior cytotoxic regimens. In some embodiments, the individual suffering from sarcoma has had no more than 2 prior cytotoxic regimens. In some embodiments, the individual suffering from sarcoma has had more than 2 prior cytotoxic regimens.

In some embodiments, there is provided a method of treating pancreatic cancer in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating pancreatic cancer in an individual comprising administering to the individual: a) an effective amount of a composition comprising nanoparticles comprising a taxane (such as paclitaxel) and a carrier protein (such as albumin), and b) an effective amount of decitabine. In some embodiments, there is provided a method of treating pancreatic cancer in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating pancreatic cancer in an individual comprising administering to the individual: a) an effective amount of a composition comprising nanoparticles comprising paclitaxel coated with an albumin (such as Abraxane^{®}), and b) an effective amount of decitabine. In some embodiments, the nanoparticle composition is administered intravenously. In some embodiments, the decitabine is administered intravenously or intraperitoneally.

In some embodiments, the pancreatic cancer includes, but is not limited to, serous microcystic adenoma, intraductal papillary mucinous neoplasm, mucinous cystic neoplasm, solid pseudopapillary neoplasm, pancreatic adenocarcinoma, pancreatic ductal carcinoma, or pancreatoblastoma.

In some embodiments, there is provided a method of treating pancreatic cancer in an individual, comprising administering to the individual: a) an effective amount of a composition comprising nanoparticles comprising a taxane (such as paclitaxel) and a carrier protein (such as albumin), wherein the taxane is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating pancreatic cancer in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 5-100 mg/m² or for example about 15-20 mg/m²) decitabine. In some embodiments, there is provided a method of treating pancreatic cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine. In some embodiments, there is provided a method of treating pancreatic cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine. In some embodiments, the nanoparticle composition is administered first followed by administration of the decitabine. In some embodiments, the decitabine is administered first followed by administration of the nanoparticle composition. In some embodiments, the administrations of the nanoparticle composition and the decitabine are concurrent. In some embodiments, the nanoparticle composition is administered three out of four weeks and the decitabine is administered three times a day on the first, second, third, fourth, fifth, or sixth (such as on days 1-3) on a four week cycle. In some embodiments, the nanoparticle composition is administered three out of four weeks and the decitabine is administered on the first, second, third, fourth, fifth, or sixth day (such as on days 1-5) on a four week cycle. In some embodiments, the decitabine is administered three times a day on days 1-3, followed by administration of the nanoparticle composition on days 8, 15, and 22 of a 28-day cycle, for a total of 6 cycles. In some embodiments, the decitabine is administered on days 1-5, followed by administration of the nanoparticle composition on days 8, 15, and 22 of a 28-day cycle, for a total of 6 cycles.

In some embodiments, there is provided a method of treating pancreatic cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine, wherein the decitabine is administered three times a day on days 1-3, followed by administration of the nanoparticle composition on days 8, 15, and 22 of a 28-day cycle, for a total of 6 cycles. In some embodiments, there is provided a method of treating pancreatic cancer in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine, wherein the decitabine is administered on days 1-5, followed by administration of the nanoparticle composition on days 8, 15, and 22 of a 28-day cycle, for a total of 6 cycles. In some embodiments, there is provided a method of treating pancreatic cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5 mg/m² to about 100 mg/m² (such as 15 mg/m²) decitabine, wherein the decitabine is intravenously or intraperitoneally administered three times a day on days 1-3, followed by intravenous administration of the nanoparticle composition on days 8, 15, and 22 of a 28-day cycle, for a total of 6 cycles. In some embodiments, there is provided a method of treating pancreatic cancer in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5-100 mg/m² (such as 20 mg/m²) decitabine, wherein the decitabine is intravenously or intraperitoneally administered on days 1-5, followed by intravenous administration of the nanoparticle composition on days 8, 15, and 22 of a 28-day cycle, for a total of 6 cycles. In some embodiments, the individual suffering from pancreatic cancer has not had prior cytotoxic regimens. In some embodiments, the individual suffering from pancreatic cancer has had no more than 2 prior cytotoxic regimens. In some embodiments, the individual suffering from pancreatic cancer has had more than 2 prior cytotoxic regimens.

In some embodiments, there is provided a method of treating breast cancer (for example, HER2 negative breast cancer or for example, triple negative breast cancer) in an individual comprising administering to the individual: a) an effective amount of a composition comprising nanoparticles comprising a taxane (such as paclitaxel) and a carrier protein (such as albumin), and b) an effective amount of a DNA methyltransferase inhibitor. In some embodiments, there is provided a method of treating breast cancer (for example, HER2 negative breast cancer or for example, triple negative breast cancer) in an individual comprising administering to the individual: a) an effective amount of a composition comprising nanoparticles comprising a taxane (such as paclitaxel) and a carrier protein (such as albumin), and b) an effective amount of decitabine. In some embodiments, there is provided a method of treating breast cancer (for example, HER2 negative breast cancer or for example, triple negative breast cancer) in an individual comprising administering to the individual: a) an effective amount of a composition comprising nanoparticles comprising paclitaxel coated with an albumin (such as Abraxane^{®}), and b) an effective amount of a DNA methyltransferase inhibitor. In some embodiments, there is provided a method of treating breast cancer (for example, HER2 negative breast cancer or for example, triple negative breast cancer) in an individual comprising administering to the individual: a) an effective amount of a composition comprising nanoparticles comprising paclitaxel coated with an albumin (such as Abraxane^{®}), and b) an effective amount of decitabine. In some embodiments, the nanoparticle composition is administered intravenously. In some embodiments, the decitabine is administered intravenously or intraperitoneally.

In some embodiments, the breast cancer is early stage breast cancer, non-metastatic breast cancer, stage IV breast cancer, locally advanced breast cancer, metastatic breast cancer, hormone receptor positive metastatic breast cancer, breast cancer in remission, breast cancer in an adjuvant setting, ductal carcinoma in situ (DCIS), invasive ductal carcinoma (IDC), or breast cancer in a neoadjuvant setting. In some embodiments, the breast cancer is hormone receptor positive metastatic breast cancer. In some embodiments, the breast cancer (which may be HER2 positive or HER2 negative) is advanced breast cancer. In some embodiments, the breast cancer is ductal carcinoma in situ. In some embodiments, the individual may be a human who has a gene, genetic mutation, or polymorphism associated with breast cancer *(e.g.,* BRCA1, BRCA2, ATM, CHEK2, RAD51, AR, DIRAS3, ERBB2, TP53, AKT, PTEN, and/or PI3K) or has one or more extra copies of a gene (*e.g.,* one or more extra copies of the HER2 gene) associated with breast cancer.

In some embodiments, there is provided a method of treating breast cancer (for example, HER2 negative breast cancer or for example, triple negative breast cancer) in an individual, comprising administering to the individual: a) an effective amount of a composition comprising nanoparticles comprising a taxane (such as paclitaxel) and albumin, wherein the taxane is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}) and a carrier protein (such as albumin), wherein the paclitaxel coated with an albumin is in the dosage range of about 60-300 mg/m² (including for example about 80-200 mg/m² or for example about 100 mg/m²), and b) about 5-500 mg/m² (including for example about 10-200 mg/m² or for example about 15-20 mg/m²) decitabine. In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine. In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine. In some embodiments, the nanoparticle composition is administered first followed by administration of the decitabine. In some embodiments, the decitabine is administered first followed by administration of the nanoparticle composition. In some embodiments, the administrations of the nanoparticle composition and the decitabine are concurrent. In some embodiments, the nanoparticle composition is administered three out of four weeks and the decitabine is administered three times a day on the first, second, third, fourth, fifth, or sixth day (such as on days 1-3) on a four week cycle. In some embodiments, the nanoparticle composition is administered three out of four weeks and the decitabine is administered on the first, second, third, fourth, fifth or sixth day (such as on days 1-5) on a four week cycle. In some embodiments, the decitabine is administered three times a day on days 1-3, followed by administration of the nanoparticle composition on days 8, 15, and 22 of a 28-day cycle, for a total of 6 cycles. In some embodiments, the decitabine is administered on days 1-5, followed by administration of the nanoparticle composition on days 8, 15, and 22 of a 28-day cycle, for a total of 6 cycles.

In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 15 mg/m²) decitabine, wherein the decitabine is administered three times a day on days 1-3 followed by administration of the nanoparticle composition on days 8, 15, and 22 of a 28-day cycle, for a total of 6 cycles. In some embodiments, there is provided a method of treating breast cancer (for example, HER2 negative breast cancer or for example, triple negative breast cancer) in an individual, comprising administering to the individual: a) an effective amount of a composition comprising nanoparticles comprising paclitaxel coated with an albumin (such as Abraxane^{®}), wherein the taxane is in the dosage range of about 100 mg/m², and b) about 5 mg/m² to about 100 mg/m² (such as 20 mg/m²) decitabine, wherein the decitabine is administered on days 1-5 followed by administration of the nanoparticle composition on days 8, 15, and 22 of a 28-day cycle, for a total of 6 cycles. In some embodiments, there is provided a method of treating breast cancer (for example, HER2 negative breast cancer or for example, triple negative breast cancer) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5 mg/m² to about 100 mg/m² (such as 15 mg/m²) decitabine, wherein the decitabine is intravenously or intraperitoneally administered three times a day on days 1-3, followed by intravenous administration of the nanoparticle composition on days 8, 15, and 22 of a 28-day cycle, for a total of 6 cycles. In some embodiments, there is provided a method of treating breast cancer (for example, HER2 negative breast cancer or for example, triple negative breast cancer) in an individual, comprising: a) intravenously administering to the individual an effective amount of a composition comprising nanoparticles comprising paclitaxel coated with an albumin (such as Abraxane^{®}), wherein the taxane is in the dosage range of about 100-150 mg/m² (such as 100 mg/m²) and b) intravenously or intraperitoneally administering to the individual about 5-100 mg/m² (such as 20 mg/m²) decitabine, wherein the decitabine is intravenously or intraperitoneally administered on days 1-5, followed by intravenous administration of the nanoparticle composition on days 8, 15, and 22 of a 28-day cycle, for a total of 6 cycles. In some embodiments, the individual suffering from breast cancer (for example, HER2 negative breast cancer or for example, triple negative breast cancer) has not had prior cytotoxic regimens. In some embodiments, the individual suffering from breast cancer (for example, HER2 negative breast cancer or for example, triple negative breast cancer) has had no more than 2 prior cytotoxic regimens. In some embodiments, the individual suffering from breast cancer (for example, HER2 negative breast cancer or for example, triple negative breast cancer) has had more than 2 prior cytotoxic regimens.

In some embodiments, the methods further comprise administration of one or more additional agent. In some embodiments, the additional agent is another agent that modifies the epigenetics in a cell, such as the agents described herein. In some embodiments, the additional agent is a chemotherapeutic agent, such as chemotherapeutic agents described in U.S. Patent Application No. 2006/0263434, incorporated herein in its entirety. In some embodiments, the additional agent is any one of dexamethasone, bortezomib, imatinib, sorafenib, gemcitabine, capecitabine, lenalidomide, sunitinib, paclitaxel, and docetaxel. For example, in some embodiments, there is provided a method of treating a proliferative disease, comprising: a) an effective amount of a composition comprising nanoparticles comprising a taxane (such as paclitaxel) and a carrier protein (such as albumin), b) an effective amount of vorinostat, and c) an effective amount of an additional agent selected from the group consisting of dexamethasone, bortezomib, imatinib, sorafenib, gemcitabine, capecitabine, lenalidomide, sunitinib, paclitaxel, and docetaxel. In some embodiments, there is provided a method of treating a proliferative disease, comprising: a) an effective amount of a composition comprising nanoparticles comprising a taxane (such as paclitaxel) and a carrier protein (such as albumin), b) an effective amount of vorinostat, and c) an effective amount of capecitabine. in some embodiments, there is provided a method of treating a proliferative disease, comprising: a) an effective amount of a composition comprising nanoparticles comprising a taxane (such as paclitaxel) and a carrier protein (such as albumin), b) an effective amount of vorinostat, and c) an effective amount of azacitidine.

In some embodiments, the method further comprises the administration of a platinum-based agent, including for example carboplatin and cisplatin. For example, in some embodiments, there is provided a method of treating a proliferative disease (such as cancer) in an individual, comprising administering to the individual: a) an effective amount of a composition comprising nanoparticles comprising a taxane (such as paclitaxel) and a carrier protein (such as albumin); and b) an effective amount of a histone deacetylase inhibitor, and c) an effective amount of a platinum-based agent. In some embodiments, there is provided a method of treating cancer (such as breast cancer, including triple negative breast cancer) in an individual, comprising administering to the individual: a) an effective amount of a composition comprising nanoparticles comprising a taxane (such as paclitaxel) and a carrier protein (such as albumin); and b) an effective amount of a histone deacetylase inhibitor, and c) an effective amount of a platinum-based agent. In some embodiments, there is provided a method of treating cancer (such as breast cancer, including triple negative breast cancer) in an individual, comprising administering to the individual: a) an effective amount of a composition comprising nanoparticles comprising paclitaxel and albumin; and b) an effective amount of vorinostat, and c) an effective amount of carboplatin. In some embodiments, there is provided a method of treating cancer (such as breast cancer, including triple negative breast cancer) in an individual, comprising: a) intravenously administering an effective amount of a composition comprising nanoparticles comprising paclitaxel and albumin; b) orally administering an effective amount of vorinostat, and c) intravenously administering an effective amount of carboplatin.

In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about 100 mg/m²); b) orally administering about 200 to about 500 (such as about 400 mg) vorinostat, and c) intravenously administering carboplatin at the dose of AUC 2-6 (such as AUC2). In some embodiments, there is provided a method of treating cancer (such as breast cancer, including triple negative breast cancer) in an individual, comprising: a) intravenously administering a composition comprising nanoparticles comprising paclitaxel and albumin at the dose of about 80 to about 200 mg/m² (such as about100 mg/m²) weekly; b) orally administering about 200 to about 500 (such as about 400 mg) vorinostat three out of seven days, and c) intravenously administering carboplatin at the dose of AUC 2-6 (such as AUC2) weekly. In some embodiments, the administrations of the nanoparticle composition, the vorinostat, and the carboplatin are concurrent. In some embodiments, the nanoparticle composition and the carboplatin are administered on day one of each week, and the vorinostat is administered on days 1-3 of each week.

The present invention in some embodiments provides a method of treating a proliferative disease in an individual comprising administering to the individual: a) an effective amount of a composition comprising nanoparticles comprising a taxane (such as paclitaxel) and a carrier protein, and b) an effective amount of at least one other agent that modifies the epigenetics in a cell. The other agent can be an inhibitor of histone deacetylase (such as vorinostat) or an inhibitor of DNA methyltransferase (such as azacitidine or decitabine). In some embodiments, the method further comprises administering to said individual a platinum-based agent. In some embodiments of any of the methods described herein, the proliferative disease is cancer, such as breast cancer. In some embodiments, the individual is negative for ER, PR, or HER2. In some embodiments, individual is negative for ER, PR, and HER2. In some embodiments, the proliferative disease is ovarian cancer. In some embodiments, the proliferative disease is lung cancer (such as non-small cell lung cancer). In some embodiments of any of the methods described above, the composition comprising nanoparticles comprising taxane and albumin and the other agent are administered simultaneously. In some embodiments of any of the methods described above, the composition comprising nanoparticles comprising taxane and albumin and the other agent are administered sequentially. In some embodiments of any of the methods described above, the composition comprising nanoparticles comprising taxane and albumin and the other agent are administered concurrently.

In some embodiments of any of the methods described above, the taxane is paclitaxel. In some embodiments of any of the methods described above, the average diameter of the nanoparticles in the composition is no greater than about 200 nm. In some embodiments of any of the methods described above, the carrier protein is albumin. In some embodiments, the weight ratio of the albumin and the taxane in the nanoparticle composition is less than about 1:1 to about 18:1. In some embodiments of any of the methods described above, the individual is a human.

The present application also provides pharmaceutical compositions comprising nanoparticles comprising a taxane (such as paclitaxel) and a carrier protein (such as albumin) for use in the treatment of a proliferative disease (such as cancer), wherein said use comprises simultaneous, sequential, and/or concurrent administration of an agent that modifies the epigenetics in a cell. In some embodiments, the invention provides a pharmaceutical composition comprising an agent that modifies the epigenetics in a cell for use in the treatment of a proliferative disease (such as cancer), wherein said use comprises simultaneous, sequential, and/or concurrent administration of a composition comprising nanoparticles comprising a taxane (such as paclitaxel) and a carrier protein (such as albumin). In some embodiments, the invention provides taxane-containing nanoparticle compositions and compositions comprising an agent that inhibits prosurvival and/or inflammatory signal for simultaneous, sequential, and/or concurrent use for treatment of a proliferative disease (such as cancer).

In some embodiments, there is provided a kit comprising: a) a composition comprising nanoparticles comprising a taxane (such as paclitaxel) and a carrier protein, and b) an effective amount of at least one other agent that modifies the epigenetics in a cell. In some embodiments, there is provided a medicine comprising: a) a composition comprising nanoparticles comprising a taxane (such as paclitaxel) and a carrier protein, and b) an effective amount of at least one other agent that modifies the epigenetics in a cell.

### Methods of treating proliferative diseases

The combination therapy methods described herein are useful for treating proliferative diseases. The methods require administration of the nanoparticle composition and the other agent in effective amounts. In some embodiments, an effective amount is an amount sufficient to delay development. In some embodiments, an effective amount is an amount sufficient to prevent or delay recurrence. An effective amount can be administered in one or more administrations. In the case of cancer, the effective amount of the drug or composition may: (i) reduce the number of cancer cells; (ii) reduce tumor size; (iii) inhibit, retard, slow to some extent and preferably stop cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer.

Thus, in some embodiments, there is provided a method of inhibiting cell proliferation (such as tumor growth) in an individual, comprising administering to the individual: a) an effective amount of a composition comprising nanoparticles comprising taxane and a carrier protein, and b) an effective amount of at least one other agent that modifies the epigenetics in a cell. In some embodiments, the effective amounts of the taxane nanoparticle composition and the other agent synergistically inhibit cell proliferation (such as tumor cell growth). In some embodiments, at least about 10% (including for example at least about any of 20%, 30%, 40%, 60%, 70%, 80%, 90%, or 100%) cell proliferation is inhibited. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by oral administration. In some embodiments, the other agent is administered intravenously or subcutaneously.

In some embodiments, there is provided a method of inhibiting tumor metastasis (such as metastasis of breast cancer, pulmonary metastasis or metastasis to the lymph node) in an individual, comprising administering to the individual: a) an effective amount of a composition comprising nanoparticles comprising taxane and a carrier protein, and b) an effective amount of at least one other agent that modifies the epigenetics in a cell. In some embodiments, the effective amounts of the taxane nanoparticle composition and the other agent synergistically inhibit tumor metastasis. In some embodiments, at least about 10% (including for example at least about any of 20%, 30%, 40%, 60%, 70%, 80%, 90%, or 100%) metastasis is inhibited. In some embodiments, method of inhibiting metastasis to lymph node is provided. In some embodiments, method of inhibiting metastasis to the lung is provided. In some embodiments, the taxane is paclitaxel. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by oral administration. In some embodiments, the other agent is administered intravenously or subcutaneously.

In some embodiments, there is provided a method of reducing (such as eradiating) pre-existing tumor metastasis (such as pulmonary metastasis or metastasis to the lymph node) in an individual, comprising administering to the individual: a) an effective amount of a composition comprising nanoparticles comprising taxane and a carrier protein, and b) an effective amount of at least one other agent that modifies the epigenetics in a cell. In some embodiments, the effective amounts of the taxane nanoparticle composition and the other agent synergistically reduces (such as eradicates) tumor metastasis. In some embodiments, at least about 10% (including for example at least about any of 20%, 30%, 40%, 60%, 70%, 80%, 90%, or 100%) metastasis is reduced. In some embodiments, method of reducing metastasis to lymph node is provided. In some embodiments, method of reducing metastasis to the lung is provided. In some embodiments, the taxane is paclitaxel. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by oral administration. In some embodiments, the other agent is administered intravenously or subcutaneously.

In some embodiments, there is provided a method of reducing incidence or burden of preexisting tumor metastasis (such as pulmonary metastasis or metastasis to the lymph node) in an individual, comprising administering to the individual: a) an effective amount of a composition comprising nanoparticles comprising taxane and a carrier protein, and b) an effective amount of at least one other agent that modifies the epigenetics in a cell. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by oral administration. In some embodiments, the other agent is administered intravenously or subcutaneously.

In some embodiments, there is provided a method of reducing tumor size in an individual, comprising administering to the individual: a) an effective amount of a composition comprising nanoparticles comprising taxane and a carrier protein, and b) an effective amount of at least one other agent that modifies the epigenetics in a cell. In some embodiments, the effective amounts of the taxane nanoparticle composition and the other agent synergistically reduces tumor size. In some embodiments, the tumor size is reduced at least about 10% (including for example at least about any of 20%, 30%, 40%, 60%, 70%, 80%, 90%, or 100%). In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the other agent is administered intravenously or subcutaneously.

In some embodiments, there is provided a method of prolonging time to disease progression of a proliferative disease (such as cancer) in an individual, comprising administering to the individual: a) an effective amount of a composition comprising nanoparticles comprising taxane and a carrier protein, and b) an effective amount of at least one other agent that modifies the epigenetics in a cell. In some embodiments, the method prolongs the time to disease progression by at least any of 1, 2, 3, 4, 5, 6, 7, 8, 9, 10, 11, or 12 weeks. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the other agent is administered intravenously or subcutaneously.

In some embodiments, there is provided a method of prolonging survival of an individual having a proliferative disease (such as cancer), comprising administering to the individual: a) an effective amount of a composition comprising nanoparticles comprising taxane and a carrier protein, and b) an effective amount of at least one other agent that modifies the epigenetics in a cell. In some embodiments, the method prolongs the survival of the individual by at least any of 1, 2, 3, 4, 5, 6, 7, 8, 9, 10, 11, 12, 18, or 24 month. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the other agent is administered intravenously or subcutaneously.

It is understood that any of the embodiments in this section apply to the embodiments provided in the section "methods of combination therapy." For example, in some embodiments, there is provided a method of reducing (such as eradiating) pre-existing tumor metastasis (such as pulmonary metastasis or metastasis to the lymph node) in an individual, comprising administering to the individual: a) an effective amount of a composition comprising nanoparticles comprising taxane and a carrier protein, and b) an effective amount of a vorinostat, wherein the nanoparticle composition and the vorinostat are administered concurrently. In some embodiments, there is provided a method of reducing (such as eradiating) pre-existing tumor metastasis (such as pulmonary metastasis or metastasis to the lymph node) in an individual, comprising administering to the individual: a) an effective amount of nanoparticles comprising paclitaxel coated with albumin (such as Abraxane^{®}), and b) an effective amount of a vorinostat, wherein the nanoparticle composition and the vorinostat are administered concurrently. In some embodiments, there is provided a method of reducing (such as eradiating) pre-existing tumor metastasis (such as pulmonary metastasis or metastasis to the lymph node) in an individual, comprising administering to the individual: a) an effective amount of a composition comprising nanoparticles comprising taxane and a carrier protein, and b) an effective amount of a histone deacetylase inhibitor, wherein the nanoparticle composition and the histone deacetylase inhibitor are administered concurrently. In some embodiments, there is provided a method of reducing (such as eradiating) pre-existing tumor metastasis (such as pulmonary metastasis or metastasis to the lymph node) in an individual, comprising administering to the individual: a) an effective amount of nanoparticles comprising paclitaxel coated with albumin (such as Abraxane^{®}), and b) an effective amount of a histone deacetylase inhibitor, wherein the nanoparticle composition and the bcl-2 are administered concurrently.

The effectiveness of the methods of the present invention can be assessed by one or more criteria, which include, but are not limited to, markers of proliferation and/or apoptosis, gene methylation, gene expression profile, and tissue histone acetylation. In some embodiments, the effectiveness of the method can be assessed by functional imaging, such as PET/CT scans and/or fludeoxyglucose F 18-position emission tomography (FDG-PET) (Sun, X. et al. J. Nucl. Med. (2011) 52(1):140-146).

Thus, for example, in some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies the epigenetics in a cell, wherein the standard uptake value (SUV) determined in a FDG-PET scan in the individual is decreased by at least about any of 10%, 20%, 30%, 40%, 50%, 60%, 70%, 80%, 90%, or 95%. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the method further comprises determining a baseline SUV value in the individual prior to the treatment. In some embodiments, the method further comprises determining the SUV value in the individual after the treatment.

In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies the epigenetics in a cell, wherein the level of C1D15 is decreased by at least about any of 10%, 20%, 30%, 40%, 50%, 60%, 70%, 80%, 90%, or 95%. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the method further comprises determining a baseline C1D15 level in the individual prior to the treatment. In some embodiments, the method further comprises determining the C1D15 level in the individual after the treatment.

In some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies the epigenetics in a cell, wherein the level of DNA methylation in one or more genes in the individual is decreased by at least about any of 10%, 20%, 30%, 40%, 50%, 60%, 70%, 80%, 90%, or 95%. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the method further comprises determining a baseline DNA methylation level in the individual prior to the treatment. In some embodiments, the method further comprises determining the DNA methylation level in the individual after the treatment. In some embodiments, the level of DNA methylation is determined based on the methylation of one or more target genes, such as ER-alpha, APC-1, RAR-beta, cyclin D2, Twist, RASSF1A, and HIN-1. The levels of methylation can be determined, for example, by quantitative multiplex methylation-specific PCR.

In some embodiments, the responsiveness of the method is determined by gene expression profile. For example, in some embodiments, there is provided a method of treating a proliferative disease (such as cancer), comprising administering to the *individual a) an effective amount of a composition comprising nanoparticles comprising a taxane (such as paclitaxel) and a carrier protein (such as albumin), and b) an effective amount of at least one other agent that modifies the epigenetics in a cell, wherein the level of expression in one or more genes in the individual is changed by at least about any of 10%, 20%, 30%, 40%, 50%, 60%, 70%, 80%, 90%, or 95%. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the method further comprises determining a baseline gene expression profile in the individual prior to the treatment. In some embodiments, the method further comprises determining the gene expression profile in the individual after the treatment. Changes of gene expression can be determined, for example, by RT-PCR or immunohistochemistry.

In some embodiments, the responsiveness of the method is determined by the level of histone deacetylation. For example, in some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of at least one other agent that modifies the epigenetics in a cell, wherein the level of histone deacetylation in the individual is decreased by at least about any of 10%, 20%, 30%, 40%, 50%, 60%, 70%, 80%, 90%, or 95%. In some embodiments, the taxane is paclitaxel. In some embodiments, the other agent is an inhibitor of a histone deacetylase (such as vorinostat). In some embodiments, the other agent is an inhibitor of DNA methyltransferase (such as azacitidine). In some embodiments, the other agent is an inhibitor of DNA methyltransferase (such as decitabine). In some embodiments, the taxane in the nanoparticle in the composition is administered by intravenous administration. In some embodiments, the other agent is administered by intraperitoneal administration. In some embodiments, the other agent is administered by oral administration. In some embodiments, the method further comprises determining a baseline histone acetylation level in the individual prior to the treatment. In some embodiments, the method further comprises determining the histone acetylation level in the individual after the treatment. Level of histone acetylation can be determined, for example, by determination of histone acetylation level in tissue and/or peripheral blood mononuclear cells.

The treatment methods can also be evaluated for safety and toxicity, for example, based on NCI CTCAE analyses.

The methods described herein are useful for treating various diseases. In some embodiments, the proliferative disease is a non-cancerous disease, including, but not limited to, fibrosis (especially pulmonary, but also other types of fibrosis, such as renal fibrosis), angiogenesis, psoriasis, atherosclerosis and smooth muscle proliferation in the blood vessels, such as stenosis or restenosis following angioplasty. In some embodiments, there is provided a method of treating any of the following diseases: restenosis, stenosis, fibrosis, angiogenesis, psoriasis, atherosclerosis, and proliferation of smooth muscle cells.

In some embodiments, the proliferative disease is cancer. In some embodiments, the proliferative disease is a benign or malignant tumor. Where hereinbefore and subsequently a tumor, a tumor disease, a carcinoma or a cancer are mentioned, also metastasis in the original organ or tissue and/or in any other location are implied alternatively or in addition, whatever the location of the tumor and/or metastasis is.

In some embodiments, the method is used to treat a primary tumor. In some embodiments, a method of treating metastatic cancer (that is, cancer that has metastasized from the primary tumor) is provided. In some embodiments, the method is for the treatment of an advanced disease or a lesser extent of disease, such as low tumor burden. In some embodiments, there is provided a method of treating cancer at an advanced stage. In some embodiments, the method is for the treatment of an early stage breast cancer. The methods may be practiced in an adjuvant setting. The methods provided herein may also be practiced in a neoadjuvant setting, *i.e.,* the method may be carried out before the primary/definitive therapy. In some embodiments, the method further comprises conducting surgery on the individual following the completion of the treatment. For example, in some embodiments when the cancer is breast cancer, breast conserving surgery or mastectomy can be carried out within about 2, 3, 4, 5, 6, 7, 8, 9, 10, 11, or 12 weeks after completion of the neoadjuvant chemotherapy.

In some embodiments, the individual has previously been treated. In some embodiments, the individual has not previously been treated. In some embodiments, the treatment is a first line therapy. In some embodiments, the breast cancer has reoccurred after a remission.

In some embodiments, the cancer is breast cancer. These methods can be used, for example, to treat, stabilize, prevent, and/or delay any type or stage of breast cancer, such as early stage breast cancer, non-metastatic breast cancer, advanced breast cancer, stage IV breast cancer, locally advanced breast cancer, metastatic breast cancer, breast cancer in remission, breast cancer in an adjuvant setting, or breast cancer in a neoadjuvant setting. In some embodiments, the method is useful for preoperative systemic therapy (PST).

In some embodiments, there is provided a method of treating breast cancer (which may be HER2 positive or HER2 negative), including, for example, advanced breast cancer, stage IV breast cancer, locally advanced breast cancer, and metastatic breast cancer. In some embodiments, the breast cancer is luminal type B breast cancer. In some embodiments, the breast cancer is basal cell breast cancer. In some embodiments, the individual is diagnosed with T2, T3, or T4 lesion, or a stage N, M0 or T1c, N1-3 and M0. In some embodiments, the individual has an ECOG performance status of 0-1. In some embodiments, the individual has skin metastasis to the ipsilateral breast. In some embodiments, the individual has undergone prior therapy (such as hormonal therapy). In some embodiments, the individual has not undergone prior therapy (such as hormonal therapy). In some embodiments, the individual is awaiting definitive surgery. In some embodiments, the breast cancer is resected breast cancer. In some embodiments, the breast cancer is unresected breast cancer, such as unresected stage II or III breast cancer.

In some embodiments, the method is for treating an individual having one or more of these risk factors resulting in a higher probability of developing breast cancer than an individual without these risk factor(s). These risk factors include, but are not limited to, age, sex, race, diet, history of previous disease, presence of precursor disease, genetic (i.e., hereditary) considerations, and environmental exposure. In some embodiments, the individual may be a human who is genetically or otherwise predisposed to developing breast cancer who has or has not been diagnosed with breast cancer. Individuals at risk for breast cancer include, e.g., those having relatives who have experienced this disease, and those whose risk is determined by analysis of genetic or biochemical markers. For example, the individual may be a human who has a gene, genetic mutation, or polymorphism associated with breast cancer (e.g., BRCA1, BRCA2, ATM, CHEK2, RAD51, AR, DIRAS3, ERBB2, and/or TP53) or has one or more extra copies of a gene (e.g., one or more extra copies of the HER2 gene) associated with breast cancer. In some embodiments, the breast cancer is HER2 negative. In some embodiments, the breast cancer is ER negative. In some embodiments, the breast cancer is PR negative. In some embodiments, the breast cancer is EP negative and HER2 negative. In some embodiments, the breast cancer is PR negative and HER2 negative. In some embodiments, the breast cancer is ER negative and PR negative. In some embodiment, the breast cancer is ER negative, PR negative, and HER2 negative.

The methods described herein are also useful for treating other solid tumors (such as advanced solid tumors). In some embodiments, there is provided a method of treating lung cancer, including, for example, non-small cell lung cancer (NSCLC, such as advanced NSCLC), small cell lung cancer (SCLC, such as advanced SCLC), and advanced solid tumor malignancy in the lung. In some embodiments, there is provided a method of treating any of ovarian cancer, head and neck cancer, gastric malignancies, melanoma (including metastatic melanoma and malignant melanoma), ovarian cancer, colorectal cancer, and pancreatic cancer.

In some embodiments, the method is useful for treating one or more of the following: cutaneous T cell lymphoma (CTCL), leukemia, follicular lymphoma, Hodgkin lymphoma, and acute myeloid leukemia.

In some embodiments, the disease is a cancer of any one of the following: basal cell carcinoma, medulloblastoma, glioblastoma, multiple myeloma, chronic myelogenous leukemia (CML), acute myelogenous leukemia, pancreatic cancer, lung cancer (small cell lung cancer and non-small cell lung cancer), esophageal cancer, stomach cancer, billary cancer, prostate cancer, liver cancer, hepatocellular cancer, gastrointestinal cancer, gastric cancer, and ovarian and bladder cancer. In some embodiments, the cancer is selected from the group consisting of pancreas ductal adenocarcinoma, colon adenocarcinoma, and ovary cystadenocarcinoma. In some embodiments, the cancer is pancreas ductal adenocarcinoma. In some embodiments, the cancer is a tumor that is poorly perfused and/or poorly vascularized.

In some embodiments, the cancer is pancreatic cancer, including for example pancreatic adenocarcinoma, pancreatic adenosquamous carcinoma, pancreatic squamous cell carcinoma, and pancreatic giant cell carcinoma. In some embodiments, the pancreatic cancer is exocrine pancreatic cancer. In some embodiments, the pancreatic cancer is endocrine pancreatic cancer (such as islet cell carcinoma). In some embodiments, the pancreatic cancer is advanced metastatic pancreatic cancer.

Other examples of cancers that may be treated by the methods of the invention include, but are not limited to, adenocortical carcinoma, agnogenic myeloid metaplasia, AIDS-related cancers (e.g., AIDS-related lymphoma), anal cancer, appendix cancer, astrocytoma (e.g., cerebellar and cerebral), basal cell carcinoma, bile duct cancer (e.g., extrahepatic), bladder cancer, bone cancer, (osteosarcoma and malignant fibrous histiocytoma), brain tumor (e.g., glioma, brain stem glioma, cerebellar or cerebral astrocytoma (e.g., pilocytic astrocytoma, diffuse astrocytoma, anaplastic (malignant) astrocytoma), malignant glioma, ependymoma, oligodenglioma, meningioma, craniopharyngioma, haemangioblastomas, medulloblastoma, supratentorial primitive neuroectodermal tumors, visual pathway and hypothalamic glioma, and glioblastoma), breast cancer, bronchial adenomas/carcinoids, carcinoid tumor (e.g., gastrointestinal carcinoid tumor), carcinoma of unknown primary, central nervous system lymphoma, cervical cancer, colon cancer, colorectal cancer, chronic myeloproliferative disorders, endometrial cancer (e.g., uterine cancer), ependymoma, esophageal cancer, Ewing's family of tumors, eye cancer (e.g., intraocular melanoma and retinoblastoma), gallbladder cancer, gastric (stomach) cancer, gastrointestinal carcinoid tumor, gastrointestinal stromal tumor (GIST), germ cell tumor, (e.g., extracranial, extragonadal, ovarian), gestational trophoblastic tumor, head and neck cancer, hepatocellular (liver) cancer (e.g., hepatic carcinoma and heptoma), hypopharyngeal cancer, islet cell carcinoma (endocrine pancreas), laryngeal cancer, laryngeal cancer, leukemia, lip and oral cavity cancer, oral cancer, liver cancer, lung cancer (e.g., small cell lung cancer, non-small cell lung cancer, adenocarcinoma of the lung, and squamous carcinoma of the lung), lymphoid neoplasm (e.g., lymphoma), medulloblastoma, ovarian cancer, mesothelioma, metastatic squamous neck cancer, mouth cancer, multiple endocrine neoplasia syndrome, myelodysplastic syndromes, myelodysplastic/myeloproliferative diseases, nasal cavity and paranasal sinus cancer, nasopharyngeal cancer, neuroblastoma, neuroendocrine cancer, oropharyngeal cancer, ovarian cancer (e.g., ovarian epithelial cancer, ovarian germ cell tumor, ovarian low malignant potential tumor), pancreatic cancer, parathyroid cancer, penile cancer, cancer of the peritoneal, pharyngeal cancer, pheochromocytoma, pineoblastoma and supratentorial primitive neuroectodermal tumors, pituitary tumor, pleuropulmonary blastoma, lymphoma, primary central nervous system lymphoma (microglioma), pulmonary lymphangiomyomatosis, rectal cancer, renal cancer, renal pelvis and ureter cancer (transitional cell cancer), rhabdomyosarcoma, salivary gland cancer, skin cancer (e.g., non-melanoma (e.g., squamous cell carcinoma), melanoma, and Merkel cell carcinoma), small intestine cancer, squamous cell cancer, testicular cancer, throat cancer, thymoma and thymic carcinoma, thyroid cancer, tuberous sclerosis, urethral cancer, vaginal cancer, vulvar cancer, Wilms' tumor, and post-transplant lymphoproliferative disorder (PTLD), abnormal vascular proliferation associated with phakomatoses, edema (such as that associated with brain tumors), and Meigs' syndrome.

In some embodiments, the cancer is a solid tumor (such as advanced solid tumor). Solid tumor includes, but is not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, Kaposi's sarcoma, soft tissue sarcoma, uterine sacronomasynovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma (including for example adenocarcinoma, clear cell renal cell carcinoma, papillary renal cell carcinoma, chromophobe renal cell carcinoma, collecting duct renal cell carcinoma, granular renal cell carcinoma, mixed granular renal cell carcinoma, renal angiomyolipomas, or spindle renal cell carcinoma.), hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

In some embodiments the lymphoid neoplasm (*e.g.,* lymphoma) is a B-cell neoplasm. Examples of B-cell neoplasms include, but are not limited to, precursor B-cell neoplasms (*e.g.,* precursor B-lymphoblastic leukemia/lymphoma) and peripheral B-cell neoplasms (*e.g.,* B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (*e.g.,* cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (*e.g.,* extranodal (*e.g.,* MALT-type +/- monocytoid B cells) and/or Nodal (*e.g.,* +/- monocytoid B cells)), splenic marginal zone lymphoma (*e.g.,* +/- villous lymphocytes), Hairy cell leukemia, plasmacytoma/plasma cell myeloma (*e.g.,* myeloma and multiple myeloma), diffuse large B-cell lymphoma (*e.g.,* primary mediastinal (thymic) B-cell lymphoma), intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia).

In some embodiments the lymphoid neoplasm (*e.g.,* lymphoma) is a T-cell and/or putative NK-cell neoplasm. Examples of T-cell and/or putative NK-cell neoplasms include, but are not limited to, precursor T-cell neoplasm (precursor T-lymphoblastic lymphoma/leukemia) and peripheral T-cell and NK-cell neoplasms (*e.g*., T-cell chronic lymphocytic leukemia/prolymphocytic leukemia, and large granular lymphocyte leukemia (LGL) (*e.g.,* T-cell type and/or NK-cell type), cutaneous T-cell lymphoma (*e.g.,* mycosis fungoides/Sezary syndrome), primary T-cell lymphomas unspecified (*e.g.,* cytological categories (*e.g.,* medium-sized cell, mixed medium and large cell), large cell, lymphoepitheloid cell, subtype hepatosplenic γδ T-cell lymphoma, and subcutaneous panniculitic T-cell lymphoma), angioimmunoblastic T-cell lymphoma (AILD), angiocentric lymphoma, intestinal T-cell lymphoma (*e.g.,* +/- enteropathy associated), adult T-cell lymphoma/leukemia (ATL), anaplastic large cell lymphoma (ALCL) (*e.g.,* CD30+, T- and null-cell types), anaplastic large-cell lymphoma, and Hodgkin's like).

In some embodiments the lymphoid neoplasm (*e.g.,* lymphoma) is Hodgkin's disease. For example, the Hodgkin's disease may be lymphocyte predominance, nodular sclerosis, mixed cellularity, lymphocyte depletion, and/or lymphocyte-rich.

In some embodiments, the cancer is leukemia. In some embodiments, the leukemia is chronic leukemia. Examples of chronic leukemia include, but are not limited to, chronic myelocytic I (granulocytic) leukemia, chronic myelogenous, and chronic lymphocytic leukemia (CLL). In some embodiments, the leukemia is acute leukemia. Examples of acute leukemia include, but are not limited to, acute lymphoblastic leukemia (ALL), acute myeloid leukemia, acute lymphocytic leukemia, and acute myelocytic leukemia (e.g., myeloblastic, promyelocytic, myelomonocytic, monocytic, and erythroleukemia).

In some embodiments, the cancer is liquid tumor or plasmacytoma. Plasmacytoma includes, but is not limited to, myeloma. Myeloma includes, but is not limited to, an extramedullary plasmacytoma, a solitary myeloma, and multiple myeloma. In some embodiments, the plasmacytoma is multiple myeloma.

In some embodiments, the cancer is multiple myeloma. Examples of multiple myeloma include, but are not limited to, IgG multiple myeloma, IgA multiple myeloma, IgD multiple myeloma, IgE multiple myeloma, and nonsecretory multiple myeloma. In some embodiments, the multiple myeloma is IgG multiple myeloma. In some embodiments, the multiple myeloma is IgA multiple myeloma. In some embodiments, the multiple myeloma is a smoldering or indolent multiple myeloma. In some embodiments, the multiple myeloma is progressive multiple myeloma. In some embodiments, multiple myeloma may be resistant to a drug, such as, but not limited to, bortezomib, dexamethasone (Dex-), doxorubicin (Dox-), and melphalan (LR).

### Modes of administration

The composition comprising nanoparticles comprising taxane (also referred to as "nanoparticle composition") and the other agent can be administered simultaneously (i.e., simultaneous administration) and/or sequentially (i.e., sequential administration).

In some embodiments, the nanoparticle composition and the other agent (including the specific agents described herein) are administered simultaneously. The term "simultaneous administration," as used herein, means that the nanoparticle composition and the other agent are administered with a time separation of no more than about 15 minute(s), such as no more than about any of 10, 5, or 1 minutes. When the drugs are administered simultaneously, the drug in the nanoparticles and the other agent may be contained in the same composition (e.g., a composition comprising both the nanoparticles and the other agent) or in separate compositions (e.g., the nanoparticles are contained in one composition and the other agent is contained in another composition).

In some embodiments, the nanoparticle composition and the other agent are administered sequentially. The term "sequential administration" as used herein means that the drug in the nanoparticle composition and the other agent are administered with a time separation of more than about 15 minutes, such as more than about any of 20, 30, 40, 50, 60 or more minutes. Either the nanoparticle composition or the other agent may be administered first. The nanoparticle composition and the other agent are contained in separate compositions, which may be contained in the same or different packages.

In some embodiments, the administration of the nanoparticle composition and the other agent are concurrent, i.e., the administration period of the nanoparticle composition and that of the other agent overlap with each other. In some embodiments, the nanoparticle composition is administered for at least one cycle (for example, at least any of 2, 3, or 4 cycles) prior to the administration of the other agent. In some embodiments, the other agent is administered for at least any of one, two, three, or four weeks. In some embodiments, the administrations of the nanoparticle composition and the other agent are initiated at about the same time (for example, within any one of 1, 2, 3, 4, 5, 6, or 7 days). In some embodiments, the administrations of the nanoparticle composition and the other agent are terminated at about the same time (for example, within any one of 1, 2, 3, 4, 5, 6, or 7 days). In some embodiments, the administration of the other agent continues (for example for about any one of 1, 2, 3, 4, 5, 6, 7, 8, 9, 10, 11, or 12 months) after the termination of the administration of the nanoparticle composition. In some embodiments, the administration of the other agent is initiated after (for example after about any one of 1, 2, 3, 4, 5, 6, 7, 8, 9, 10, 11, or we months) the initiation of the administration of the nanoparticle composition. In some embodiments, the administrations of the nanoparticle composition and the other agent are initiated and terminated at about the same time. In some embodiments, the administrations of the nanoparticle composition and the other agent are initiated at about the same time and the administration of the other agent continues (for example for about any one of 1, 2, 3, 4, 5, 6, 7, 8, 9, 10, 11, or 12 months) after the termination of the administration of the nanoparticle composition. In some embodiments, the administration of the nanoparticle composition and the other agent stop at about the same time and the administration of the other agent is initiated after (for example after about any one of 1, 2, 3, 4, 5, 6, 7, 8, 9, 10, 11, or we months) the initiation of the administration of the nanoparticle composition.

In some embodiments, the administration of the nanoparticle composition and the other agent are non-concurrent. For example, in some embodiments, the administration of the nanoparticle composition is terminated before the other agent is administered. In some embodiments, the administration of the other agent is terminated before the nanoparticle composition is administered. The time period between these two non-concurrent administrations can range from about two to eight weeks, such as about four weeks.

The dosing frequency of the drug-containing nanoparticle composition and the other agent may be adjusted over the course of the treatment, based on the judgment of the administering physician. When administered separately, the drug-containing nanoparticle composition and the other agent can be administered at different dosing frequency or intervals. For example, the drug-containing nanoparticle composition can be administered weekly, while another agent can be administered more or less frequently. In some embodiments, sustained continuous release formulation of the drug-containing nanoparticle and/or other agent may be used. Various formulations and devices for achieving sustained release are known in the art. Exemplary dosing frequencies are further provided herein.

The nanoparticle composition and the other agent can be administered using the same route of administration or different routes of administration. Exemplary administration routes are further provided herein. In some embodiments (for both simultaneous and sequential administrations), the taxane in the nanoparticle composition and the other agent are administered at a predetermined ratio. For example, in some embodiments, the ratio by weight of the taxane in the nanoparticle composition and the other agent is about 1 to 1. In some embodiments, the weight ratio may be between about 0.001 to about 1 and about 1000 to about 1, or between about 0.01 to about 1 and 100 to about 1. In some embodiments, the ratio by weight of the taxane in the nanoparticle composition and the other agent is less than about any of 100:1, 50:1, 30:1, 10:1, 9:1, 8:1, 7:1, 6:1, 5:1, 4:1, 3:1, 2:1, and 1:1 In some embodiments, the ratio by weight of the taxane in the nanoparticle composition and the other agent is more than about any of 1:1, 2:1, 3:1, 4:1, 5:1, 6:1, 7:1, 8:1, 9:1, 30:1, 50:1, 100:1. Other ratios are contemplated.

The doses required for the taxane and/or the other agent may (but not necessarily) be lower than what is normally required when each agent is administered alone. Thus, in some embodiments, a subtherapeutic amount of the drug in the nanoparticle composition and/or the other agent are administered. "Subtherapeutic amount" or "subtherapeutic level" refer to an amount that is less than therapeutic amount, that is, less than the amount normally used when the drug in the nanoparticle composition and/or the other agent are administered alone. The reduction may be reflected in terms of the amount administered at a given administration and/or the amount administered over a given period of time (reduced frequency).

In some embodiments, enough other agent is administered so as to allow reduction of the normal dose of the drug in the nanoparticle composition required to effect the same degree of treatment by at least about any of 5%, 10%, 20%, 30%, 50%, 60%, 70%, 80%, 90%, or more. In some embodiments, enough drug in the nanoparticle composition is administered so as to allow reduction of the normal dose of the other agent required to effect the same degree of treatment by at least about any of 5%, 10%, 20%, 30%, 50%, 60%, 70%, 80%, 90%, or more.

In some embodiments, the dose of both the taxane in the nanoparticle composition and the other agent are reduced as compared to the corresponding normal dose of each when administered alone. In some embodiments, both the taxane in the nanoparticle composition and the other agent are administered at a subtherapeutic, i.e., reduced, level. In some embodiments, the dose of the nanoparticle composition and/or the other agent is substantially less than the established maximum toxic dose (MTD). For example, the dose of the nanoparticle composition and/or the other agent is less than about 50%, 40%, 30%, 20%, or 10% of the MTD.

In some embodiments, the dose of taxane and/or the dose of the other agent is higher than what is normally required when each agent is administered alone. For example, in some embodiments, the dose of the nanoparticle composition and/or the other agent is substantially higher than the established maximum toxic dose (MTD). For example, the dose of the nanoparticle composition and/or the other agent is more than about 50%, 40%, 30%, 20%, or 10% of the MTD of the agent when administered alone.

In some embodiments, the amount of a taxane (e.g., paclitaxel) in the composition is included in any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of a taxane (e.g., paclitaxel) or derivative thereof in the effective amount of the composition (e.g., a unit dosage form) is in the range of about 5 mg to about 500 mg, such as about 30 mg to about 300 mg or about 50 mg to about 200 mg. In some embodiments, the concentration of the taxane (e.g., paclitaxel) in the composition is dilute (about 0.1 mg/ml) or concentrated (about 100 mg/ml), including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some embodiments, the concentration of the taxane (e.g., paclitaxel) is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of a taxane (e.g., paclitaxel) in the nanoparticle composition include, but are not limited to, at least about any of 25 mg/m², 30 mg/m², 50 mg/m², 60 mg/m², 75 mg/m², 80 mg/m², 90 mg/m², 100 mg/m², 120 mg/m², 125 mg/m², 150 mg/m², 160 mg/m², 175 mg/m², 180 mg/m², 200 mg/m², 210 mg/m², 220 mg/m², 250 mg/m², 260 mg/m², 300 mg/m², 350 mg/m², 400 mg/m², 500 mg/m², 540 mg/m², 750 mg/m², 1000 mg/m², or 1080 mg/m² of a taxane (e.g., paclitaxel). In various embodiments, the composition includes less than about any of 350 mg/m², 300 mg/m², 250 mg/m², 200 mg/m², 150 mg/m², 120 mg/m², 100 mg/m², 90 mg/m², 50 mg/m², or 30 mg/m² of a taxane (e.g., paclitaxel). In some embodiments, the amount of the taxane (e.g., paclitaxel) per administration is less than about any of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some embodiments, the effective amount of a taxane (e.g., paclitaxel) in the composition is included in any of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². In some embodiments, the effective amount of a taxane (e.g., paclitaxel) in the composition is about 5 to about 300 mg/m², such as about 20 to about 300 mg/m², about 50 to about 250 mg/m², about 100 to about 150 mg/m², about 120 mg/m², about 130 mg/m², or about 140 mg/m², or about 260 mg/m².

In some embodiments of any of the above aspects, the effective amount of a taxane (e.g., paclitaxel) in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, or 20 mg/kg. In various embodiments, the effective amount of a taxane (e.g., paclitaxel) in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of a taxane (e.g., paclitaxel).

Exemplary dosing frequencies for the nanoparticle composition (and as indicated below for the other agent) include, but are not limited to, weekly without break; weekly, three out of four weeks; once every three weeks; once every two weeks; weekly, two out of three weeks. In some embodiments, the composition is administered about once every 2 weeks, once every 3 weeks, once every 4 weeks, once every 6 weeks, or once every 8 weeks. In some embodiments, the composition is administered at least about any of 1x, 2x, 3x, 4x, 5x, 6x, or 7x (i.e., daily) a week, or three times daily, two times daily. In some embodiments, the intervals between each administration are less than about any of 6 months, 3 months, 1 month, 20 days, 15 days, 12 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week.

In some embodiments, the taxane in the nanoparticle composition is administered weekly. In some embodiments, the taxane in a nanoparticle composition is administered every two weeks. In some embodiments, the taxane in the nanoparticle composition is administered every three weeks. In some embodiments, the other agent is administered 1x, 2x, 3x, 4x, 5x, 6x, or 7 times a week. In some embodiments, the other agent is administered every two weeks or two out of three weeks. In some embodiments, the taxane is paclitaxel. In some embodiment, the other agent is vorinostat. In some embodiments of the above dosages and/or administrations, the taxane is paclitaxel and the other agent is vorinostat.

The administration of the nanoparticle composition (and for the other agent) can be extended over an extended period of time, such as from about a month up to about seven years. In some embodiments, the composition is administered over a period of at least about any of 2, 3, 4, 5, 6, 7, 8, 9, 10, 11, 12, 18, 24, 30, 36, 48, 60, 72, or 84 months. In some embodiments, the taxane (e.g., paclitaxel) is administered over a period of at least one month, wherein the interval between each administration is no more than about a week, and wherein the dose of the taxane (e.g., paclitaxel) at each administration is about 0.25 mg/m² to about 75 mg/m², such as about 0.25 mg/m², to about 25 mg/m² or about 25 mg/m² to about 50 mg/m².

In some embodiments, the dosage of a taxane (e.g., paclitaxel) in a nanoparticle composition can be in the range of 5-400 mg/m² when given on a 3 week schedule, or 5-250 mg/m² when given on a weekly schedule. For example, the amount of a taxane (e.g., paclitaxel) can be about 60 to about 300 mg/m² (e.g., about 260 mg/m²) when given on a three week schedule.

Other exemplary dosing schedules for the administration of the nanoparticle composition (e.g., paclitaxel/albumin nanoparticle composition) include, but are not limited to, 100 mg/m², weekly, without break; 75 mg/m² weekly, 3 out of four weeks; 100 mg/m², weekly, 3 out of 4 weeks; 125 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m², twice a week; and 150-250 mg/m² twice a week. The dosing frequency of the composition may be adjusted over the course of the treatment based on the judgment of the administering physician.

In some embodiments, the individual is treated for at least about any of one, two, three, four, five, six, seven, eight, nine, or ten treatment cycles. The compositions described herein allow infusion of the composition to an individual over an infusion time that is shorter than about 24 hours. For example, in some embodiments, the composition is administered over an infusion period of less than about any of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes.

Other exemplary dose of the taxane (in some embodiments paclitaxel) in the nanoparticle composition include, but is not limited to, about any of 50 mg/m², 60 mg/m², 75 mg/m², 80 mg/m², 90 mg/m², 100 mg/m², 120 mg/m², 160 mg/m², 175 mg/m², 200 mg/m², 210 mg/m², 220 mg/m², 260 mg/m², and 300 mg/m². For example, the dosage of paclitaxel in a nanoparticle composition can be in the range of about 100-400 mg/m² when given on a 3 week schedule, or about 50-250 mg/m² when given on a weekly schedule.

The dosing frequency of the other agent can be the same or different from that of the nanoparticle composition. Exemplary frequencies are provided above. As further example, the other agent can be administered three times a day, two times a day, daily, 6 times a week, 5 times a week, 4 times a week, 3 times a week, two times a week, weekly. In some embodiments, the other agent is administered twice daily or three times daily. Exemplary amounts of the other agent include, but are not limited to, any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg, about 500 to about 550 mg, about 550 to about 600 mg, about 600 to about 650 mg, about 650 to about 700 mg, about 700 mg to about 800 mg, about 800 mg to about 850 mg, about 850 mg to about 900 mg, about 900 mg to about 950 mg, about 950 mg to about 1000 mg. For example, the other agent can be administered at a dose of about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). For example, in some embodiments, vorinostat is administered (for example by oral administration) at about 20-100 mg/kg (including for example 30 mg/kg, 40 mg/kg, 50 mg/kg, 60 mg/kg, 70 mg/kg, 80 mg/kg), three times a week. In some embodiments, azacitidine is administered (for example by intraperitoneal administration) at about 20-200 mg/kg/day (including for example 50 mg/kg/day, 80 mg/kg/day, 100 mg/kg/day, 120 mg/kg/day, 140 mg/kg/day, 180 mg/kg/day). In some embodiments, decitabine is administered (for example by intraperitoneal administration) at about 0.75-4 mg/kg/day (including for example 1.0 mg/kg/day, 1.5 mg/kg/day, 2.00 mg/kg/day, 2.5 mg/kg/day, 3.0 mg/kg/day, 3.5 mg/kg/day).

In some embodiments, the effective amount of taxane in the nanoparticle composition is between about 45 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 80 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 80 mg/m² to about 300 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 150 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 80 mg/m² to about 150 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane (e.g., paclitaxel) in the nanoparticle composition is about 100 mg/m². In some embodiments, the effective amount of taxane in the nanoparticle composition is between about 170 mg/m² to about 200 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 200 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane (e.g., paclitaxel) in the nanoparticle composition is about 260 mg/m². In some embodiments of any of the above methods, the effective amount of the other agent is about 20-30 mg/kg, about 30-40 mg/kg, about 40-50 mg/kg, about 50-60 mg/kg, about 60-70 mg/kg, about 70-80 mg/kg, about 80-100 mg/kg, or about 100-120 mg/kg.

In some embodiments, the effective amount of taxane in the nanoparticle composition is between about 45 mg/m² to about 350 mg/m² and the effective amount of the other agent is about 80 mg to about 1000 mg (including for example about 80 to about 100 mg, about 100 to about 200 mg, about 200 to about 300 mg, about 300 to about 400 mg, about 400 to about 500 mg, about 500 to about 600 mg, about 600 to about 700 mg, about 700 to about 800 mg, about 800 to about 900 mg, about 900 mg to about 1000 mg). In some embodiments, the effective amount of taxane in the nanoparticle composition is between about 80 mg/m² to about 350 mg/m² and the effective amount of the other agent is about 80 mg to about 1000 mg (including for example about 80 to about 100 mg, about 100 to about 200 mg, about 200 to about 300 mg, about 300 to about 400 mg, about 400 to about 500 mg, about 500 to about 600 mg, about 600 to about 700 mg, about 700 to about 800 mg, about 800 to about 900 mg, about 900 mg to about 1000 mg). In some embodiments, the effective amount of taxane in the nanoparticle composition is between about 80 mg/m² to about 300 mg/m² and the effective amount of the other agent is about 80 mg to about 1000 mg (including for example about 80 to about 100 mg, about 100 to about 200 mg, about 200 to about 300 mg, about 300 to about 400 mg, about 400 to about 500 mg, about 500 to about 600 mg, about 600 to about 700 mg, about 700 to about 800 mg, about 800 to about 900 mg, about 900 mg to about 1000 mg). In some embodiments, the effective amount of taxane in the nanoparticle composition is between about 150 mg/m² to about 350 mg/m² and the effective amount of the other agent is about 80 mg to about 1000 mg (including for example about 80 to about 100 mg, about 100 to about 200 mg, about 200 to about 300 mg, about 300 to about 400 mg, about 400 to about 500 mg, about 500 to about 600 mg, about 600 to about 700 mg, about 700 to about 800 mg, about 800 to about 900 mg, about 900 mg to about 1000 mg). In some embodiments, the effective amount of taxane in the nanoparticle composition is between about 80 mg/m² to about 150 mg/m² and the effective amount of the other agent is about 80 mg to about 1000 mg (including for example about 80 to about 100 mg, about 100 to about 200 mg, about 200 to about 300 mg, about 300 to about 400 mg, about 400 to about 500 mg, about 500 to about 600 mg, about 600 to about 700 mg, about 700 to about 800 mg, about 800 to about 900 mg, about 900 mg to about 1000 mg). In some embodiments, the effective amount of taxane in the nanoparticle composition is between about 170 mg/m² to about 200 mg/m² and the effective amount of the other agent is about 80 mg to about 1000 mg (including for example about 80 to about 100 mg, about 100 to about 200 mg, about 200 to about 300 mg, about 300 to about 400 mg, about 400 to about 500 mg, about 500 to about 600 mg, about 600 to about 700 mg, about 700 to about 800 mg, about 800 to about 900 mg, about 900 mg to about 1000 mg). In some embodiments, the effective amount of taxane in the nanoparticle composition is between about 200 mg/m² to about 350 mg/m² and the effective amount of the other agent is about 80 mg to about 1000 mg (including for example about 80 to about 100 mg, about 100 to about 200 mg, about 200 to about 300 mg, about 300 to about 400 mg, about 400 to about 500 mg, about 500 to about 600 mg, about 600 to about 700 mg, about 700 to about 800 mg, about 800 to about 900 mg, about 900 mg to about 1000 mg). In some embodiments, the effective amount of taxane (e.g., paclitaxel) in the nanoparticle composition is about 100 mg/m². In some embodiments of any of the above methods, the effective amount of the other agent is about 100-200 mg, about 200-300 mg, about 300-400 mg, about 400-500 mg.

In some embodiments, the effective amount of paclitaxel in the nanoparticle composition is about 100 mg/m² and the effective amount of the other agent (such as vorinostat) is about 400 mg.

In some embodiments, the effective amount of paclitaxel in the nanoparticle composition is about 50-300 mg/m² (including for example about 100 mg/m²) and the effective amount of the other agent (such as azacitidine) is about 10-200 mg/mw (including for example about 50-100 mg/m² or for example about 75 mg/m²). In some embodiments, the effective amount of paclitaxel in the nanoparticle composition is about 50-300 mg/m² (including for example about 100 mg/m²) and the effective amount of the other agent (such as decitabine) is about 10-200 mg/m² (including for example about 50-100 mg/m² or for example about 75 mg/m²).

The nanoparticle composition (and the other agent) described herein can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some embodiments, sustained continuous release formulation of the composition may be used. In one variation of the invention, nanoparticles (such as albumin nanoparticles) can be administered by any acceptable route including, but not limited to, orally, intramuscularly, transdermally, intravenously, through an inhaler or other air borne delivery systems and the like.

A combination of the administration configurations described herein can be used. The combination therapy methods described herein may be performed alone or in conjunction with another therapy, such as surgery, radiation, chemotherapy, immunotherapy, gene therapy, and the like. Additionally, a person having a greater risk of developing the proliferative disease may receive treatments to inhibit or and/or delay the development of the disease.

As will be understood by those of ordinary skill in the art, the appropriate doses of other agents will be approximately those already employed in clinical therapies wherein the other agent are administered alone or in combination with other agents. Variation in dosage will likely occur depending on the condition being treated. As described above, in some embodiments, the other agents may be administered at a reduced level.

### Nanoparticle compositions

The nanoparticle compositions described herein comprise nanoparticles comprising (in various embodiments consisting essentially of) a taxane (such as paclitaxel) and a carrier protein (such as albumin). Nanoparticles of poorly water soluble drugs (such as taxane) have been disclosed in, for example, U.S. Pat. Nos. 5,916,596; 6,506,405; 6,749,868, 6,537,579 and 7,820,788 and also in U.S. Pat. Pub. Nos. 2006/0263434, and 2007/0082838; PCT Patent Application WO08/137148.

In some embodiments, the composition comprises nanoparticles with an average or mean diameter of no greater than about 1000 nanometers (nm), such as no greater than about any of 900, 800, 700, 600, 500, 400, 300, 200, and 100 nm. In some embodiments, the average or mean diameters of the nanoparticles is no greater than about 200 nm. In some embodiments, the average or mean diameters of the nanoparticles is no greater than about 150 nm. In some embodiments, the average or mean diameters of the nanoparticles is no greater than about 100 nm. In some embodiments, the average or mean diameter of the nanoparticles is about 20 to about 400 nm. In some embodiments, the average or mean diameter of the nanoparticles is about 40 to about 200 nm. In some embodiments, the nanoparticles are sterile-filterable.

In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 400 nm, including for example about 20 to about 200 nm, about 40 to about 200 nm, about 30 to about 180 nm, and any one of about 40 to about 150, about 50 to about 120, and about 60 to about 100 nm.

In some embodiments, the carrier protein has sulfhydral groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the taxane (such as paclitaxel) coated with a carrier protein, such as albumin (e.g., human serum albumin). In some embodiments, the composition comprises taxane in both nanoparticle and non-nanoparticle forms, wherein at least about any one of 50%, 60%, 70%, 80%, 90%, 95%, or 99% of the taxane in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of taxane that is substantially free of polymeric materials (such as polymeric matrix).

In some embodiments, the nanoparticle composition is substantially free (such as free) of surfactants (such as Cremophor®, Tween 80, or other organic solvents used for the administration of taxanes). In some embodiments, the nanoparticle composition contains less than about any one of 20%, 15%, 10%, 7.5%, 5%, 2.5%, or 1% organic solvent. In some embodiments, the weight ratio of carrier protein (such as albumin) and taxane in the nanoparticle composition is about 18:1 or less, such as about 15:1 or less, for example about 10:1 or less. In some embodiments, the weight ratio of carrier protein (such as albumin) and taxane in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of carrier protein and taxane in the nanoparticle portion of the composition is about any one of 1:2, 1:3, 1:4, 1:5, 1:10, 1:15, or less. In some embodiments, the weight ratio of the carrier protein (such as albumin) and the taxane in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the nanoparticle composition comprises one or more of the above characteristics.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

The nanoparticles described herein comprise a taxane and a carrier protein. The term "proteins" refers to polypeptides or polymers of amino acids of any length (including full length or fragments), which may be linear or branched, comprise modified amino acids, and/or be interrupted by non-amino acids. The term also encompasses an amino acid polymer that has been modified naturally or by intervention; for example, disulfide bond formation, glycosylation, lipidation, acetylation, phosphorylation, or any other manipulation or modification. Also included within this term are, for example, polypeptides containing one or more analogs of an amino acid (including, for example, unnatural amino acids, etc.), as well as other modifications known in the art. The proteins described herein may be naturally occurring, i.e., obtained or derived from a natural source (such as blood), or synthesized (such as chemically synthesized or by synthesized by recombinant DNA techniques).

Examples of suitable carrier proteins include proteins normally found in blood or plasma, which include, but are not limited to, albumin, immunoglobulin including IgA, lipoproteins, apolipoprotein B, alpha-acid glycoprotein, beta-2-macroglobulin, thyroglobulin, transferin, fibronectin, factor VII, factor VIII, factor IX, factor X, and the like. In some embodiments, the carrier protein is non-blood protein, such as casein, α-lactalbumin, and β-lactoglobulin. The carrier proteins may either be natural in origin or synthetically prepared. In some embodiments, the pharmaceutically acceptable carrier comprises albumin, such as human serum albumin. Human serum albumin (HSA) is a highly soluble globular protein of Mᵣ 65K and consists of 585 amino acids. HSA is the most abundant protein in the plasma and accounts for 70-80 % of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (see, e.g., Tullis, JAMA, 237, 355-360, 460-463, (1977)) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Other albumins are contemplated, such as bovine serum albumin. Use of such non-human albumins could be appropriate, for example, in the context of use of these compositions in non-human mammals, such as the veterinary (including domestic pets and agricultural context).

Human serum albumin (HSA) has multiple hydrophobic binding sites (a total of eight for fatty acids, an endogenous ligand of HSA) and binds a diverse set of taxanes, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (198a), Vorum, Dan. Med. Bull., 46, 379-99 (1999), Kragh-Hansen, Dan. Med. Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (199b), and Carter et al., Adv. Protein. Chem., 45, 153-203 (1994)). Paclitaxel and propofol have been shown to bind HSA (see, e.g., Paal et al., Eur. J. Biochem., 268(7), 2187-91 (200a), Purcell et al., Biochim. Biophys. Acta, 1478(a), 61-8 (2000), Altmayer et al., Arzheimittelforschuhg, 45, 1053-6 (1995), and Garrido et al., Rev. Esp. Anestestiol. Reanim., 41, 308-12 (1994)). In addition, docetaxel has been shown to bind to human plasma proteins (see, e.g., Urien et al., Invest. New Drugs, 14(b), 147-51 (1996)).

The carrier protein (such as albumin) in the composition generally serves as a carrier for the taxane, i.e., the carrier protein in the composition makes the taxane more readily suspendable in an aqueous medium or helps maintain the suspension as compared to compositions not comprising a carrier protein. This can avoid the use of toxic solvents (or surfactants) for solubilizing the taxane, and thereby can reduce one or more side effects of administration of the taxane into an individual (such as a human). Thus, in some embodiments, the composition described herein is substantially free (such as free) of surfactants, such as Cremophor (including Cremophor EL^{®} (BASF)). In some embodiments, the nanoparticle composition is substantially free (such as free) of surfactants. A composition is "substantially free of Cremophor" or "substantially free of surfactant" if the amount of Cremophor or surfactant in the composition is not sufficient to cause one or more side effect(s) in an individual when the nanoparticle composition is administered to the individual.

The amount of carrier protein in the composition described herein will vary depending on other components in the composition. In some embodiments, the composition comprises a carrier protein in an amount that is sufficient to stabilize the taxane in an aqueous suspension, for example, in the form of a stable colloidal suspension (such as a stable suspension of nanoparticles). In some embodiments, the carrier protein is in an amount that reduces the sedimentation rate of the taxane in an aqueous medium. For particle-containing compositions, the amount of the carrier protein also depends on the size and density of nanoparticles of the taxane.

A taxane is "stabilized" in an aqueous suspension if it remains suspended in an aqueous medium (such as without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (such as human). Stability of the suspension is generally (but not necessarily) evaluated at a storage temperature (such as room temperature (such as 20-25 °C) or refrigerated conditions (such as 4 °C)). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40°C.

In some embodiments, the carrier protein is present in an amount that is sufficient to stabilize the taxane in an aqueous suspension at a certain concentration. For example, the concentration of the taxane in the composition is about 0.1 to about 100 mg/ml, including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg /ml. In some embodiments, the concentration of the taxane is at least about any of 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, and 50 mg/ml. In some embodiments, the carrier protein is present in an amount that avoids use of surfactants (such as Cremophor), so that the composition is free or substantially free of surfactant (such as Cremophor).

In some embodiments, the composition, in liquid form, comprises from about 0.1% to about 50% (w/v) (e.g. about 0.5% (w/v), about 5% (w/v), about 10% (w/v), about 15% (w/v), about 20% (w/v), about 30% (w/v), about 40% (w/v), or about 50% (w/v)) of carrier protein. In some embodiments, the composition, in liquid form, comprises about 0.5% to about 5% (w/v) of carrier protein.

In some embodiments, the weight ratio of carrier protein, e.g., albumin, to the taxane in the nanoparticle composition is such that a sufficient amount of taxane binds to, or is transported by, the cell. While the weight ratio of carrier protein to taxane will have to be optimized for different carrier protein and taxane combinations, generally the weight ratio of carrier protein, e.g., albumin, to taxane (w/w) is about 0.01:1 to about 100:1, about 0.02:1 to about 50:1, about 0.05:1 to about 20:1, about 0.1:1 to about 20:1, about 1:1 to about 18:1, about 2:1 to about 15:1, about 3:1 to about 12:1, about 4:1 to about 10:1, about 5:1 to about 9:1, or about 9:1. In some embodiments, the carrier protein to taxane weight ratio is about any of 18:1 or less, 15:1 or less, 14:1 or less, 13:1 or less, 12:1 or less, 11:1 or less, 10:1 or less, 9:1 or less, 8:1 or less, 7:1 or less, 6:1 or less, 5:1 or less, 4:1 or less, and 3:1 or less. In some embodiments, the weight ratio of the carrier protein (such as albumin) and the taxane in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the carrier protein allows the composition to be administered to an individual (such as human) without significant side effects. In some embodiments, the carrier protein (such as albumin) is in an amount that is effective to reduce one or more side effects of administration of the taxane to a human. The term "reducing one or more side effects of administration of the taxane" refers to reduction, alleviation, elimination, or avoidance of one or more undesirable effects caused by the taxane, as well as side effects caused by delivery vehicles (such as solvents that render the taxanes suitable for injection) used to deliver the taxane. Such side effects include, for example, myelosuppression, neurotoxicity, hypersensitivity, inflammation, venous irritation, phlebitis, pain, skin irritation, peripheral neuropathy, neutropenic fever, anaphylactic reaction, venous thrombosis, extravasation, and combinations thereof. These side effects, however, are merely exemplary and other side effects, or combination of side effects, associated with taxanes can be reduced.

In some embodiments, the composition comprises Abraxane^{®} (*nab-*paclitaxel). Abraxane^{®} is a formulation of paclitaxel stabilized by human albumin USP, which can be dispersed in directly injectable physiological solution. When dispersed in a suitable aqueous medium such as 0.9% sodium chloride injection or 5% dextrose injection, Abraxane^{®} forms a stable colloidal suspension of paclitaxel. The mean particle size of the nanoparticles in the colloidal suspension is about 130 nanometers. Since HSA is freely soluble in water, Abraxane^{®} can be reconstituted in a wide range of concentrations ranging from dilute (0.1 mg/ml paclitaxel) to concentrated (20 mg/ml paclitaxel), including for example about 2 mg/ml to about 8 mg/ml, about 5 mg/ml.

Methods of making nanoparticle compositions are known in the art. For example, nanoparticles containing taxanes (such as paclitaxel) and carrier protein (such as albumin) can be prepared under conditions of high shear forces (e.g., sonication, high pressure homogenization, or the like). These methods are disclosed in, for example, U.S. Pat. Nos. 5,916,596; 6,506,405; 6,749,868, 6,537,579, 7,820,788 and also in U.S. Pat. Pub. No. 2007/0082838, 2006/0263434and PCT Application WO08/137148.

Briefly, the taxane (such as paclitaxel) is dissolved in an organic solvent, and the solution can be added to a human serum albumin solution. The mixture is subjected to high pressure homogenization. The organic solvent can then be removed by evaporation. The dispersion obtained can be further lyophilized. Suitable organic solvent include, for example, ketones, esters, ethers, chlorinated solvents, and other solvents known in the art. For example, the organic solvent can be methylene chloride or chloroform/ethanol (for example with a ratio of 1:9, 1:8, 1:7, 1:6, 1:5, 1:4, 1:3, 1:2, 1:1, 2:1, 3:1, 4:1, 5:1, 6:1, 7:1, 8:1, or 9:1.

### Other components in the nanoparticle compositions

The nanoparticles described herein can be present in a composition that include other agents, excipients, or stabilizers. For example, to increase stability by increasing the negative zeta potential of nanoparticles, certain negatively charged components may be added. Such negatively charged components include, but are not limited to bile salts of bile acids consisting of glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylcholine stearoyloleoylphosphatidylcholine, stearoylarachidoylphosphatidylcholine, and dipalmitoylphosphatidylcholine. Other phospholipids including L-α-dimyristoylphosphatidylcholine (DMPC), dioleoylphosphatidylcholine (DOPC), distearyolphosphatidylcholine (DSPC), hydrogenated soy phosphatidylcholine (HSPC), and other related compounds. Negatively charged surfactants or emulsifiers are also suitable as additives, e.g., sodium cholesteryl sulfate and the like.

In some embodiments, the composition is suitable for administration to a human. In some embodiments, the composition is suitable for administration to a mammal such as, in the veterinary context, domestic pets and agricultural animals. There are a wide variety of suitable formulations of the nanoparticle composition (see, e.g., U.S. Pat. Nos. 5,916,596, 6,096,331 and 7,820,788). The following formulations and methods are merely exemplary and are in no way limiting. Formulations suitable for oral administration can consist of (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Examples of suitable carriers, excipients, and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. The formulations can additionally include lubricating agents, wetting agents, emulsifying and suspending agents, preserving agents, sweetening agents or flavoring agents.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Injectable formulations are preferred.

In some embodiments, the composition is formulated to have a pH range of about 4.5 to about 9.0, including for example pH ranges of any of about 5.0 to about 8.0, about 6.5 to about 7.5, and about 6.5 to about 7.0. In some embodiments, the pH of the composition is formulated to no less than about 6, including for example no less than about any of 6.5, 7, or 8 (such as about 8). The composition can also be made to be isotonic with blood by the addition of a suitable tonicity modifier, such as glycerol.

### Kits, medicines, and compositions

The invention also provides compositions (such as pharmaceutical compositions), medicine, kits, and unit dosages useful for methods described herein. Also provided are any use described herein whether in the context of use as a medicament and/or use for manufacture of a medicament.

Kits of the invention include one or more containers comprising taxane-containing nanoparticle compositions (or unit dosage forms and/or articles of manufacture) and/or at least one other agent that modifies the epigenetics in a cell, and in some embodiments, further comprise instructions for use in accordance with any of the methods described herein. The kit may further comprise a description of selection an individual suitable or treatment. Instructions supplied in the kits of the invention are typically written instructions on a label or package insert (e.g., a paper sheet included in the kit), but machine-readable instructions (e.g., instructions carried on a magnetic or optical storage disk) are also acceptable.

In some embodiments, the kit comprises a) a composition comprising nanoparticles comprising a taxane (such as paclitaxel) and a carrier protein (such as albumin), and b) an effective amount of at least one other agent that modifies the epigenetics in a cell. In some embodiments, the kit comprises a) a composition comprising nanoparticles comprising a taxane (such as paclitaxel) and a carrier protein (such as albumin), b) an effective amount of at least one other agent that modifies the epigenetics in a cell, and c) instructions for administering the nanoparticles and the other agents simultaneously, sequentially, or concurrently for treatment of a proliferative disease (such as cancer). In some embodiments, the taxane is any of paclitaxel, docetaxel, and ortataxel. In some embodiments, the kit comprises nanoparticles comprising a) a composition comprising nanoparticles comprising paclitaxel coated with an albumin (such as Abraxane^{®}), b) an effective amount of at least one other agent that modifies the epigenetics in a cell, and c) instructions for administering the nanoparticles and the other agents simultaneously, sequentially, and/or concurrently, for the effective treatment of a proliferative disease (such as cancer).

In some embodiments, the kit comprises a) a composition comprising nanoparticles comprising a taxane (such as paclitaxel) coated with a carrier protein (such as albumin), b) a composition comprising nanoparticles comprising at least one other agent that modifies the epigenetics in a cell and a carrier protein (such as albumin), and c) instructions for administering the nanoparticle compositions simultaneously, sequentially, and/or concurrently, for treatment of a proliferative disease (such as cancer). In some embodiments, the kit comprises nanoparticles comprising a) a composition comprising nanoparticles comprising paclitaxel coated with an albumin (such as Abraxane^{®}), b) a composition comprising nanoparticles comprising at least one other agent that modifies the epigenetics in a cell and a carrier protein (such as albumin), and c) instructions for administering the nanoparticle compositions simultaneously, sequentially, and/or concurrently, for the effective treatment of a proliferative disease (such as cancer).

The nanoparticles and the other agents can be present in separate containers or in a single container. It is understood that the kit may comprise one distinct composition or two or more compositions wherein one composition comprises nanoparticles and one composition comprises an other agent.

The kits of the invention are in suitable packaging. Suitable packaging include, but is not limited to, vials, bottles, jars, flexible packaging (e.g., sealed Mylar or plastic bags), and the like. Kits may optionally provide additional components such as buffers and interpretative information. The present application thus also provides articles of manufacture, which include vials (such as sealed vials), bottles, jars, flexible packaging, and the like.

The instructions relating to the use of the nanoparticle compositions generally include information as to dosage, dosing schedule, and route of administration for the intended treatment. The containers may be unit doses, bulk packages (e.g., multi-dose packages) or sub-unit doses. For example, kits may be provided that contain sufficient dosages of the taxane (such as taxane) as disclosed herein to provide effective treatment of an individual for an extended period, such as any of a week, 2 weeks, 3 weeks, 4 weeks, 6 weeks, 8 weeks, 3 months, 4 months, 5 months, 7 months, 8 months, 9 months, or more. Kits may also include multiple unit doses of the taxane and pharmaceutical compositions and instructions for use and packaged in quantities sufficient for storage and use in pharmacies, for example, hospital pharmacies and compounding pharmacies.

Also provided are medicines for treating proliferative diseases. In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the kit comprises nanoparticles comprising a) a composition comprising nanoparticles comprising paclitaxel coated with an albumin (such as Abraxane^{®}), and b) at least one other agent that modifies the epigenetics in a cell, and c) instructions for administering the nanoparticles and the other agents simultaneously, sequentially, and/or concurrently, for the effective treatment of a proliferative disease (such as cancer).

Also provided are medicines for treating a lymphoid neoplasm (for example, CLL/SLL or lymphoma, such as refractory DLBC lymphoma). In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the at least one other agent that modifies the epigenetics in a cell is azacitidine. In some embodiments there is provided a kit comprising nanoparticles comprising a) a composition comprising nanoparticles comprising paclitaxel coated with an albumin (such as Abraxane^{®}), and b) azacitidine, and c) instructions for administering the nanoparticles and the azacitidine simultaneously, sequentially, and/or concurrently, for the effective treatment of lymphoid neoplasm (for example, CLL/SLL or lymphoma, such as refractory DLBC lymphoma). In some embodiments, the at least one other agent that modifies the epigenetics in a cell is decitabine. In some embodiments there is provided a kit comprising nanoparticles comprising a) a composition comprising nanoparticles comprising paclitaxel coated with an albumin (such as Abraxane^{®}), and b) decitabine, and c) instructions for administering the nanoparticles and the decitabine simultaneously, sequentially, and/or concurrently, for the effective treatment of lymphoid neoplasm (for example, CLL/SLL or lymphoma, such as refractory DLBC lymphoma).

Also provided are medicines for treating ovarian cancer. In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the at least one other agent that modifies the epigenetics in a cell is azacitidine. In some embodiments there is provided a kit comprising nanoparticles comprising a) a composition comprising nanoparticles comprising paclitaxel coated with an albumin (such as Abraxane^{®}), and b) azacitidine, and c) instructions for administering the nanoparticles and the azacitidine simultaneously, sequentially, and/or concurrently, for the effective treatment of ovarian cancer. In some embodiments, the at least one other agent that modifies the epigenetics in a cell is decitabine. In some embodiments there is provided a kit comprising nanoparticles comprising a) a composition comprising nanoparticles comprising paclitaxel coated with an albumin (such as Abraxane^{®}), and b) decitabine, and c) instructions for administering the nanoparticles and the decitabine simultaneously, sequentially, and/or concurrently, for the effective treatment of ovarian cancer.

Also provided are medicines for treating endometrial cancer (e.g. uterine cancer). In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the at least one other agent that modifies the epigenetics in a cell is azacitidine. In some embodiments there is provided a kit comprising nanoparticles comprising a) a composition comprising nanoparticles comprising paclitaxel coated with an albumin (such as Abraxane^{®}), and b) azacitidine, and c) instructions for administering the nanoparticles and the azacitidine simultaneously, sequentially, and/or concurrently, for the effective treatment of endometrial cancer (e.g. uterine cancer). In some embodiments, the at least one other agent that modifies the epigenetics in a cell is decitabine. In some embodiments there is provided a kit comprising nanoparticles comprising a) a composition comprising nanoparticles comprising paclitaxel coated with an albumin (such as Abraxane^{®}), and b) decitabine, and c) instructions for administering the nanoparticles and the decitabine simultaneously, sequentially, and/or concurrently, for the effective treatment of endometrial cancer (e.g. uterine cancer).

Also provided are medicines for treating lung cancer. In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the at least one other agent that modifies the epigenetics in a cell is azacitidine. In some embodiments there is provided a kit comprising nanoparticles comprising a) a composition comprising nanoparticles comprising paclitaxel coated with an albumin (such as Abraxane^{®}), and b) azacitidine, and c) instructions for administering the nanoparticles and the azacitidine simultaneously, sequentially, and/or concurrently, for the effective treatment of lung cancer. In some embodiments, the at least one other agent that modifies the epigenetics in a cell is decitabine. In some embodiments there is provided a kit comprising nanoparticles comprising a) a composition comprising nanoparticles comprising paclitaxel coated with an albumin (such as Abraxane^{®}), and b) decitabine, and c) instructions for administering the nanoparticles and the decitabine simultaneously, sequentially, and/or concurrently, for the effective treatment of lung cancer.

Also provided are medicines for treating sarcoma. In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the at least one other agent that modifies the epigenetics in a cell is azacitidine. In some embodiments there is provided a kit comprising nanoparticles comprising a) a composition comprising nanoparticles comprising paclitaxel coated with an albumin (such as Abraxane^{®}), and b) azacitidine, and c) instructions for administering the nanoparticles and the azacitidine simultaneously, sequentially, and/or concurrently, for the effective treatment of sarcoma. In some embodiments, the at least one other agent that modifies the epigenetics in a cell is decitabine. In some embodiments there is provided a kit comprising nanoparticles comprising a) a composition comprising nanoparticles comprising paclitaxel coated with an albumin (such as Abraxane^{®}), and b) decitabine, and c) instructions for administering the nanoparticles and the decitabine simultaneously, sequentially, and/or concurrently, for the effective treatment of sarcoma.

Also provided are medicines for treating pancreatic cancer. In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the at least one other agent that modifies the epigenetics in a cell is azacitidine. In some embodiments there is provided a kit comprising nanoparticles comprising a) a composition comprising nanoparticles comprising paclitaxel coated with an albumin (such as Abraxane^{®}), and b) azacitidine, and c) instructions for administering the nanoparticles and the azacitidine simultaneously, sequentially, and/or concurrently, for the effective treatment of pancreatic cancer. In some embodiments, the at least one other agent that modifies the epigenetics in a cell is decitabine. In some embodiments there is provided a kit comprising nanoparticles comprising a) a composition comprising nanoparticles comprising paclitaxel coated with an albumin (such as Abraxane^{®}), and b) decitabine, and c) instructions for administering the nanoparticles and the decitabine simultaneously, sequentially, and/or concurrently, for the effective treatment of pancreatic cancer.

Also provided are medicines for treating breast cancer (for example, HER2 negative breast cancer or for example, triple negative breast cancer). In some embodiments, the medicine comprises a) a composition comprising nanoparticles comprising a taxane (such as paclitaxel) and a carrier protein (such as albumin), and b) at least one other agent that modifies the epigenetics in a cell. In some embodiments, the taxane is any of paclitaxel, docetaxel, and ortataxel. In some embodiments, the at least one other agent that modifies the epigenetics in a cell is azacitidine. In some embodiments there is provided a kit comprising nanoparticles comprising a) a composition comprising nanoparticles comprising paclitaxel coated with an albumin (such as Abraxane^{®}), and b) azacitidine, and c) instructions for administering the nanoparticles and the azacitidine simultaneously, sequentially, and/or concurrently, for the effective treatment of breast cancer (for example, HER2 negative breast cancer or for example, triple negative breast cancer). In some embodiments, the at least one other agent that modifies the epigenetics in a cell is decitabine. In some embodiments there is provided a kit comprising nanoparticles comprising a) a composition comprising nanoparticles comprising paclitaxel coated with an albumin (such as Abraxane^{®}), and b) decitabine, and c) instructions for administering the nanoparticles and the decitabine simultaneously, sequentially, and/or concurrently, for the effective treatment of breast cancer (for example, HER2 negative breast cancer or for example, triple negative breast cancer).

The nanoparticles and the other agents can be present in separate containers or in a single container. It is understood that the medicine may comprise one distinct composition or two or more compositions wherein one composition comprises nanoparticles and one composition comprises another agent.

The kits, medicines, and compositions of this invention may include any one or more aspects or parameters described herein.

### Exemplary Embodiments

In one aspect, the invention provides a method of treating a proliferative disease in an individual comprising administering to the individual: a) an effective amount of a composition comprising nanoparticles comprising a taxane and a carrier protein, and b) an effective amount of at least one other agent that modifies the epigenetics in a cell.

In an embodiment of the above aspect, said other agent is an inhibitor of histone deacetylase.

In an embodiment of the above aspect, said other agent is vorinostat.

In a further embodiment, the method further comprises administering to said individual a platinum-based agent.

In an embodiment of the above aspect, said other agent is an inhibitor of DNA methytransferase.

In a further embodiment, the other agent is azacitidine. In another further embodiment, the other agent is decitabine.

In a further embodiment of the above embodiments, the proliferative disease is cancer. In a further embodiment, the cancer is breast cancer. In yet a further embodiment, the individual is negative for ER, PR, or HER2. In yet a further embodiment, the individual is negative for ER, PR, and HER2.

In a further embodiment of the above embodiments, the cancer is ovarian cancer.

In a further embodiment of the above embodiments, the cancer is non-small lung cancer.

In an embodiment of any of the above embodiments, the composition comprising nanoparticles comprising taxane and albumin and the other agent are administered simultaneously.

In an embodiment of any of the above embodiments, the composition comprising nanoparticles of taxane and albumin and the other agent are administered sequentially.

In another embodiment of any of the above embodiments, the composition comprising nanoparticles of taxane and albumin and the other agent are administered concurrently.

In an embodiment of any of the above embodiments, the taxane is paclitaxel.

In an embodiment of any of the above embodiments, the average diameter of the nanoparticles in the composition is no greater than about 200 nm.

In an embodiment of any of the above embodiments, the carrier protein is albumin. In a further embodiment, the weight ratio of the albumin and the taxane in the nanoparticle composition is less than about 1:1 to about 18:1. In a further embodiment, the weight ratio of the albumin and the taxane in the nanoparticle composition is less than about 1:1 to about 9:1.

In an embodiment of any of the above embodiments, the individual is a human.

In another aspect, the invention provides a kit comprising: a) a composition comprising nanoparticles comprising a taxane and a carrier protein, and b) an effective amount of at least one other agent that modifies the epigenetics in a cell.

In another aspect, the invention provides a medicine comprising: a) a composition comprising nanoparticles comprising a taxane and a carrier protein, and b) an effective amount of at least one other agent that modifies the epigenetics in a cell.

Those skilled in the art will recognize that several embodiments are possible within the scope and spirit of this invention. The invention will now be described in greater detail by reference to the following non-limiting examples. The following examples further illustrate the invention but, of course, should not be construed as in any way limiting its scope.

### EXAMPLES

### Example 1. Treatment with carboplatin and nab-paclitaxel (CP) with or without vorinostat in HER2-negative primary operable breast cancer.

This study included both a run-in phase to investigate the safety of 12 weekly doses of carboplatin ("C") (AUC 2) and nab-paclitaxel ("P") (100 mg/m²) with vorinostat 400mg orally ("po") daily (first 3 out of every 7 days) in women with unresected stage II-III HER2-negative breast cancer and a randomized phase II portion. The purpose of the randomized double-blind phase II study was to evaluate response and surrogate biomarkers to carboplatin and nab-paclitaxel (CP) with or without vorinostat as preoperative systemic therapy (PST) in HER2-negative primary operable breast cancer.

The primary objective of the study was to evaluate the primary pathological complete response rate (pCR). The secondary objectives of the study were to evaluate the safety of these regimens in these patients; to evaluate the clinical response rates; to correlate baseline and change (C1D15) in SUV on FDG PET with clinical and pathological response to CP +/-vorinostat; and to correlate baseline and C1D15 markers of proliferation and apoptosis with clinical and pathological response to CP +/- vorinostat. The exploratory objectives of the study were to compare clinical and pathological response to the regimen in women with basal-like tumors and non basal-like tumors; to evaluate baseline and change in gene methylation and expression profiles; and to evaluate baseline and change in tissue histone acetylation.

The non-randomized run-in phase occurred prior to the primary study in which 6-12 subjects were to be enrolled to confirm the dose of vorinostat to be studied. The primary study was a randomized, double-blind phase II study with a 1:1 randomization to either chemotherapy and placebo or chemotherapy and vorinostat. Eligible patients were stratified by hormone receptor status. There was no formal comparison of response rates between the two treatment arms. A sample size of 31 patients per arm would enable detection of a 25% pCR rate from a null response rate of 10% using a Simon two-stage design with 80% power and a 10% Type I error rate.

The study design is shown in Figure 1. The eligible patients were randomized to receive either (i) 12 weekly doses of carboplatin ("C") (AUC 2) and nab-paclitaxel ("P") (100 mg/m²) or (ii) 12 weekly doses of carboplatin ("C") (AUC 2) and nab-paclitaxel ("P") (100 mg/m²) with vorinostat 400mg orally ("po") daily (first 3 out of every 7 days). N= 62 (31 participants for each arm).

Eligible women were 18 years or older awaiting definitive surgery or preoperative systemic therapy. The eligibility criteria for the study included: histologically confirmed invasive breast cancer; any EP or PR status; HER2-negative; T2, T3, or T4 lesion, any N, M0 or T1c, N1-3,M0; ECOG performance status 0-1; adequate blood counts and organ function (including ANC ≥ 1,500/mm³; platelet count ≥ 150,000/mm³; hemoglobin ≥ 9 g/dL; creatinine ≤ 1.5 times the upper limit of normal (ULN) with creatinine clearance ≥ 50 mL/min; bilirubin with normal limits; and aspartate aminotransferase ("AST") and alanine aminotransferase ("ALT") ≤ 2.5 times (ULN)); no prior chemotherapy, radiation therapy or hormonal therapy for the current cancer.

Six women completed the run-in-phase without dose-limiting toxicities. 6 patients had been randomized in the phase II portion, 2 women completed the phase II portion. The patient characteristics are shown in Table 1.

**Table 1.**

| **Characteristic** | **Run In Phase** | **Phase 2** |
|---|---|---|
| Number | 6 | 6 |
| Median Age (range) | 48 (34 - 72) | 48 (39-63) |
| Median Tumor size, cm (range) | 6.5 cm (1.5 - 13) | 6.5cm (3.5-11.5) |
| Nodal status | | |
| - Negative | 1 (17%) | 0 (0%) |
| - Positive | 5 (83%) | 6 (100%) |
| Receptor status | | |
| - ER-/PR- | 4 (67%) | 2 (33%) |
| - ER+/PR+ | 2 (33%) | 4 (66%) |
| - ER-/PR+ | 0 (0%) | 0 (0%) |

### Example 2. Evaluating response and surrogate biomarkers to the treatment with carboplatin and nab-paclitaxel (CP) with or without vorinostat as preoperative chemotherapy in HER2-negative primary operable breast cancer.

This randomized double-blind phase II trial studies the effects of the treatment of carboplatin and paclitaxel albumin-stabilized nanoparticle formulation (nab-paclitaxel) with or without vorinostat in women with breast cancer that can be removed by surgery. In addition, this study evaluates the response and surrogate biomarkers to the treatment with carboplatin and nab-P (CP) with or without vorinostat,.

There are two arms for this study. Arm I is Active Comparator: patients receive carboplatin intravascular ("IV") and paclitaxel albumin-stabilized nanoparticle formulation IV on day 1 and an oral placebo on days 1-3. Treatment repeats weekly for 12 weeks in the absence of disease progression or unacceptable toxicity. Drug used for Arm I are: carboplatin given IV; paclitaxel albumin-stabilized nanoparticle formulation given IV; and placebo given orally. Arm II is Experimental: patients receive carboplatin and paclitaxel albumin-stabilized nanoparticle formulation as in arm I and oral vorinostat on days 1-3. Treatment repeats weekly for 12 weeks in the absence of disease progression or unacceptable toxicity. Drug used for Arm II are: carboplatin given IV; paclitaxel albumin-stabilized nanoparticle formulation given IV; vorinostat given orally.

The primary objective of the study is to determine pathological complete response (pCR) rates in patients with HER2-negative primary operable breast cancer treated with neoadjuvant therapy comprising carboplatin and paclitaxel albumin-stabilized nanoparticle formulation (CP) with versus without vorinostat. The secondary objectives of the study are: to evaluate the safety of these regimens in these patients; to estimate clinical complete response (cCR) rates in patients treated with these regimens; to correlate baseline and change (day 15) in surrogate uptake values (SUV) on FDG-PET with pathological and clinical response in patients treated with these regimens, and to determine what percent of women with ≥ 25% or ≥ 50% reduction in SUV on day 15 achieve a pCR and a cCR to CP with versus without vorinostat; to correlate baseline and change in markers of proliferation with pathological and clinical response in patients treated with these regimens; and to evaluate long term outcomes (e.g., recurrence of the breast cancer, development of a new cancer, or death) for patients treated with these regimens. The tertiary objectives are: to evaluate baseline and change in candidate gene methylation and expression profiles; to evaluate baseline and change in tissue and peripheral blood mononuclear cell histone acetylation; to compare cCR and pCR in women with basal-like features versus other subtypes.

The patient eligibility for the study includes: 18 Years and older; female; no healthy volunteers. The criteria for disease characteristics include: histologically confirmed infiltrating ductal breast cancer by core needle biopsy (mixed ductal and lobular disease allowed; infiltrating lobular cancer allowed in the run-in portion only); unresected, clinically measurable disease, meeting one of the following clinical staging criteria: (i) T2, T3, or T4 lesion, any N, M0 (ii) T1c, N1-3,M0; patients with skin metastases to the ipsilateral breast for whom chemotherapy is planned prior to definitive surgery are eligible for the primary study portion; HER2-negative disease; hormone receptor status meeting one of the following criteria: (i) estrogen receptor (ER)-negative and progesterone receptor (PR)-negative (ii) ER-positive (grade II or III) and PR-positive or PR-negative (note: any ER or PR status for the run-in portion).

The patient characteristics include: ECOG performance status 0-1; menopausal status not specified; ANC ≥ 1,500/mm³; platelet count ≥ 150,000/mm³; hemoglobin ≥ 9 g/dL; creatinine ≤ 1.5 times the upper limit of normal (ULN); creatinine clearance ≥ 50 mL/min; total bilirubin normal; AST(SGOT) and ALT(SGPT) ≤ 2.5 times (ULN); alkaline phosphatase ≤ 2.5 times ULN; PT such that INR ≤ 1.5 (or an in-range INR, usually between 2 and 3, if a patient is on a stable dose of therapeutic warfarin) and PTT ≤ ULN; Adequate cardiac function defined as no evidence of PR prolongation or AV block on baseline electrocardiogram (ECG); willing to use effective, non-hormonal contraception while on treatment and for at least 3 months thereafter; not pregnant or nursing; no pre-existing peripheral neuropathy ≥ grade 2; no history of severe hypersensitivity reaction to any drug formulated with polysorbate 80 or to E. coli-derived products; no history of allergic reactions attributed to compounds of similar chemical or biologic composition to vorinostat; no medical condition which, in the opinion of the investigator, puts the patient at risk of potentially serious complications while on this therapy

The criteria for patients' prior concurrent therapy include: at least 4 weeks since prior valproic acid or other histone deacetylase inhibitor; no prior chemotherapy, radiotherapy, or endocrine therapy for this cancer (prior tamoxifen or raloxifene or another agent for prevention of breast cancer allowed as long as the patient has discontinued the treatment ≥ 1 month prior to baseline study biopsy); no systemic treatment for prior cancer within the past 5 years (primary study portion); no prior or ongoing systemic treatment for this cancer (primary study portion); no concurrent combination antiretroviral therapy for HIV-positive patients; no other concurrent histone deacetylase inhibitor; no other concurrent chemotherapy, antiestrogen therapy, radiotherapy, or other investigational systemic therapy; no other concurrent biologic therapy; no other concurrent investigational drugs.

The study comprises a randomized phase II study (primary study portion) and a 6-12 patient run-in portion. For the run-in portion, patients receive carboplatin IV and paclitaxel albumin-stabilized nanoparticle formulation IV on day 1 and oral vorinostat on days 1-3. Treatment repeats weekly for 12 weeks in the absence of disease progression or unacceptable toxicity. Once safety of the combination of chemotherapy and vorinostat is confirmed, subsequently enrolled patients are entered to the primary study portion.

For the primary study portion, patients are stratified by hormone receptor status (estrogen receptor (ER)-negative and progesterone receptor (PR)-negative vs ER-positive and/or PR-positive). Patients are randomized to 1 of 2 treatment arms. Arm I: Patients receive carboplatin IV and paclitaxel albumin-stabilized nanoparticle formulation IV on day 1 and an oral placebo on days 1-3. Treatment repeats weekly for 12 weeks in the absence of disease progression or unacceptable toxicity. Arm II: Patients receive carboplatin and paclitaxel albumin-stabilized nanoparticle formulation as in arm I and oral vorinostat on days 1-3. Treatment repeats weekly for 12 weeks in the absence of disease progression or unacceptable toxicity.

Within 2-4 weeks after completion of neoadjuvant chemotherapy, patients undergo breast conserving surgery or mastectomy at the discretion of the treating physician.

Patients undergo tumor tissue biopsy at baseline, day 15, and at the time of definitive surgery. Samples are analyzed by immunohistochemistry (IHC), RNA extraction, and gene expression analysis using RT-PCR to identify candidate markers for response and molecular profiles that may be relevant to an understanding of drug mechanisms. Methylation of relevant genes (e.g., ERalpha, APC-1, RARbeta, cyclin D2, Twist, RASSF1A, and HIN-1) are evaluated by quantitative multiplex methylation-specific PCR. Changes in gene expression as a result of treatment are determined by IHC or quantitative RT-PCR. Blood samples are collected at baseline, day 15, at the time of definitive surgery, and 4 weeks after surgery for DNA methylation studies, pharmacogenomic studies, and histone acetylation assays. Patients also undergo fludeoxyglucose F 18-positron emission tomography (FDG-PET) or PET/CT at baseline and day 15 to assess treatment response as measured by standardized uptake values.

### Example 3. Using FDG-PET prior to neoadjuvant therapy and post neoadjuvant therapy to predict response to the therapy in breast cancer patients.

This study used functional imaging, FDG-PET, to predict response to neoadjuvant therapy in patients with early stage breast cancer. For the patients with early stage breast cancer, FDG-PET was performed at baseline and 7 days after commencement of neoadjuvant therapy in women with early breast cancer. Figure 2 shows the FDG-PET results from 2 patients. Patient 1 (top, Figure 2) had a right-sided breast mass (Standardized Uptake Value ("SUV") of 12.4) prior to neoadjuvant therapy and a reduction in SUV to 6.7 based on FDG-PET. Patient 1 had a partial response to the therapy. Patient 2 (bottom, Figure 2) had a left-sided breast mass (SUV of 31) prior to neoadjuvant therapy and a reduction in SUV to 9.9 based on FDG-PET. Patient 2 had a complete response to therapy.

### Example 4. Phase I/II clinical trial of azacitidine (Vidaza) with Abraxane® for advanced or metastatic solid tumors and breast cancer

This study evaluates the effect of Abraxane® and azacitidine for treatment of solid tumor.

Patients with advanced or metastatic solid tumors, including advanced and solid breast cancer patients, are recruited for this study. Breast cancer patients who are HER2 and neu negative are recruited.

Azacitidine is administered subcutaneously or intravenously at dose levels of 50, 75, and 100 mg/m². The administration is carried out daily for five consecutive days. Abraxane® is administered intravenously at the dose of 100 mg/m² on days 8, 15, and 22 in a four week cycle. The treatment is carried out for six cycles.

The primary endpoints for evaluating the patients include overall response rate, safety, and adverse events. The secondary endpoints for evaluating the patients include progression free survival and clinical responses.

### Example 5. Correlative study of SPARC expression in NSCLC patients treated with the combination of Abraxane and DNA methyltransferase inhibitors

This study evaluates the SPARC expression levels in non-small cell lung cancer patients who are treated with the combination of Abraxane® and azacitidine or decitabine.

Patients with non-small cell lung cancer are recruited, and divided into three groups. Group I patients are administered with Abraxane® alone. Group II patients are administered with the combination of Abraxane® and azacitidine. Group III patients are administered with Abraxane® and decitabine.

Azacitidine is administered subcutaneously or intravenously at dose levels of 50, 75, and 100 mg/m². The administration is carried out daily for five consecutive days each week.

Decitabine is administered intravenously or subcutaneously at 20-45 mg/m² daily for five consecutive days each week.

Abraxane® is administered intravenously at the dose of 100 mg/m² on days 8, 15, and 22 in a four week cycle.

The treatment is carried out for six cycles. The correlation between SPARC expression and response to the treatment is analyzed.

### Example 6. Evaluation of the Efficacy of Intraperitoneal and Intravenous Abraxane® with and without Decitabine in an Intraperitoneal Ovarian Carcinoma model in athymic mice.

The objective of this study is to evaluate and compare the efficacy of intraperitoneal treatments with Abraxane® alone or with Decitabine ("DAC") versus intravenous Abraxane® alone or with Decitabine in an intraperitoneal model of human ovarian carcinoma (OVCAR-3) in athymic mice.

Abraxane is reconstituted with sterile 0.9% saline and then diluted to the appropriate concentration. 50 mg of lyophilized Decitabine/Dacogen is reconstituted in 20 ml of sterile saline. Store 1.5 ml aliquots frozen at -80°C. Prior to dosing, aliquots of the stock solution are diluted 1:10 (dilute to total volume of 15 ml) with saline to obtain 0.25 mg/ml for IP or IV dosing at 2 mg/kg (approximately 50 ml/mouse).

A total of 99 animals (at least 9 animals per test group) are used in this study to evaluate the effect of Abraxane® and DAC intraperitoneally and intravenously administered in an intraperitoneal ovarian carcinoma model. Female NCr nude mice from Taconic are used in the study. Weight: 20-30 grams on the day of implantation. Age: at least 6 weeks old on the day of randomization; btc-x8014 ovcar/2.

Tumor Injection Procedure: Each animal are weighed, and then injected intraperitoneally with 1-4x106 OVCAR-3 cells. The cells re suspended in 0.5ml of Phosphate Buffered Saline (PBS).

Test Article Dosing (IP): Animals are injected intraperitoneally (IP) with 10 ml/kg of either Abraxane or DAC or both starting 1 week after implantation. Control animals are given 10ml/kg IP. Abraxane is administered qdx5 starting on Day 8 after cell implant (Dosing Day 1). DAC is given on days -4, -3,-2 and days 8, 9, and 10.

Test Article Dosing (IV): Animals are injected intravenously (IV) via tail vein with 10 ml/kg of either Abraxane or DAC or both starting 1 week after implantation. Control animals are given 10ml/kg IP. Abraxane is administered qdx5 starting on Day 8 after cell implant (Dosing Day 1). DAC is given on days -4, -3,-2 and days 8, 9, and 10.

Tumor Growth Assessment: Animals are examined grossly at the time they are weighed. Observations of apparent abdominal distention and palpation for intra abdominal masses are performed and findings recorded.

Body Weights: Animals are weighed prior to tumor cell injection, then 2-3 times per week until sacrifice.

Clinical Observations: Animals are observed once daily for general appearance, cachexia, and other abnormalities.

Mortality/Morbidity: All animals are examined twice daily for mortality/morbidity. Any animal judged moribund by the study director are euthanized. Euthanized animals and any animals found dead prior to rigor mortis are necropsied, at the discretion of the Study Director and Sponsor Representative.

Necropsy: After euthanasia, ascites fluid is collected if present. Large tumors is excised and divided equally into 3 sections. One third of the tumor is placed into 10 % neutral buffered formalin overnight, transferred to 95% ethanol, and stored @ room temperature for IHC analysis. The other 2 sections are dispensed into 2 separate tubes and snap frozen in liquid nitrogen for gene array and methylation analysis. Cryo tapes are used for labeling each container with black permanent markers.

The summary of the different treatment groups is provided in Table 2.
Table 2

**Table 1 Treatment Groups**

| Group | No. | Treatment | Abraxane Dose | DAC Dose | Frequency | Route |
|---|---|---|---|---|---|---|
| A | 9 | Saline control | | | Qdx5 | IP |
| B | 9 | Abraxane | 13.4 mg/kg | | Qdx5 (Days 1, 2,3,4,5) | IP |
| C | 9 | Abraxane | 30.0 mg/kg | | Qdx5 (Days 1, 2,3,4,5) | **IP** |
| D | 9 | DAC | | 2 mg/kg | Days -4, -3,-2, 8, 9, 10 | **IP** |
| E | 9 | DAC + | 13.4 mg/kg | 2 mg/kg | Qdx5 (Days 1, 2,3,4,5) + Days -4, -3,-2, 8, 9, 10 | IP |
| F | 9 | DAC + | 30.0 mg/kg | 2 mg/kg | Qdx5 (Days 1, 2,3,4,5) + Days -4, -3,-2, 8, 9, 10 | IP |
| G | 9 | Abraxane | 13.4 mg/kg | | Qdx5 (Davs 1,2,3,4,5) | IV |
| H | 9 | Abraxane | 30.0 mg/kg | | Qdx5 (Days 1, 2,3,4,5) | IV |
| I | 9 | DAC | | 2 mg/kg | Days -4, -3,-2, 8, 9, 10 | IV |
| J | 9 | DAC + | 13.4 mg/kg | 2 mg/kg | Qdx5 (Days 1, 2,3,4,5) + Days -4, -3,-2, 8, 9, 10 | IV |
| K | 9 | DAC + | 30.0 mg/kg | 2 mg/kg | Qdx5 (Days 1, 2,3,4,5) + Days -4, -3,-2, 8, 9, 10 | IV |

### Example 7. A Phase I/II Study of the Hypomethylating Agent Azacitadine with the Nanoparticle Albumin Bound Paclitaxel in the Treatment of Patients with Advanced or Metastatic Solid Tumors and Breast Cancer

This study evaluates the effect of paclitaxel and azacitidine for treatment of refractory advanced or metastatic solid tumors, including lymphoma, sarcoma, ovarian, lung, endometrial, pancreatic, and breast cancer.

Patients with evaluable advanced or metastatic solid tumors, including lymphoma, adequate organ function, PS 0-2, and unlimited prior cytotoxic chemotherapies, including taxanes, were eligible. Cohorts of 3-6 patients were enrolled and treated.

Escalating doses of azacitidine (75-100 mg/m²) were administered to patients subcutaneously on days 1-5, followed by a fixed dose of paclitaxel (100 mg/ mg/m²) on days 8, 15, and 22 of a 28-day cycle, for a total of 6 cycles. Serum and/or tissue samples, where appropriate, were collected for correlative studies.

Patients were initially permitted to have an unlimited number of prior chemotherapies. However, the protocol was amended to permit no more than 2 prior cytotoxic regimens after two of the first five patients experienced dose limited toxicities and dose reduction of paclitaxel occurred in 3 patients. All affected patients had 4 or more prior cytotoxic regimens. Cohort 2 was treated at the same dose level (azacitidine 75mg/m²) with no dose limited toxicities. Cohort 3 was treated at the next dose level (azacitidine 100 mg/m²). Two of 4 had dose limited toxicities of prolonged grade 4 neutropenia. Therefore, the maximum tolerated dose (MTD) of azacitidine in this regimen is 75 mg/m². Three additional patients were treated at the MTD dose with no grade 4 toxicity in cycle 1. To date, 16 patients have enrolled in the phase I part. Two patients were removed before completing cycle 1 because of disease progression. One patient was removed after cycle 4 for noncompliance.

Clinical activity included 1 durable complete response in refractory diffuse large B cell lymphoma, 6 partial responses in ovarian and endometrial cancer, 4 stable diseases in lung, sarcoma and pancreatic cancer, 1 unconfirmed partial response in breast cancer, and 1 progressive disease in chronic lymphocytic leukemia/small lymphocytic lymphoma. Two breast cancer patients in the phase II part had unconfirmed partial responses and are still getting treatments.

The results of this study suggest that an azacitidine dose of 75 mg/m² followed by weekly doses of 100 mg/m² paclitaxel was well tolerated and seemed to result in dramatic responses in heavily pre-treated patients with cancer of diverse histology.

### Example 8. SPARC expression and methylation status in NSCLC cell lines and PD NSCLC primary tumors

This study evaluates the expression of SPARC in NSCLC cell lines and patient-derived (PD) NSCLC specimens. SPARC expression was examined by RT PCR in 13 NSCLC cell lines and 22 PD NSCLC tumor xenografts. Specimens were considered SPARC positive when SPARC expression was 75% or more relative to the expression of the endogenous control, GAPDH. Specimens were considered as SPARC negative if SPARC expression was less than 25% to the expression of GAPDH. Levels in-between are considered SPARC intermediate. As shown in Table 3, 8 of 13 (62%) NSCLC cell lines were SPARC negative relative to the expression of internal control GAPDH. Four of 13 (31%) NSCLC cell lines were SPARC positive. For the 22 PD NSCLC xenografts, 15 of them (68%) were SPARC negative; two were SPARC intermediate. The others are SPARC positive. These results are concordant with a previous report (Suzuki, M. et al. Br J Cancer 2005 92:942-948).

### Materials and Methods

Cell Lines and Minimally Passaged (Fewer than 10 Passages) Primary Patient-Derived Tumor Specimen

NSCLC cell lines (H226, H358, H441, H522, H661, H727, H1299, H1650, H1703, H2935, H460, H157, and A549) were purchased from American Type Culture Collection (Manassas, VA) and maintained in Roswell Park Memorial Institute medium 1640 with 10% fetal bovine serum (Sigma, St Louis, MO), 100 units/ml penicillin, and 100 µg/ml streptomycin (Cellgro, Manassas, VA) at 37°C in 5% CO₂.

All patient-derived (PD) NSCLC tumor specimens were derived from patients whose surgeries were performed at Roswell Park Cancer Institute (RPCI; Buffalo, NY). Specimens were processed through the Tissue Procurement Facility for pathologic assessment. Surgical samples were obtained with informed consent from patients and under a research protocol approved by the institutional review board and the research ethics committee at RPCI. Samples were examined for presence of malignant/normal areas before transplantation into animals. Animal experiments were approved by the Institutional Animal Care and Use Committee.

### Real-Time Polymerase Chain Reaction

Total RNA was extracted from the samples with Trizol reagent (Invitrogen; Carlsbad, CA), and first-strand complementary DNA was generated using SuperScript III First-Strand Synthesis System (Invitrogen). The forward polymerase chain reaction (PCR) amplification primer of SPARC was 5'-AAGATCCATGAGAATGAGAAG-3' (Ex8-S), and the reverse primer was 5-AAAAGCGGGTGGTGCAATG-3' (Ex9-AS). Semiquantitative PCR was carried out for 4 minutes at 95°C for initial denaturation, followed by 33 cycles of 94°C for 25 seconds, 56°C for 25 seconds, and 68°C for 40 seconds. Glyceraldehyde-3-phosphate dehydrogenase (GAPDH) was used as endogenous control. Quantitative real-time (RT) PCR was done with SYBR GreenER qPCR superMix for ABI PRISM (Invitrogen) using ABI 7300 RT PCR system (Invitrogen), and GAPDH was used as a housekeeping gene for purpose of normalization.

**Table 3. SPARC expression status analysis in NSCLC cell lines and patient-derived primary NSCLC xenografts**

| **Sample** | **Detector** | **Task** | **Avg Ct** | **dCt (Target-ENDO)** | **Relative Expression (1/2)^dCt** | **SPARC Status** |
|---|---|---|---|---|---|---|
| H157 | SPARC | Target | 21.85 | -0.67 | 1.591073 | Positive |
| | GADPH | ENDO | 22.52 | | | |
| H226 | SPARC | Target | 19.56 | -2.16 | 4.469149 | Positive |
| | GADPH | ENDO | 21.72 | | | |
| H358 | SPARC | Target | 33.03 | 10.28 | 0.000804 | Negative |
| | GADPH | ENDO | 22.75 | | | |
| H441 | SPARC | Target | 35.43 | 11.79 | 0.000282 | Negative |
| | GADPH | ENDO | 23.64 | | | |
| H460 | SPARC | Target | 34.21 | 11.07 | 0.000465 | Negative |
| | GADPH | ENDO | 23.14 | | | |
| H522 | SPARC | Target | 32.11 | 6.6 | 0.010309 | Negative |
| | GADPH | ENDO | 25.51 | | | |
| H661 | SPARC | Target | 22.79 | -1.29 | 2.445281 | Positive |
| | GADPH | ENDO | 24.08 | | | |
| H727 | SPARC | Target | 31.39 | 7.82 | 0.004425 | Negative |
| | GADPH | ENDO | 23.57 | | | |
| H1299 | SPARC | Target | 29.06 | 7.83 | 0.004395 | Negative |
| | GADPH | ENDO | 21.23 | | | |
| H1650 | SPARC | Target | 21.6 | -0.25 | 1.189207 | Positive |
| | GADPH | ENDO | 21.85 | | | |
| H1703 | SPARC | Target | 33.11 | 12 | 0.000244 | Negative |
| | GADPH | ENDO | 21.11 | | | |
| H2935 | SPARC | Target | 26.22 | 1.77 | 0.293209 | Intermediate |
| | GADPH | ENDO | 24.45 | | | |
| A549 | SPARC | Target | 33.41 | 12.36 | 0.00019 | Negative |
| | GADPH | ENDO | 21.06 | | | |
| NSCLC_16384 | SPARC | Target | n/a | n/d | n/d | Negative |
| | GADPH | ENDO | 26.69 | | | |
| NSCLC_16325 | SPARC | Target | n/a | n/d | n/d | Negative |
| | GADPH | ENDO | 25.17 | | | |
| NSCLC_17265 | SPARC | Target | 31.45 | 0.49 | 0.712025 | Intermediate |
| | GADPH | ENDO | 30.96 | | | |
| NSCLC_16947 | SPARC | Target | n/a | n/d | n/d | Negative |
| | GADPH | ENDO | 27.36 | | | |
| NSCLC_17228 | SPARC | Target | 22.58 | -3.46 | 11.00433 | Positive |
| | GADPH | ENDO | 26.05 | | | |
| NSCLC_17291 | SPARC | Target | 23.43 | 0.38 | 0.768438 | Positive |
| | GADPH | ENDO | 23.05 | | | |
| NSCLC_17246 | SPARC | Target | 24.45 | -2.64 | 6.233317 | Positive |
| | GADPH | ENDO | 27.08 | | | |
| NSCLC_16898 | SPARC | Target | 31.2 | 4.89 | 0.033726 | Negative |
| | GADPH | ENDO | 26.31 | | | |
| NSCLC_17531 | SPARC | Target | 24.54 | -0.19 | 1.140764 | Positive |
| | GADPH | ENDO | 24.73 | | | |
| NSCLC_16591 | SPARC | Target | 40 | 3.75 | 0.074325 | Negative |
| | GADPH | ENDO | 36.25 | | | |
| NSCLC_16372 | SPARC | Target | 30.84 | 0.04 | 0.972655 | Positive |
| | GADPH | ENDO | 30.8 | | | |
| NSCLC_15848 | SPARC | Target | 40 | 7.6 | 0.005154 | Negative |
| | GADPH | ENDO | 32.4 | | | |
| NSCLC_16465 | SPARC | Target | 40 | 9.19 | 0.001712 | Negative |
| | GADPH | ENDO | 30.81 | | | |
| NSCLC_15946 | SPARC | Target | 34.82 | 1.88 | 0.271684 | Intermediate |
| | GADPH | ENDO | 32.94 | | | |
| NSCLC xenograft 1 | SPARC | Target | 40 | 8.84 | 0.002182 | Negative |
| | GADPH | ENDO | 31.16 | | | |
| NSCLC xenograft 2 | SPARC | Target | 38.87 | 6.54 | 0.010746 | Negative |
| | GADPH | ENDO | 32.33 | | | |
| NSCLC xenograft 3 | SPARC | Target | 40 | 8.36 | 0.003044 | Negative |
| | GADPH | ENDO | 31.64 | | | |
| NSCLC xenograft 4 | SPARC | Target | 40 | 6.2 | 0.013602 | Negative |
| | GADPH | ENDO | 33.8 | | | |
| NSCLC xenograft 5 | SPARC | Target | 40 | 9.88 | 0.001061 | Negative |
| | GADPH | ENDO | 30.12 | | | |
| NSCLC xenograft 6 | SPARC | Target | 40 | 7.64 | 0.005013 | Negative |
| | GADPH | ENDO | 32.36 | | | |
| NSCLC xenograft 7 | SPARC | Target | 40 | 5.73 | 0.018841 | Negative |
| | GADPH | ENDO | 34.27 | | | |
| NSCLC xenograft 8 | SPARC | Target | 40 | 8.39 | 0.002981 | Negative |
| | GADPH | ENDO | 31.61 | | | |

| | | | | | | |
|---|---|---|---|---|---|---|
| ENDO, endogenous control; n/a, not applicable | | | | | | |

### Example 9. Tumoral SPARC reexpression with demethylating agent

The objective of this study was to determine if demethylation of the promoter region of the SPARC gene results in reexpression of the SPARC gene in cancer cells. DNA methylation in gene promoter regions is a mechanism to silence gene expression. The loss of SPARC expression in a large proportion of NSCLC specimens may be due to the methylation in the promoter region of SPARC gene was investigated. Representative samples of NSCLC cell lines or PD xenografts were treated with decitabine (5 µM) in vitro for three days and promoter methylation was analyzed.

Genomic DNA was obtained from cell lines and primary tumors by using DNeasy Blood and Tissue Kit (Qiagen; Valencia, CA) according to the manufacturer's handbook. The DNA methylation pattern in the CpG island of SPARC was determined using the method of methylation-specific PCR (MSP) and with the EZ DNA Methylation Kit (Zymo research, Irvine, CA). Primers for the methylated reaction were Sparc-unmsp-F: 5'- TTTTTTAGATTGTTTGGAGAGTG-3' (sense), Sparcunmsp- R: 5'-AACTAACAACATAAACAAAAATATC-3' (antisense), sparc-msp-F: 5'-GAGAGCGCGTTTTGTTTGTC-3' (sense), and sparc-msp-R: 5'-AACGACGTAAACGAAAATATCG-3' (antisense). PCR amplification was carried out with bisulfite-treated DNA as a template using specific primer sequences for the methylated and unmethylated status of the gene. Water blanks were included with each assay. PCR amplification was carried out for 12 minutes at 94°C for initial denaturation, followed by 40 cycles of 94°C for 30 seconds, 58°C for 25 seconds, and 68°C for 45 seconds. Results were confirmed by repeating the bisulfite treatment and MSP for all samples. PCR products were visualized on 1.5% agarose gels.

Western blot analysis was carried out using standard methods. Polyclonal antihuman SPARC antibody was purchased from R&D System (Minneapolis, MN). NSCLC cell lines A549, H460, and H157 were treated with 5 µM decitabine for 3 days in vitro and then harvested for RT-PCR and Western blot analysis. PD NSCLC xenografts and H460 xenografts treated with or without decitabine at 1.5 mg/kg/d. Xenografts were harvested for SPARC expression analysis. GAPDH were used as the endogenous control. Xenograft lysates were prepared and subjected to immunoblot analysis using 50 µg of cellular protein as described previously (Yu, C. et al. Blood 2003 102:3765-3774). The levels of β-action (Sigma) were measured as control for equal loading. Immunoblots were developed with enhanced chemiluminescence (Amersham Bioscience, Golden Valley, MN).

H226, a SPARC-positive cell line, has the highest relative SPARC expression ratio to endogenous control GAPDH (4.469, Table 3). Methylation-specific PCT (MSP) results showed that there was no methylation detected within its promoter (Figure 3). H460, a SPARC-negative cell line (Table 3), is methylated in the *SPARC* promoter region, and there is weak PCR signal with the unmethylated promoter region. On treatment with decitabine, the SPARC expression is up-regulated. This is shown as the increase of unmethylated promoter of *SPARC* in H460 (Figure 3), which manifested as the upregulation of SPARC protein (Figure 4A). Similar results were also observed in vitro with NSCLC cell lines (A549, H460, and H157) and in vivo with PD xenografts (NSCLC_16325 and NSCLC_16384) and H460 xenograft (Figures 3 and 4). The loss of SPARC expression in NSCLC specimens is largely attributed to the methylation in the promoter region of SPARC gene. These results show that SPARC expression can be up-regulated by exposure to decitabine in vitro or in vivo.

### Example 10. Endogenous tumor SPARC expression and response to Abraxane® in vivo.

This study compares the antitumor efficacy of Abraxane® with equitoxic dose of taxol in a series of PD NSCLC xenografts with different SPARC expression status. SPARC expression in tumors has been shown to facilitate the transport of albumin-bound drugs (Trieu V et al. Proc. Ann. AACR 2007 abstract 3480). Abraxane® is an albumin-bound paclitaxel. NSCLC_16372 is a SPARC-positive tumor and as shown in Figure 5A, the growth of a NSCLC_16372 xenograft was suppressed by treatment with either taxol or Abraxane® compared with vehicle control. Abraxane® was more effective than taxol in inhibiting xenograft growth (p = 0.0239). Similar results were observed in NSCLC_15946, a SPARC-intermediate specimen. The tumor growth was significantly inhibited with either taxol (p = 0.0004) or Abraxane® (p < 0.0001) compared with vehicle control. Again, exposure to Abraxane® displayed a more profound growth suppression than taxol (p < 0.0001; Figure 5B). NSCLC_16465 and NSCLC_16591 are SPARC-negative tumors. Neither administration of taxol nor administration of Abraxane® showed any effect on tumor growth compared with vehicle control in NSCLC_16,591 (p = 0.3628 of taxol to vehicle; p = 0.1826 of Abraxane® to vehicle; Figure 5D). Interestingly, in NSCLC_16465 xenografts, Abraxane® demonstrated significantly greater antitumor effect compared with taxol (p < 0.0001), which showed no difference compared with vehicle control. Abraxane® is, thus, generally more effective than taxol at equitoxic doses in inhibiting the growth of PD NSCLC xenografts, and this effect is not consistently correlated with tumor SPARC expression status.

### Reagents

Abraxane® was provided by Abraxis BioScience. Taxol was purchased from Ben Venue Labs (Bedford, OH). Decitabine was purchased from Sigma-Aldrich (St. Louis, MO). All the drugs were prepared and aliquoted and stored at -80°C for use within 1 week. For decitabine experiments with NSCLC cell lines in vitro, cells were cultured in medium with DEC (5 µM) for 5 days, with medium changes on days 1 and 3. Either Abraxane® or taxol was added to the changed media on day 3. Control cells were only treated with vehicle of 1% DMSO in 1 ml of media. Cells were harvested for RNA extraction, SPARC expression analysis, or cell death rate assessment on day 5.

### Experiments with Tumor Xenografts In Vivo

Experimental studies were carried out using 6- to 8-week-old CB.17 severe combined immunodeficiency (SCID)/SCID with an average body weight ∼25 g. H460 (5 x 10⁶ cells) or PD NSCLC xenografts (mouse-to-mouse passage), which were cut into small fragments (2-3 mm in size), were implanted subcutaneously at the flank region. Tumor growth was monitored by periodic visual inspection at the site of implantation, and the dimensions of the xenografts were measured every 2 to 3 days. Tumor volume was calculated using the following formula: V = LD x (SD)²/2, where V is the tumor volume, LD is the longest tumor diameter, and SD is the shortest tumor diameter.

To establish a maximally tolerated dose to be used in experiments involving pretreatment with decitabine, groups of mice implanted with H460 cell lines were tested at four dose levels of decitabine, *i.e.,* 0.75, 1.5, 2, and 4 mg/kg, administered intraperitoneally as a single dose and monitored for viability (weight loss and death). Two mice from each group were killed 48 hours after completing decitabine treatment alone and tumor xenografts harvested to affirm SPARC-expression status. For combination drug treatment, Abraxane® was administered by tail vein injection at 30 mg/kg/d for 5 consecutive days. Taxol and decitabine were used at 13.4 mg/kg/d and 1.5 mg/kg/d, respectively. The doses for Abraxane® and taxol have been previously demonstrated to be equitoxic at these levels (Desai N et al. Clin Cancer Res 2006 12:1317-1324). At the end of drug treatment, the mice were killed. Xenografts were harvested and assayed to determine SPARC status.

### Example 11. Tumor SPARC reexpression/upregulation and response to Abraxane® or Taxol.

The study evaluates tumor SPARC expression upregulation on treatment with decitabine in vitro and in vivo. To establish a tolerable dose to be used in experiments in vivo, groups of mice implanted with H460 cell lines were tested at four dose levels of decitabine, *i.e.,* 0.75, 1.5, 2, and 4 mg/kg, respectively. This is based on prior publication showing that decitabine administered twice daily intraperitoneally for 5 days was toxic at 10 mg/kg/dose, and 2 mg/kg dose was associated with mild toxicity (Guo, ZS et al Cancer Res 2006 22:1105-1113). There is dose-dependent reexpression of SPARC in H460 xenografts on treatment with decitabine. Nevertheless, decitabine at 2 and 4 mg/kg was toxic with 6/8 (75%) mice dead between 8 and 12 days after only one decitabine dose (without Abraxane® treatment). Decitabine dose at 1.5 mg/kg was subsequently used in the combination experiments.

To further elucidate the effect of SPARC expression status on the differential antitumor efficacy of Abraxane® in comparison with equitoxic dose of taxol in vivo, experiments in two SPARC-negative PD NSCLC primary xenografts (NSCLC_16325 or NSCLC_16384) and one SPARC negative established NSCLC cell line (H460) were performed. First, SPARC expression in xenografts was up-regulated on exposure to decitabine (1.5 mg/kg; Figure 4B). Second, compared with vehicle, on treatment with either Abraxane® or taxol alone, only administration with Abraxane® in NSCLC_16325 showed significant antitumor efficacy (p = 0.0065). There was, however, no significant difference between Abraxane® compared with taxol in suppressing the growth of these SPARC-negative PD xenografts (p = 0.4577 in NSCLC_16325 and *p* = 0.9897 of NSCLC_16384; Figures 6A, B). Third, we found that the antitumor activities of both taxol and Abraxane® were enhanced to a similar degree by pretreatment with DEC; however, this activity did not reach statistical significance (p = 0.1374 of Abraxane^{®} + decitabine versus Abraxane^{®} and *p* = 0.4713 of taxol + decitabine versus taxol in 16,325; *p* = 0.8844 of Abraxane® + decitabine versus Abraxane® and *p* = 0.7410 of taxol + decitabine versus taxol in NSCLC_16,384; Figures 6A, B). Finally, in H460 xenografts, Abraxane® showed superior growth-suppressing activity compared with vehicle or taxol (p = 0.0002). Nevertheless, decitabine pretreatment resulted in minimal change to Abraxane® antitumor activity, whereas a synergistic effect was seen in combination with taxol (p = 0.0097). Nonetheless, the tumor growth inhibition achieved by Abraxane® alone seemed greater compared with decitabine with equitoxic dose of taxol, though not statistically significant (84% of Abraxane® versus 66% of decitabine + taxol, *p* = 0.5402) (Figure 6C).

Pretreatment with decitabine to enhance antitumor efficacy of taxol or Abraxane® was also verified in SPARC-negative NSCLC cell lines A549 and H460 in vitro. As shown in Figure 7, decitabine enhanced the cytotoxicity of both taxol and Abraxane® in a wide range of doses (2.5, 5, 10, 20, and 50 nM). There is no difference in the antitumor activity between taxol and Abraxane® at equimolar doses.

### Methods

Cell death rate analysis: NSCLC cells were seeded into 24-well plates and treated with DMSO or agents. Cells were then harvested and stained with trypan blue and viewed under light microscope. Treated cells were harvested and resuspended in trypan blue/phosphate-buffered saline solution. The number of dead/nonviable cells (blue cells) and viable cells (white cells) were counted in representative randomly selected regions, and the ratio of blue cells to white cells is calculated as the cell deathrate (%). Each experiment was performed in triplicate at least three times.

Statistical Analysis: Data from in vitro studies are expressed as the mean and standard deviation representing results from at least three independent experiments using cells derived from separate batches of cultures. To describe the observed variability in the in vivo data and test for differences between groups, a multivariate linear model was fit to each dependent variable (tumor volume). All tests were two sided and tested at a 0.05 nominal significance level. SAS version 9.2 statistical software (Cary, NC) was used for all statistical analyses. Difference was considered statistically significant when the calculated *p* value was less than 0.05.

The invention refers *inter alia* to the following items:
1. A method of treating a proliferative disease in an individual comprising administering to the individual:
   a) an effective amount of a composition comprising nanoparticles comprising a taxane and a carrier protein, and
   b) an effective amount of at least one other agent that modifies the epigenetics in a cell.
2. The method according to item 1, wherein said other agent is an inhibitor of histone deacetylase.
3. The method according to item 2, wherein said other agent is vorinostat.
4. The method according to item 3, further comprising administering to said individual a platinum-based agent.
5. The method according to item 1, wherein said other agent is an inhibitor of DNA methytransferase.
6. The method according to item 5, wherein the other agent is azacitidine.
7. The method according to item 5, wherein the other agent is decitabine.
8. The method according to any one of items 1-7, wherein the proliferative disease is cancer.
9. The method according to item 8, wherein the cancer is breast cancer.
10. The method according to item 9, wherein the individual is negative for ER, PR, or HER2.
11. The method according to item 10, wherein the individual is negative for ER, PR, and HER2.
12. The method according to item 8, wherein the cancer is ovarian cancer.
13. The method according to item 8, wherein the cancer is non-small lung cancer.
14. The method according to any one of items 1-13, wherein the composition comprising nanoparticles comprising taxane and albumin and the other agent are administered simultaneously.
15. The method according to any one of items 1-13, wherein the composition comprising nanoparticles of taxane and albumin and the other agent are administered sequentially.
16. The method according to any one of items 1-13, wherein the composition comprising nanoparticles of taxane and albumin and the other agent are administered concurrently.
17. The method according to any one of items 1-16, wherein the taxane is paclitaxel.
18. The method according to any one of items 1-17, wherein the average diameter of the nanoparticles in the composition is no greater than about 200 nm.
19. The method according to any one of items 1-18, wherein the carrier protein is albumin.
20. The method according to item 19, wherein the weight ratio of the albumin and the taxane in the nanoparticle composition is less than about 1:1 to about 18:1.
21. The method according to item 20, wherein the weight ratio of the albumin and the taxane in the nanoparticle composition is less than about 1:1 to about 9:1.
22. The method according to any one of items 1-21, wherein the individual is a human.
23. A kit comprising: a) a composition comprising nanoparticles comprising a taxane and a carrier protein, and b) an effective amount of at least one other agent that modifies the epigenetics in a cell.
24. A medicine comprising: a) a composition comprising nanoparticles comprising a taxane and a carrier protein, and b) an effective amount of at least one other agent that modifies the epigenetics in a cell.

## Claims

1. A composition comprising nanoparticles comprising a taxane and a carrier protein for use in a method of treating a proliferative disease in an individual, wherein the method further comprises administering to the individual at least one other agent that inhibits a DNA methyltransferase.

2. The composition for use according to claim 1, wherein said other agent is decitabine.

3. The composition for use according to claim 1, wherein said other agent is azacitidine.

4. The composition for use according to claim 3, wherein azacitidine is administered by oral administration.

5. The composition for use according to any one of claims 1-4, wherein the proliferative disease is cancer.

6. The composition for use according to claim 5, wherein the cancer is breast cancer.

7. The composition for use according to claim 6, wherein the individual is negative for ER, PR, and/or HER2.

8. The composition for use according to claim 5, wherein the cancer is ovarian cancer or non-small lung cancer.

9. The composition for use according to any one of claims 1-8, wherein the composition and the other agent are administered simultaneously, sequentially, or concurrently.

10. The composition for use according to any one of claims 1-9, wherein the taxane is paclitaxel, and/or wherein the average diameter of the nanoparticles in the composition is no greater than about 200 nm.

11. The composition for use according to any one of claims 1-10, wherein the carrier protein is albumin.

12. The composition for use according to claim 11, wherein the weight ratio of the albumin and the taxane in the composition is less than about 1:1 to about 18:1, or wherein the weight ratio of the albumin and the taxane in the nanoparticle composition is less than about 1:1 to about 9:1.

13. The composition for use according to any one of claims 1-12, wherein the individual is a human.

14. A kit comprising: a) a composition comprising nanoparticles comprising a taxane and a carrier protein, and b) at least one other agent that inhibits a DNA methyltransferase.

15. A medicine comprising: a) a composition comprising nanoparticles comprising a taxane and a carrier protein, and b) at least one other agent that inhibits a DNA methyltransferase.
